(19) Europäisches Patentamt
European Patent Office
Office européen des brevets

(11) EP 1 439 223 A1

(12) EUROPEAN PATENT APPLICATION
published in accordance with Art. 158(3) EPC

(43) Date of publication:
21.07.2004 Bulletin 2004/30

(21) Application number: 02800744.1

(22) Date of filing: 02.10.2002

(51) Int Cl.[7]: **C12N 15/00**, C07K 14/00, C12N 5/10, C12P 21/02, C12P 21/00, C07K 16/28, G01N 33/50, G01N 33/15, A01K 67/027, C12R 1/91

(86) International application number:
PCT/JP2002/010280

(87) International publication number:
WO 2003/031620 (17.04.2003 Gazette 2003/16)

(84) Designated Contracting States:
AT BE CH CY DE DK ES FI FR GB GR IE IT LI LU MC NL PT SE TR
Designated Extension States:
AL LT LV MK RO SI

(30) Priority: 02.10.2001 JP 2001306851
12.07.2002 JP 2002204385

(71) Applicants:
- MOCHIDA PHARMACEUTICAL CO., LTD.
Shinjuku-ku Tokyo 160-8515 (JP)
- Kazusa Dna Research Institute
Kisarazu-shi, Chiba 292-0818 (JP)

(72) Inventors:
- OHARA, Osamu, Kazusa DNA Research Institute
Kisarazu-shi, Chiba 292-0818 (JP)
- NAGASE, Takahiro,
Kazusa DNA Research Institute
Kisarazu-shi, Chiba 292-0818 (JP)
- KATOU, Yutaka,
Mochida Pharmaceutical Co., Ltd.
Tokyo 160-8515 (JP)
- TAKAHASHI, Tomohiro,
Mochida Pharmaceut.Co., Ltd.
Tokyo 160-8515 (JP)
- 0HKAWA, Kazuhumi,
Mochida Pharmaceutical Co., Ltd.
Tokyo 160-8515 (JP)
- SHIRAKAWA, Kamon,
Mochida Pharmaceutical Co., Ltd.
Tokyo 160-8515 (JP)

(74) Representative: Wablat, Wolfgang, Dr. Dr.
Wablat . Lange . Karthaus
Anwaltssozietät
Potsdamer Chaussee 48
14129 Berlin (DE)

# (54) NOVEL CLASS II CYTOKINE RECEPTOR

(57) A novel class II cytokine receptor gene and a protein, and a method of efficiently evaluating an activity controller for the protein are provided.

A DNA comprising the nucleotide sequence represented by SEQ ID NO:1; a class II cytokine receptor encoded by the DNA; an antisense nucleic acid for the DNA sequence; and a method of evaluating an activity controller for the protein.

EP 1 439 223 A1

## Description

### FIELD OF THE INVENTION

The present invention relates to a novel class II cytokine receptor, a DNA encoding the protein and its fragment, an expression vector including the DNA, a transformant produced by using the expression vector, an antibody having a reactivity with the protein or its partial peptide, a method of determining SJ2368 using the antibody, and a method of screening for an agent capable of controlling an activity or an expression of the protein.

### BACKGROUND OF THE INVENTION

Cells control their functions and their growth and differentiation by communicating with other cells. Cytokine is a general name of a group of glycoproteins involved in intercellular signal transduction. Cytokine activates a signal transduction pathway within cells by specifically binding to a cytokine receptor present on a cell membrane. The signal is transduced to cell nucleus so that the expression of a gene is controlled and the growth, differentiation, death and functional expression of cells are regulated.

A cytokine receptor is classified into several families based on its structural characteristics. Many cytokine receptors such as IL-2, 3, 4, 5, 6, 7, 9 and 11, granulocyte macrophage colony stimulating factor (GM-CSF), granulocyte colony stimulating factor (G-CSF), erythropoietin (EPO), growth hormone (GH) and the like belong to a class I cytokine receptor family. A specific sequence comprising about 200 amino acid residues is present in an extracellular domain of a receptor belonging to this family. It is referred to as "cytokine receptor homologous unit". Interferon (IFN) $\alpha/\beta$ receptor, IFN$\gamma$ receptor, IL-10 receptor , IL-20 receptor and the like belong to a class II cytokine receptor family. The receptor belonging to this family has a class II cytokine receptor motif comprising three tryptophan and two sets of S-S bonds in its extracellular domain.

Cytokine recognized by the class II cytokine receptor family includes IL-10 family such as IL-10, 19, 20, IL-TIF (inductor from T cell; IL-22), AK155 (IL-26), MDA-7 (melanoma differetiation-associated factor-7; IL-24) and the like. Among the IL-10 family, IL-TIF has an activity of inducing an acute phase inflammatory protein in HepG2 hepatocytes (Laure et al., Proc. Natl. Acad. Sci. USA, Vol. 97, pp. 10144-10149 (2000)) and IL-20 is believed to be associated with psoriasis from the analysis of transgenic mice (Blumberg et al. Cell, Vol. 104, pp. 9-19 (2001)). MDA-7 inhibits the growth of tumour cells when it transforms the cells (Su et al., Proc. Natl. Acad. Sci. USA, Vol. 95, pp. 14400-14405 (1998)). Thus, the relation of the novel IL-10 family with pathological conditions becomes clear rapidly.

Among the IL-10 family, the receptors for IL-10, IL-20 and IL-TIF have been identified. While, with respect to IL-19, MDA-7 and AK155, its receptor protein is not identified. So, the isolation and identification of a novel class II cytokine receptor recognizing the I1-10 family is desired.

### SUMMARY OF THE INVENTION

The present invention provides an agent and a method useful for preventing and treating of inflammatory diseases and malignant tumours by identifying a novel class II cytokine receptor and its gene.

The present invention relates to a novel gene (sj2368) and a novel class II cytokine receptor (SJ2368) encoded by this gene. And, the present invention provides a host cell transformed with the gene, an antisense nucleic acid controlling the expression of SJ2368, an antibody against SJ2368 or its partial peptide, a method of determining SJ2368 using the antibody, and a method of screening for an agent capable of controlling an expression or an activity of SJ2368.

<Nucleic acid>

The present invention provides the gene sj2368 encoding SJ2368 as described below in detail. Specifically, the gene sj2368 is a DNA encoding any one of the following proteins (A) to (D):

(A) a human class II cytokine receptor comprising the amino acid sequence represented by SEQ ID NO:2;
(B) a human soluble SJ2368 protein comprising the amino acid sequence represented by SEQ ID NO:20;
(c) a mouse class II cytokine receptor comprising the amino acid sequence represented by SEQ ID NO:14;
(D) a mouse soluble SJ2368 protein comprising the amino acid sequence represented by SEQ ID NO:18.

Specific example of the DNA encoding a human class II cytokine receptor comprising the amino acid sequence represented by SEQ ID NO: 2 is a cDNA represented by SEQ ID NO: 1 or 3. In addition to cDNAs represented by SEQ ID NO: 1 and 3, genomic DNAs that said cDNAs come from are also included within the present invention. A human soluble SJ2368 protein comprising the amino acid sequence represented by SEQ ID NO:20 is a splicing variant of the human class II cytokine receptor comprising the amino acid sequence represented by SEQ ID NO:2 . Thus, the sequence of a DNA encoding the human soluble SJ2368 protein comprising the amino acid sequence represented by SEQ ID NO:20 is derivable from the nucleotide sequence of the cDNA represented by SEQ ID NO:1 or 3. The nucleotide sequence encoding the amino acid sequence represented by SEQ ID NO:20 is represented by SEQ ID NO: 24. Example of a DNA encoding the mouse class II cytokine receptor comprising the amino acid sequence represented by SEQ ID NO:14 is a cDNA represented by SEQ ID NO: 15 or 16. In addition to cDNAs represented by SEQ ID NO:15 and 16, genomic DNAs that said cDNAs come from are also included within the present invention. The mouse soluble SJ2368 protein comprising the amino acid sequence represented by SEQ ID NO: 18 is a splicing variant of the mouse class II cytokine receptor comprising the amino acid sequence represented by SEQ ID NO:14. Thus, the sequence of a DNA encoding the mouse soluble SJ2368 protein comprising the amino acid sequence represented by SEQ ID NO: 18 is derivable from the nucleotide sequence of the cDNA represented by SEQ ID NO: 15 or 16. The nucleotide sequence encoding the amino acid sequence represented by SEQ ID NO: 18 is represented by SEQ ID NO:25. The term "derivable" as used herein means that a nucleotide sequence in question is obtained by deleting at least a part of the original DNA sequence.

Although the present gene can be isolated and identified from human spleen and mouse liver, the gene may be a DNA obtained by cloning using a genetic engineering technique such as a standard hybridization or a chemical synthetic technique such as a phosphoramidite method based on the sequence as disclosed herein. The form of the gene may be a cDNA, a genomic DNA and a chemically synthesized DNA, however not limited thereto.

The DNA of the present invention may be a single-stranded DNA. Alternatively, it may bind to a DNA or an RNA having the sequence complementary thereto to form a double- or triple-strand. The DNA may be labeled with an enzyme such as horseradish peroxidase (HRPO) ; a radioisotope; a fluorescent substance; a chemiluminescent substance; and the like.

If the nucleotide sequence of sj2368 is provided, a sequence of an RNA and a sequence of a complementary DNA and RNA are univocally determined. Therefore, it should be understood that the present invention also provides an RNA corresponding to the DNA of the present invention as well as a DNA and an RNA having a sequence complementary to the DNA of the present invention. "DNA" and "polynucleotide" are interchangeably used herein.

The DNA of the present invention also includes a DNA hybridizable with the DNA comprising the nucleotide sequence represented by SEQ ID No. 1 or 15 under stringent conditions.

Variations of the nucleotide sequence represented by SEQ ID NO:1 or 15 are acceptable as long as they are hybridizable with the DNA comprising the nucleotide sequence represented by SEQ ID NO:1 or 15 under stringent conditions and a protein encoded by said DNA is a class II cytokine receptor. It should be understood that a DNA sequence partially modified by, for example, the presence of plural codons encoding the same amino acid residue due to the degeneracy of codon; and various artificial treatments such as site-specific mutation, random mutation by treating with a mutagen, mutation, deletion, linkage and the like of the DNA fragment by cleaving with a restriction enzyme are included within the present invention as long as the DNA mutant is hybridizable with the DNA represented by SEQ ID No. 1 or 15 under stringent conditions and encodes a class II cytokine receptor even if its sequence is different from the DNA sequence represented by SEQ ID No. 1 or 15.

Variation of the nucleotide sequence represented by SEQ ID NO:24 or 25 are acceptable as long as they are hybridizable with the DNA comprising the nucleotide sequence represented by SEQ ID NO:24 or 25 under stringent conditions and a protein encoded by said DNA is the soluble SJ2368 protein.

The DNA mutant is acceptable as long as it has an identity with the DNA sequence represented by SEQ ID No. 1 or 15 or the DNA sequence of the splicing variant derivable from said sequence of at least 70%, preferably at least 80%, more preferably at least 90%. The identity in DNA sequence can be analyzed by BLAST (J. Mol. Evol., Vol. 36, pp. 290-300 (1993); J. Mol. Biol., Vol. 215, pp. 403-410 (1990)). The term "hybridizable" means that a DNA is hybridizable with the nucleic acid represented by SEQ ID No. 1 or 15 or the nucleic acid of the splicing variant derivable from said sequence by southern hybridization under stringent conditions. For example, if a probe labeled with DIG DNA Labeling kit (Cat No. 1175033 of Roche Diagnostics) is used, the hybridization is conducted in a DIG Easy Hyb solution (Cat No. 1603558 of Roche Diagnostics) at the temperature of, for example, 32°C (preferably 37°C, more preferably 42°C) and the membrane is washed in, for example, a 0.5 x SSC solution (containing 0.1% (w/w) SDS) at 50°C (preferably 65°C) (note: 1 x SSC is 0.15M NaCl and 0.015M sodium citrate).

The DNA comprising the nucleotide sequence represented by SEQ ID NO: 1 or 15, the DNA of the splicing variant derivable from said DNA, or its partial fragment is believed to be useful as a specific probe for diseases associated with the protein of the present invention such as sepatic organ disorders, autoimmune diseases, inflammatory diseases, allergic diseases, tumors and the like.

The DNA of the present invention can be used to commercially produce SJ2368. And, the DNA can be used for testing an expression status of the protein of the present invention in a tissue by labeling with an enzyme or the like. That is, an expression amount of an mRNA as an index of an expression amount of the protein of the present invention in a cell is confirmed by using the DNA as a probe so that a cell and culturing conditions of the cell suitable for the preparation of the protein of the present invention can be determined. In addition, diseases associated with the protein of the present invention such as septic organ disorders, autoimmune diseases, inflammatory diseases, allergic diseases, tumors and the like can be diagnosed.

Further, an abnormality or polymorphism on the nucleic acid sequence can be tested and/or diagnosed by any method such as PCR-RFLP (Restriction fragment length polymorphism) method, PCR-SSCP (Single strand conformation polymorphism) method, sequencing method and the like, using a part of the DNA of the present invention as a primer.

And, the DNA of the present invention can be used in gene therapy for diseases where the expression or activity of the protein of the present invention is impaired, by introducing the DNA of the present invention into *in vivo* cells.

The DNA of the present invention is very useful in the preparation of a transformant, the production of a recombinant protein SJ2368 using said transformant and the screening of a compound specifically inhibiting the expression of SJ2368.

The transformant of the present invention can be produced according to a method known for those skilled in the art. For example, the DNA of the present invention can be incorporated into a suitable host cell using any one of vectors commercially available or easily obtained by those skilled in the art. Then, the expression of the sj2368 gene within a host cell can be suitably controlled by placing the sj2368 gene under the influence of an expression control gene, typical examples of which are a promoter and an enhancer. This technique is suitable for being used in the production of SJ2368 using the transformed host cell as well as the investigation of mechanisms how to inhibit the expression of the sj2368 gene and the screening of an agent capable of inhibiting the expression of said gene.

For example, by contacting any test substances with a cell transformed with the vector including the sj2368 gene under suitable conditions, an agent capable of promoting or inhibiting the expression of the sj2368 gene can be searched among the test substances or evaluated.

By using the DNA of the present invention in combination with a known technique, a transgenic animal can be produced from a suitable animal such as mouse or the like. And, it is possible to produce the so-called knockout non-human animal in which an orthologue gene corresponding to the human sj2368 is destroyed in the animal by using the sj2368 gene of the present invention. By analyzing physical, biological, pathological and genetic characteristics of this model animal, functions of the gene and the protein of the present invention can be elucidated. Further, by introducing the human sj2368 of the present invention into an animal whose endogenous gene is destroyed, a model animal having only human sj2368 can be produced.

It becomes possible to identify functions of the gene sj2368 or the protein SJ2368 by observing the above transgenic animal, especially a transgenic animal largely expressing the gene sj2368 or the protein SJ2368 of the present invention or a transgenic animal lacking the orthologue gene of sj2368 or the protein SJ2368. This model animal is also useful for developing or evaluating an agent targeting a transferred human SJ2368, And it becomes possible to assess an agent specifically acting on the gene sj2368 or the protein SJ2368 or supplementing its functions and the like at *in vivo* level. The thus-screened agent is expected to be an agent affecting the biological control where SJ2368 functions specifically.

<Protein SJ2368>

The present invention provides the protein SJ2368 encoded by sj2368. Specifically, SJ2368 is a human class II cytokine receptor comprising the amino acid sequence represented by SEQ ID NO:2. Another embodiment of SJ2368 is a mouse class II cytokine receptor comprising the amino acid sequence represented by SEQ ID NO:14.

SJ2368 has a class II cytokine receptor motif comprising three tryptophans and two sets of S-S bond in its extracellular domain. And, it is similar to an $\alpha$-strand of IL-20 receptor, a mouse IFN $\alpha/\beta$ receptor and an IL-10 receptor. Thus, SJ2368 is assumed to be a novel receptor recognizing an IL-10 family. And, it is assumed that any tyrosine residue among three tyrosine residues in intracellular domain is phosphorylated by a ligand bond so that a JAK-STAT system is activated and the phosphorylated STAT is passed into a nucleus, thereby signals are transduced within cells. And, the class II cytokine receptor of the present invention is expressed in important organs such as liver and it is expressed in peripheral blood leucocyte, coronary arterial blood endothelial cell, B cell and T cell at cellular level. Thus, it is understood that SJ2368 is a protein participating in organ disorders such as sepsis and the like and the control of an effector function of an immuno-competent cell. The activity of SJ2368 as a class II cytokine receptor can be confirmed by any method such as the method described in Example 6.

As described in the above, SJ2368 has characteristics recognized in the class II cytokine family at high level, but its homology over the full length amino acid sequence is 21.6% or less. The homology with an IL-20 receptor is highest. This homology was determined by Maximum Matching of GENETX-WIN (Ver 4.0.5) of (Software Development Co., Ltd.). From this result, it is strongly suggested that SJ2368 plays a characteristic role which is not observed in any other class II cytokine receptor molecules in the onset or progress of an inflammatory reaction. Therefore, a pharmaceutical compound targeting SJ2368 is expected to be usable as a medicine having a new characteristic.

A polypeptide or a protein comprising an amino acid sequence wherein substitution, deletion and/or addition of one or more amino acids has occurred in the amino acid sequence of the protein represented by SEQ ID NO:2 or 14 is included within the scope of the present invention as long as it is a protein having the activity as a class II cytokine receptor.

And, the present invention provides a soluble SJ2368 protein. The soluble SJ2368 protein as used herein means a non- transmembrane protein comprising at least a part of the extracellular domain of the SJ2368 protein. Typical example of the soluble Sj2368 protein is a protein comprising the amino acid sequence represented by SEQ ID NO:20 and a protein comprising the amino acid sequence represented by SEQ ID NO:18. The protein comprising the amino acid sequence represented by SEQ ID NO:20 is a human soluble SJ2368 protein which is a splicing variant of the human SJ2368. The protein comprising the amino acid sequence represented by SEQ ID NO:18 is a mouse soluble SJ2368 protein which is a splicing variant of the mouse SJ2368. Example of other soluble SJ2368 proteins includes a protein resulting from cleaving the extracellular domain of the SJ2368 protein expressed on a cell surface. Example of another soluble SJ2368 proteins includes a fusion protein comprising a polypeptide having at least a part of the extracellular domain of the SJ2368 protein and any other polypeptide. At least a part of the extracellular domain of the SJ2368 protein as used herein may contain a class II cytokine receptor motif such as tryptophan (W) at No. 43, 68 or 111 and a pair of cysteine residues at Nos. 74 and 82, Nos. 195 and 217 in SEQ ID NO : 2 . Preferable example of at least a part of the extracellular domain of the SJ2368 protein is a region comprising the amino acid residues at Nos. 43 to 217 in SEQ ID NO: 2 and a region comprising the amino acid residues at Nos. 43 to 216 in SEQ ID NO:14.

A polypeptide or a protein comprising an amino acid sequence wherein substitution, deletion and/or addition of one or more amino acids has occurred in an amino acid sequence of the protein represented by SEQ ID NO: 20 or 18 is included within the scope of the present invention as long as it has an activity of the soluble SJ2368 protein.

Side chains of amino acid residues which are constitutional elements of a protein are different in terms of hydrophobicity, charge, size and the like, but they are known to have several highly conservative relationships such that they do not substantially affect a three-dimensional structure (also called as conformation) of the entire protein. Examples of the substitutions of amino acid residues include glycine (Gly) and proline (Pro); Gly and alanine (Ala) or valine (Val); leucine (Leu) and isoleucine (Ile); glutamic acid (Glu) and glutamine (Gln); aspartic acid (Asp) and asparagine (Asn); cysteine (Cys) and threonine (Thr); Thr and serine (Ser) or Ala; lysine (Lys) and arginine (Arg); and the like. Since Ala, Val, Leu, Ile, Pro, methionine (Met), phenylalanine (Phe), tryptophane (Trp), Gly and Cys are classified as non-polar amino acids, they are understood to have similar properties to each other. Non-charged polar amino acids include Ser, Thr, tyrosine (Tyr), Asn and Gln. Acidic amino acids include Asp and Glu. Basic amino acids include Lys, Arg and histidine (His). Even if the conservation as defined above is lost, many mutants maintaining functions essential for the protein (in the present invention, the function as the class II cytokine receptor or the soluble SJ2368 protein) are known for those skilled in the art. Further, in several similar proteins conserved between different species, it is recognized that they maintain essential functions even if several amino acids are deleted or inserted concentratedly or scatteringly.

Accordingly, a mutant protein resulting from substitution, insertion, deletion and the like of one or more amino acids in the amino acid sequence represented by SEQ ID No. 2 or 14 are included within the scope of the present invention as long as it has the activity as a class II cytokine receptor. The activity as a class II cytokine receptor as used herein is at least one of (a) an activity of binding to a ligand and (b) an activity of causing an intracellular signal transduction. These activities can be confirmed by any method such as the method described in Example 6. The intracellular signal transduction as used herein is specifically an activation of a JAK-STAT system. The activation of a JAK-STAT system can be confirmed by an activation of the gene expression by STAT or STAT-binding to a target sequence. The target sequence of STAT means a DNA sequence to which STAT is bound when said STAT is activated, one example of which is a γ-response region (GRP) of a Fc receptor I. Even if several amino acid residues are different, a protein having a function substantially similar in quality to that of SJ2368 comprising the amino acid sequence represented by SEQ ID NO: 2 or 14 is included within the scope of the present invention. Therefore, it should be understood that a change in strength of activity, a change in molecular weight of the protein due to a difference in glycosylation and the like is acceptable as long as the ability of binding to a ligand or the activity of causing an intercellular signal transduction is the same in quality. It is believed that the SJ2368 protein has the following property (a) and (b) and additionally an activity of controlling an immunological function in immuno-competent cells (especially T cells):

(a) its expression is induced by stimulating with both PMA and Ionomycine in CD3 positive peripheral blood mononuclear cells, CD4 positive peripheral mononuclear cells and CD8 positive peripheral blood mononuclear cells;
(b) its expression on CD16 positive peripheral blood mononuclear cells, CD19 positive peripheral mononuclear cells and CD33 positive peripheral blood mononuclear cells is not observed and its expression is not induced even by stimulating with both PMA and Ionomycine in these cells.

A mutant protein resulting from substitution, insertion, deletion or the like of one or more amino acids in the amino acid sequence represented by SEQ ID No. 20 or 18 are included within the scope of the present invention as long as the mutant protein has an activity as the soluble SJ2368 protein. The activity of soluble SJ2368 protein as used herein is at least one of the following (a) to (c):

(a) an activity of enhancing the production of interferon-γ (IFN-γ) on T cell;
(b) an activity of suppressing the production of IL-4 on T cell; and
(c) an activity of suppressing the proliferation of lymphocytes in a mixed lymphocyte reaction.

"An activity of enhancing" or "an activity of suppressing" as used herein means that the determined value in the presence of the soluble SJ2368 protein is different from that in the absence of the soluble SJ2368 protein in each determination system. The determined value is suitably determined depending on a system to be used. For example, the suppressing ratio or the enhancement ratio calculated by the following equation is 15% or higher, preferably 20% or higher, more preferably 30% or higher, even preferably 50% or higher.

Suppression or enhancement ratio (%) =

[an absolute value of (determined value of a group without the addition of the soluble SJ2368 protein) minus (determined value

of a group with the addition of the soluble SJ2368 protein)] /

(determined value of a group without the addition of the soluble

SJ2368 protein) x 100

When the soluble SJ2368 protein acts on T cell, the production of interferon-γ (IFN-γ) is enhanced by the T cell and the production of IL-4 is suppressed by the T cell. This event can be considered to result from the induction of Th1 differentiation by SJ2368. The activity of enhancing the production of IFN-γ by T cell, the activity of suppressing the production of IL-4 by T cell and the activity of suppressing the proliferation of lymphocytes in a mixed lymphocyte reaction can be confirmed by any method such as the method described in Example 10.

It became clear that the soluble SJ2368 is present in sera of normal subjects at a concentration of 8 to 18 ng/ml (average = 11.4 ng/ml), that the soluble SJ2368 has the tendency of showing higher value in sera of patients suffering from hepatic failure, dementia, osteoporosis, cardiac failure, pulmonary carcinoma and angina pectoris, and that the soluble SJ2368 is associated with these diseases.

The above changes in amino acids are found in the nature such as the diversity caused by a gene polymorphism or the like. Further, it can be produced artificially according to a known method for those skilled in the art, for example, mutagenesis using a mutagene such as NTG and site-specific mutagenesis using various recombinant gene techniques. The site and the number of the mutation of amino acids are not particularly limited as long as the resultant mutant protein is a class II cytokine receptor. The mutation number is generally within several tens of amino acids, preferably within 10 amino acids, more preferably within 1 or several amino acids.

In the present invention, SJ2368 can be understood as an entire molecule having all domain structures as described above. And, it can be understood as a partial peptide maintaining a functional domain having at least one of the functions of SJ2368, especially a domain participating in the bindability to a ligand. It has been reported that among transmembrane proteins, a partial peptide having a ligand binding site may present as a free (or solubilized) partial peptide, for example, by separating it from other domains while keeping its characteristic configuration. Since such a partial peptide maintains a bindability to a specific ligand, it can be possible to search compounds having a bindability to said protein using the above partial peptide. It has been known that the soluble SJ2368 protein is practically present as described above. It should be understood that a partial peptide comprising at least a part of the extracellular domain of SJ2368 is a substance substantially equivalent to the protein of the present invention as long as it has an activity equivalent to that of the above soluble SJ2368. Preferable embodiment of the partial peptide of SJ2368 includes a peptide comprising the intracellular domain of SJ2368. The intracellular domain of SJ2368 participates in a function of transducing signals within cells, one of the functions of SJ2368. Therefore, a peptide comprising the intracellular domain of SJ2368 is included within the scope of the present invention as long as it maintains a signal transducing function. Such a partial peptide may be linked to the entire or a part of any other domains (for example, a transmembrane region) of SJ2368. Alternatively, it may be in the form of a fusion protein with any other protein or peptide. The extracellular domain of the human SJ2368 comprises an amino acid sequence from N-terminal to No. 225 in the amino acid sequence represented by SEQ ID NO:2. The extracellular domain of the mouse SJ2368 comprises an amino acid sequence from N-terminal to No. 224 in the amino acid sequence represented by SEQ ID NO:14. It is considered that a ligand binds to this domain. And, the intracellular domain of the human SJ2368 comprises an amino acid sequence from No. 253 to C-terminal in the amino acid sequence represented by SEQ ID NO:2. The intracellular domain of the mouse SJ2368 comprises an amino acid sequence from No. 253 to C-terminal in the amino acid sequence represented by SEQ ID NO: 14. It is considered that signals are transduced within cells through this domain. The length of amino acid residues in each domain may vary slightly depending on the method predicting a domain to be used, for example, the difference of a program predicting a signal sequence or a program predicting a transmembrane region, but the difference in length should be acceptable. The change in the amino acid sequence is acceptable as long as it maintains an activity as the domain.

Thus, the present invention provides the following peptides:

(a) a polypeptide comprising the amino acid sequence at Nos. 21 to 225 in the amino acid sequence represented by SEQ ID NO:2;
(b) a human SJ2368 extracellular domain comprising an amino acid sequence wherein deletion, substitution or addition of one or more amino acids has occurred in the amino acid sequence of the polypeptide as defined in (a);
(c) a polypeptide comprising the amino acid sequence at Nos. 21 to 224 in the amino acid sequence represented by SEQ ID NO:14;
(d) a mouse SJ2368 extracellular domain comprising an amino acid sequence wherein deletion, substitution or addition of one or more amino acids has occurred in the amino acid sequence of the polypeptide as defined in (c);
(e) a polypeptide comprising the amino acid sequence at Nos. 253 to 520 in the amino acid sequence represented by SEQ ID NO:2;
(f) a human SJ2368 intracellular domain comprising an amino acid sequence wherein deletion, substitution or addition of one or more amino acids has occurred in the amino acid sequence of the polypeptide as defined in (e);
(g) a polypeptide comprising the amino acid sequence at Nos. 253 to 535 in the amino acid sequence represented by SEQ ID NO: 14; (h) a mouse SJ2368 intracellular domain comprising an amino acid sequence wherein deletion, substitution or addition of one or more amino acids has occurred in the amino acid sequence of the polypeptide as defined in (g).

A DNA encoding the above partial peptide is included within the scope of the DNA of the present invention. The DNA encoding the partial peptide is derivable from the DNA represented by SEQ ID NO:1 or 15.

A fusion protein comprising the extracellular domain of SJ2368, a fusion protein comprising the intracellular domain of SJ2368 and a fusion protein comprising the soluble SJ2368 are included within the scope of the present invention. Other polypeptide to be linked is not particularly limited as long as the resultant fusion protein has at least one activity among the activities of SJ2368 or soluble SJ2368. Example of the polypeptide to be linked includes an extracellular or intracellular domain of a class II cytokine receptor family, a Fc fragment of immunoglobulin (sometime abbreviated to "Fc") and the like. In case of IgG, the Fc fragment comprises a hinge region, a CH2 region and a CH3 region. A part of the Fc fragment such as a hinge region, a CH2 region, a CH3 region or a CH4 region or any combination thereof is usable. A source of the immunoglobulin is not particularly limited, but the immunoglobulin from human is preferable. A class and a subclass are not always limited. IgG1, IgG2, IgG3, IgG4, IgA1, IgA2, IgM, IgE, IgD and the like are usable. For example, IgG is preferable. Preferable example of the fused protein includes a fusion protein comprising the extracellular domain of the mouse SJ2368 and Fc represented by SEQ ID NO:17 and a fusion protein comprising the extracellular domain of the human SJ2368 and Fc represented by SEQ ID NO:19. Other example of a polypeptide to be linked includes an $\alpha$-strand of IL10 receptor and the example of a fusion protein comprising the extracellular domain of an $\alpha$-strand of IL10 receptor and the intracellular domain of SJ2368 is represented by SEQ ID: 22 and 23.

A DNA encoding the above fusion protein is included within the DNA of the present invention.

In a protein having a signal sequence, it may function as a maturation protein when the signal sequence is cleaved. Thus, it should be understood that a maturation peptide resulting from the cleavage of a signal sequence from the protein of the present invention is substantially a substance equivalent to the protein of the present invention. In case of the human SJ2368, it is predicted that a signal sequence will be present around the amino acid residues at Nos. 8 to 20 in the amino acid sequence represented by SEQ ID NO: 2. The aforementioned protein may be in the form of conventional salts.

The protein of the present invention or its partial peptide can be used in screening for an agent capable of controlling an activity of said protein. The thus-screened compounds and the like are expected to be useful as an effective therapeutic or preventive agent for diseases associated with the protein of the present invention such as septic organ disorders, autoimmune diseases, inflammatory diseases, allergic diseases, tumors and the like.

<Antibody>

Further, the present invention provides an antibody binding to SJ2368. The antibody of the present invention is an antibody specifically recognizing the entire SJ2368 or its partial peptide as an antigen. It includes a monoclonal antibody and/or a polyclonal antibody. And, it may be an antibody belonging to any one of five classes (IgG, IgA, IgM, IgD and IgE) classified by structure, physical-chemical properties and immunological properties of immunoglobulins or either subclass classified by the type of H chain. Further, it may be a fragment such as $F(ab')_2$ produced by digesting an immunoglobulin with, for example, pepsin, Fab produced by digesting an immunoglobulin with papain and the like, or a chimeric antibody and a humanized antibody. In addition, an antibody having functions of not also specifically recognizing SJ2368 or its partial peptide as an antigen but also controlling the activity of SJ2368 is also included within the scope of the present invention. An antibody having the function of controlling the activity of SJ2368 means a neutralizing antibody inhibiting the binding of SJ2368 to a ligand, a neutralizing antibody inhibiting the homo- or hetero-oligomeriziation by binding to SJ2368, or an agonist antibody having an activity of causing an intercellular signal transduction by binding to SJ2368. These antibodies are useful in investigational or clinical detection of SJ2368 and the like.

<Antisense nucleic acid>

The present invention provides the so-called antisense nucleic acid capable of inhibiting the biosynthesis of SJ2368 at a nucleic acid level *in vivo.* The antisense nucleic acid means a nucleic acid which binds to DNA or RNA involved in carrying a genetic information during either of a transcription stage from a genome region to a pre-mRNA essential for the production of mRNA encoding SJ2368, a processing stage from the pre-mRNA to a mature mRNA, a stage of passing through a nuclear membrane or a translation stage into a protein so as to affect the normal stream of the transmission of the genetic information and thereby to inhibit the expression of the protein. It may comprises a sequence complementary to the entire or either part of the nucleic acid sequence of the gene SJ2368. A preferable nucleic acid (including DNA and RNA) comprises a sequence corresponding to or complementary to the entire or a part of the nucleic acid sequence represented by SEQ ID NO: 1 or 3. Another preferable nucleic acid (including DNA and RNA) comprises a sequence corresponding to or complementary to the entire or a part of the nucleic acid sequence represented by SEQ ID NO: 15 or 16. When the mRNA transcripted from the genome region contains an intron structure or a non-coding region at 5' or 3'-terminal, an antisense nucleic acid corresponding to or complementary to the sequence of the non-coding region will have functions equivalent to those of the antisense nucleic acid of the present invention.

The antisense nucleic acid of the present invention includes a DNA and an RNA as well as all of derivatives similar to the DNA and the RNA in configuration and functions. Example of the antisense nucleic acid includes a nucleic acid having any other substance bound at 3'- or 5'-terminal, a nucleic acid wherein at least one of bases, sugars and phosphates of the oligonucleotide is substituted or modified, a nucleic acid having a non-naturally occurring base, sugar or phosphate, a nucleic acid having a backbone other than the sugar-phosphate backbone and the like. These nucleic acids are suitable as derivatives, in which at least one of a nuclease resistance, a tissue selectivity, a cell permeability and a binding power is improved. That is, the form of the nucleic acid is not limited as long as the nucleic acid can inhibit the activity and the expression of SJ2368.

And, the antisense nucleic acid having a nucleotide sequence complementary to the nucleotide sequence hybridizable with a loop portion of mRNA forming a stem loop, i.e. the nucleotide sequence of a region forming a stem loop is generally preferable in the present invention. Alternatively, an antisense nucleic acid capable of binding to near a translation initiation codon, a ribosome binding site, a capping site and a splicing site, i.e. an antisense nucleic acid having a sequence complementary to that of these sites is also preferable since generally it can be expected to be very effective in inhibiting the expression.

In order to make the above antisense nucleic acid introduced into a cell and act efficiently, it is preferable that the length of the antisense nucleic acid of the present invention is 15 to 30 bases, preferably 15 to 25 bases, more preferably 18 to 22 bases.

The effect of the antisense nucleic acid of the present invention in the inhibition of the expression can be evaluated by a known method, for example, by preparing an expression plasmid by linking a reporter gene such as luciferase and the like to the DNA containing a part of an expression control region, a 5'-un-translated region, a region near a translational initiation site or a translated region of the gene of the present invention, adding a test substance in a system such as a system comprising *in vitro* transcription (Ribo max systems; Promega) combined with *in vitro* translation (Rabbit Reticulocyte Lysate Systems; Promega) under the condition where the gene of the present invention is transcripted or transplanted and determining an expression amount of the reporter gene.

The antisense nucleic acid of the present invention is useful as a preventive or therapeutic agent for diseases associated with SJ2368 since it can inhibit the expression of sj2368 *in vivo*.

BRIEF DESCRIPTION OF DRAWINGS

Fig. 1 is the scheme showing characteristics of the primary structure of SJ2368.

Fig. 2 shows the expression profile of the sj2368 gene in human various organs and cells.

Fig. 3 is the western blots of human SJ2368-His and human SJ2368-Fc.

Fig. 4 is the scheme showing the gene structure of human sj2368. The heavy line represents an exon. Arabic numerals thereon is the number of bases present between position numbers of SEQ ID NO:3. Roman numerals is a number of an exon. The numerical value between exons represents the length of an intron.

Fig. 5 is the scheme showing the gene structure of human IL10Rα chain gene. The line, the numerical value and the like have the meanings identical with that described in relation to Fig. 4.

Fig. 6 is the summary of the results of in silico cloning (5'-side) of mouse sj2368.

Fig. 7 is the summary of the results of in silico cloning (3'-side) of mouse sj2368.

Fig. 8 shows the amino acid alignment between human SJ2368 and mouse SJ2368. The upper row shows the amino acid sequence of human SJ2368 and the lower row shows the amino acid sequence of mouse SJ2368. A boxed domain, "-" and a vertical line represent a transmembrane domain, a gap sequence and a break of exons, respectively. A portion underlined in the mouse sequence is a deletion by alternative splicing.

Fig. 9 is the scheme of the gene structures of human sj2368 and mouse sj2368. The line, the numerical value and the like have the meanings identical with that described in relation to Fig. 4.

Fig. 10 is the western blot of mouse SJ2368-Fc. M, 1 and 2 represent elecrophoresed lanes of a size marker, a supernatant of a transfectant in which mouse SJ2368-Fc was expressed and a supernatant of a transfectant of a vector plasmid.

Fig. 11 shows the effect of mouse SJ2368-Fc on mouse MLR. Relative ratio = (BrdU incorporated upon culturing in the presence of the protein) / (BrdU incorporated upon culturing in the absence of the protein) x 100
The white bar, the gray bar and the black bar each represents the value when 1 μg/ml, 3 μg/ml and 10 μg/ml of the protein was added. CONT-Fc is a chimeric protein comprising an extracellular domain of other I type transmembrane protein molecule and a human IgG1 Fc domain.

Fig. 12 shows the results when the specificity of the mouse and rat monoclonal antibodies F1157-10-2, F1120-21-3 by the western blotting method. The full length is a sample from a cell expressing SJ2368, the soluble is a sample from a cell expressing the soluble SJ2368 (splicing variant) and the vector is a sample from cells in which a vector plasmid was transfected.

Fig. 13 is the standard curve of SJ2368-His as determined in the sandwich ELISA system.

Fig. 14 is the concentration of the soluble SJ2368 protein in culture supernatants of various cell lines as determined in ELISA system.

Fig. 15 is the concentration of the soluble SJ2368 protein in sera of patients suffering from various diseases and healthy subjects as determined in ELISA system.

Fig. 16 shows the results from staining a COS transfectant with the OG labeled F1120-21-3 and then analyzing by flow cytometry. The solid line is the staining result of a transfectant in which a SJ2368 expression plasmid was transfected and the dotted line is the staining result of a transfectant in which a vector plasmid was transfected.

Fig. 17 shows the analytical results of the expression of a chimeric receptor comprising IL10R α-strand and SJ2368 by flow cytometry.

A: The solid line represents the result of the staining of a HEK293 transfectant in which a IL10R α-chain expression plasmid was transfected with a PE labeled anti-IL10R antibody. The dotted line represents the result of the staining of a HEK293 transfectant in which a vector plasmid was transfected with a PE labeled anti-IL10R antibody.
B: The solid line represents the result of the staining of a HEK293 transfectant in which a CR10A expression plasmid was transfected with a PE labeled anti-IL10R antibody. The dotted line represents the result of the staining of a HEK293 transfectant in which a vector plasmid was transfected with a PE labeled anti-IL10R antibody.
C: The solid line represents the result of the staining of a HEK293 transfectant in which a CR10B expression plasmid was transfected with a PE labeled anti-IL10R antibody. The dotted line represents the result of the staining of a HEK293 transfectant in which a vector plasmid was transfected with a PE labeled anti-IL10R antibody.
D: The solid line represents the result of the staining of a HEK293 transfectant in which a SJ2368 expression plasmid was transfected with a PE labeled anti-IL10R antibody. The dotted line represents the result of the staining of a HEK293 transfectant in which a vector plasmid was transfected with a PE labeled anti-IL10R antibody.

## BEST MODE FOR CARRYING OUT THE INVENTION

<Nucleic acid>

Example of the method for obtaining the DNA of the present invention from a DNA library includes a method comprising screening a suitable genomic DNA library or cDNA library according to a screening method such as a screening method via hybridization, an immunoscreening method using an antibody and the like, amplifying a clone having the desired DNA and digesting with a restriction enzyme or the like. In the screening method via hybridization, the hybridization can be conducted for any cDNA library or a genomic DNA library using the DNA having the nucleotide sequence represented by SEQ ID No. 1 or 15 or a part thereof labeled with $^{32}P$ or the like as a probe according to a known method (see, for example, T. Maniatis, et al., Molecular Cloning, a Laboratory Manual, Cold Spring Harbor Laboratory, New York (1982)). The antibody used in the immunoscreening method may be the antibody of the present invention as described below. The novel DNA of the present invention may be also obtained by PCR (Polymerase Chain Reaction) using a genomic DNA library or a cDNA library as a template. The PCR is performed for any DNA library according to a known method (see, for example, A.I. Michael, et al., PCR Protocols, a Guide to Methods and Applications, Academic Press (1990)) using sense and antisense primers synthesized based on the nucleotide sequence of SEQ ID NO: 1, thereby the DNA of the present invention can be obtained. As the DNA library used in the above methods, a DNA library having the DNA of the present invention is selected and used. Any DNA library can be used as long as it comprises the DNA of the present invention. A commercially available DNA library may be also used. Alternatively, a cDNA library may be constructed according to a known method (see J. Sambrook et al., Molecular Cloning, a Laboratory Manual, 2nd ed., Cold Spring Harbor Laboratory, New York (1989)) by selecting cells suitable for the construction of the cDNA library from cells having the DNA of the present invention.
And, the DNA of the present invention can be prepared based on the sequence as disclosed herein by a chemical synthetic technique such as a phosphoramidite method and the like.
The recombinant vector including the DNA of the present invention may have any form such as a circular form or a linear form. The recombinant vector may have any other nucleotide sequence in addition to the entire or a part of the DNA of the present invention, if necessary. The other nucleotide sequence includes an enhancer sequence, a promoter sequence, a ribosome binding sequence, a nucleotide sequence used for amplifying the number of copies, a nucleotide sequence encoding a signal peptide, a nucleotide sequence encoding any other polypeptide, a poly adenylation signal, a splicing sequence, a replication origin, a nucleotide sequence of the gene acting as a selective marker and the like. One preferable example of the recombinant vector of the present invention is an expression vector.
In the gene recombination, it is possible to add a translational initiation codon or a translational stop codon to the DNA of the present invention using a suitable synthetic DNA adaptor, or to newly produce or delete a suitable restriction site within the nucleotide sequence. This is the technique routinely conducted by those skilled in the art. Such a processing can be suitably and easily conducted based on the DNA of the present invention.
As the vector including the DNA of the present invention, a suitable vector is selected and used depending on the type of a host used. The vector may be a plasmid. Alternatively, various viruses may be used, non-limiting examples of which include bacteriophage, baculovirus, retrovirus, vaccinia virus and the like.
The gene of the present invention can be expressed under the control of a promoter sequence inherent in said gene. Using the expression system, an agent promoting or suppressing the transcription of the gene of the present invention can be efficiently screened. Any other suitable expression promoter can be used by linking it to the promoter sequence inherent in said gene upstream of the gene of the present invention or replacing it with the promoter sequence. In this case, the promoter may be suitably selected depending on a host or an object of expression. For example, if a host is E. coli, a T7 promoter, a lac promoter, a trp promoter, a λPL promoter or the like can be used. If a host is a yeast, a PHO5 promoter, a GAP promoter, an ADH promoter or the like can be used. If a host is an animal cell, a promoter from SV 40, a retrovirus promoter, an elongation factor 1α promoter or the like can be used. These lists are not exclusive.

A method of introducing the DNA into a vector is known (see J. Sambrook et al., Molecular Cloning, a Laboratory Manual, 2nd ed., Cold Spring Harbor Laboratory, New York (1989)). That is, each of the DNA and the vector is digested with suitable restriction enzymes and the resultant fragments are ligated with a DNA ligase.

<Protein>

The protein of the present invention can be prepared from various cells and tissues expressing said protein. Alternatively, it can be chemically synthesized in a peptide synthesizer (for example, Peptide Synthesizer Model 433A; Applied Biosystems Japan) or it can be produced by recombination method using a suitable host cell selected from prokaryotic and eukaryotic cells. However, a genetic engineering technique and a recombinant protein produced thereby are preferable in view of purity.

A host cell to be transformed with the recombinant vector described in the previous section is not limitative. Many cells of low organisms available in genetic engineering techniques, typical examples of which are E. coli, B. subtilis and S.cerevisiae; and animal cells, typical examples of which are insect cell, COS7 cell, CHO cell and HeLa cell, can be used in the present invention.

The transformant of the present invention can be obtained by transforming a suitable host cell with the recombinant vector of the present invention. As the method of introducing the recombinant vector described in the previous section into a host cell, some methods are known such as an electroporation, a protoplast method, an alkali metal method, a calcium phosphate precipitation method, a DEAE dextran method, a microinjection method, a method using virus particles and the like (see "Handbook of Genetic Engineering", Special Issue of Experimental Medicines, published by Yodosha Co., Ltd. (March 20, 1991)). Either method may be used.

For preparing the present protein by a genetic engineering technique, the above transformant is cultured to collect a culture mixture followed by purifying the protein. The transformant can be cultured according to a standard method. Many textbooks are available, for example, "Experimental Procedures in Microbiology", edited by The Japanese Biochemical Society, published by Tokyo Kagaku Dozin Co., Ltd. (1992)) describing the culture of transformants, for reference.

As a method for purifying the protein of the present invention from the culture mixture, a suitable method is selected among conventional methods for purifying proteins. The conventional methods include salting out, ultrafiltration, isoelectric precipitation, gel filtration, electrophoresis, ion-exchange chromatography, hydrophobic chromatography and various affinity chromatographies including antibody chromatography, chromato-focusing, adsorption chromatography, reverse phase chromatography and the like. If necessary, HPLC systems or the like may be used to conduct several purification methods in a suitable order.

It is possible to express the protein of the present invention as a fusion protein with any other protein such as a Fc fragment of immunoglobulin or any tag such as glutathione S transferase, protein A, hexahistidine tag, FLAG tag and the like. The thus-expressed fusion protein may be cleaved with a suitable protease such as thrombin, enterokinase and the like. This may be more effective for the preparation of the protein. When a Fc fragment of immunoglobulin is used, the activity or the function of the protein of the present invention can be increased or modified by, for example, imparting an ability of forming a dimmer. For purifying the protein of the present invention, conventional methods may be suitably combined. Especially if the protein is expressed in the form of a fusion protein, it is preferable to purify according to a method characteristic to such a form.

One of methods for preparing the present protein by a genetic engineering technique is the synthesis of a cell-free system using a recombinant DNA molecule (J. Sambrook et al., Molecular Cloning, 2nd ed. (1989)).

As mentioned above, the protein of the present invention can be prepared in the form of a single protein or a fusion protein with any other different protein. The form of the protein of the present invention is not limited to them. Further, it is possible to transform the protein of the present invention to various forms. For example, it is thought that the protein can be processed according to various methods known for those skilled in the art such as various chemical modifications for the protein, the binding of the protein to a polymeric substance such as polyethylene glycol and the like, the binding of the protein to an insoluble carrier and the like. And, the presence or absence of glycosylation or the difference in glycosylation degree is found depending on a host cell used. It should be understood that all of the above proteins are included within the scope of the present invention as long as said proteins have an ability of binding to a ligand or an activity of causing an intercellular signal transduction as SJ2368.

The protein of the present invention is useful since it can be used as an antigen for the production of an antibody or it can be used for screening for an agent capable of binding to said protein or an agent capable of controlling an activity of said protein.

SJ2368 of the present invention can highly express a desired molecule on a surface of the above transformant, especially an animal cell, by culturing the cell. When a suitable fragment of SJ2368 such as an extracellular region protein fragment thereof is prepared as a soluble protein, such a fragment can be prepared by preparing a transformant using a DNA encoding the extracellular region or each domain according to the above method and culturing the resultant transformant so as to secret it in the culture supernatant.
On the other hand, when SJ2368 is present in a periplasm or cytoplasm of a transformant, the transformant suspended in a suitable buffer is treated by an ultrasonic treatment, a freeze-thawing treatment, a treatment with lysozyme or the like to destroy a cell wall and/or a cell membrane and further treated by a centrifugation, a filtration or the like to obtain a membrane fraction containing the protein of the present invention. This fraction is solubilized using a suitable surfactant to prepare a crude solution. Thereafter the desired protein can be isolated and purified from the crude solution according to a routine method.

<sj2368 gene recombinant non-human animal>

The present invention provides a sj2368 gene recombinant non-human animal. The sj2368 gene recombinant non-human animal includes transgenic non-human animals and knock out non-human animals. In the transgenic non-human animal of the present invention, the expression of the protein of the present invention can be controlled in terms of its level, its time, its sites and the like by artificially inserting the gene encoding said protein on a chromosome of the animal. Non-limiting examples of a non-human animal include bovine, goat, sheep, pig, mouse, horse, chicken and the like. Among the non-human animals, a non-human mammalian animal is preferable.
By using sj2368 of the present invention, a transgenic non-human mammalian animal can be produced. The transgenic non-human mammalian animal is included within the scope of the present invention. The transgenic non-human mammalian animal can be produced according to a routine method conventionally used in the production of transgenic animals (see, for example, "Experimental Manual of Genesis, published by Kodansha Scientific Ltd., edited by Motoya KATSUKI under supervision of Tatsuj i NOMURA (1987)). That is, the gene or the recombinant vector of the present invention is introduced into a totipotent cell of a non-human animal to produce subjects and thereafter only a subject in which the gene introduced is incorporated in a genome of a somatic cell is selected.
Specifically, in case of a transgenic mouse, a DNA prepared such that the sj2368 gene can be expressed is directly poured into a pronucleic oosperm obtained from a normal C57Black/6 mouse. More specifically, a construct is prepared by introducing the sj2368 gene downstream of a suitable promoter by linking. Thereafter, a linear DNA is obtained by removing the sequence from a prokaryote as much as possible, if necessary. This DNA is directly poured into a pronucleus of the pronucleic oosperm using a fine glass needle.
The oosperm is transplanted in an uterus of another pseudopregnant mouse as an allomother. The pseudopregnant mouse is generally prepared by mating an ICR female mouse with a vasectomized or vasoligated male mouse. A genomic DNA is extracted from a tissue from the transplated embryo and confirmed whether the sj2368 gene is introduced or not by PCR or southern blotting, thereby a transgenic mouse is obtained.
The so-called "knockout mouse" can be produced based on the nucleotide sequence of sj2368 (or a mouse homologous gene of sj2368). The term "knockout mouse" used herein means a mouse in which an endogenous gene encoding the protein of the present invention is knocked out (inactivated) . The knockout mouse can be produced by, for example, a positive-negative selection method via homologous recombination (see, for example, US patent Nos. 5,464,764, 5,487,992 and 5,627,059; Proc. Natl. Acad. Sci. USA, Vol. 86, pp. 8932-8935 (1989); Nature, Vol. 342, pp. 435-438 (1989)). Such a knockout mouse is one embodiment of the present invention.
Recently, the production of clone animals by nuclear transplantation in medium or large animals became possible. In this connection, transgenic and knockout animals have been practically produced using this technique. That is, a somatic cell or a germinal cell is subjected to homologous recombination based on the nucleotide sequence of sj2368 (or a homologous gene of sj2368 in each animal) in the same way as that applied to ES cells and then a nucleus is obtained from the resultant cell and used to obtain a clone animal. This animal is a knockout animal in which sj2368(or a homologous gene of sj2368 in each animal) is lost. Or, sj2368 (or a homologous gene of sj2368 in each animal) is introduced in any cell of any animal and then the resultant nucleus is used to obtain a clone animal, thereby a transgenic animal can be produced. Such a knockout non-human animal and a transgenic non-human animal are one embodiment of the present invention irrespective of its species.

<Antibody>

The antibody of the present invention may be polyclonal or monoclonal. Either antibody can be obtained by referring to a known method (see, for example, "Experimental Procedures in Immunology", edited by Japan Society for Immunology, published by Japan Society for Immunology), as describe below in brief.
For obtaining the novel antibody, an animal is inoculated with the protein of the present invention as an immunogen and if necessary a suitable adjuvant such as Freund's complete adjuvant (FCA), Freund's incomplete adjuvant (FIA) and the like. If necessary, a booster at an interval of 2 to 4 weeks may be conducted. After the booster, an anti-serum is obtained by taking a blood sample. The protein of the present invention used as an antigen is that obtained in any method as long as it has a purity sufficient to be usable in the production of an antibody. A partial polypeptide of the protein of the present invention may be suitably used as an immunogen. If the polypeptide used as an immunogen is a low-molecular weight polypeptide, i.e. a polypeptide comprising about 10 to 20 amino acids, it may be linked to a carrier such as keyhole limpet hemocyanin (KLH) and the like and used as an antigen. Animals to be immunized is not particularly limited, but it is preferable to use by selecting an animal species capable of producing the objective antibody among animals conventionally used in immunological experiments by those skilled in the art such as rat, mouse, rabbit, sheep, horse, chicken, goat, pig, cattle and the like.

A polyclonal antibody can be obtained by purifying the resultant anti-serum. The purification may be conducted by suitably combining known methods such as salting-out, ion exchange chromatography, affinity chromatography and the like.

A monoclonal antibody is obtained as follows: An anti-body-producing cell such as a splenic cell, a lymphocyte and the like is taken from an immunized animal. The cell is fused with a myeloma cell strain or the like according to a known method using polyethylene glycol, Sendai virus, an electric pulse or the like to produce a hybridoma. Thereafter, a clone producing an antibody which binds to the protein of the present invention is selected and cultured. By purifying a supernatant of the culture of the selected clone, a monoclonal antibody is obtained. The purification may be conducted by suitably combining known methods such as salting-out, ion exchange chromatography, affinity chromatography and the like.

The antibody is also obtained by a genetic engineering technique. For example, an mRNA is obtained from a splenic cell or a lymphocyte of an animal immunized with the protein of the present invention or its partial polypeptide or from a hybridoma producing a monoclonal antibody against the protein of the present invention or its partial polypeptide. Based on the thus-obtained mRNA, a cDNA library is constructed. A clone producing the antibody which reacts with the antigen is screened and the thus-screened clone is cultured. The objective antibody can be purified from the culture mixture by combined known methods. When the antibody is used for therapy, a humanized antibody is preferable with respect to immunogenicity. The humanized antibody can be prepared by immunizing a mouse whose immune system has replaced with a human immune system (see, for example, Nat. Genet., Vol. 15, pp. 146-157 (1997)). Alternatively, the humanized antibody can be engineered using hypervariable regions of the monoclonal antibody (Method in Enzymology, Vol. 203, pp. 99-121 (1999)).

<Antisense nucleic acid>

The antisense nucleic acid can be prepared according to a known method (see, for example, edited by Stanley T. Crooke and Bernald Lebleu, in Antisense Research and Applications, published by CRC Publisher, Florida (1993)). If DNA and RNA are native, the antisense nucleic acid of the present invention can be obtained by synthesizing in a chemical synthesizer or conducting a PCR using sj2368 as a template. Alternatively, a part of derivatives such as methyl phosphonate type and phosphorothioate type can be synthesized in a chemical synthesizer (for example, Expedite Model 8909; Applied Biosystems Japan). Then, such a derivative may be synthesized according to a manual attached to the chemical synthesizer and the thus-synthesized product may be purified by HPLC using a reverse phase chromatography or the like, thereby the antisense nucleic acid can be obtained.

When the DNA and the antisense nucleic acid of the present invention is used as a diagnostic probe, they are labeled with a radioisotope, an enzyme, a fluorescent substance, a luminescent substance or the like according to a known method. Subsequently, a DNA or mRNA is prepared from a specimen according to a known method and it is used as a test substance. This test substance is reacted with the labeled probe and then the product is washed to remove the labeled probe unreacted. If the test substance contains the gene sj2368 or RNA, said antisense nucleic acid binds thereto. The presence or absence of the binding formation can be known by using a luminescence, a fluorescent, a radioactivity or the like from the enzyme, a fluorescent substance or a luminescent substance labeled; or a radioisotope as an index.

When the DNA, the antisense nucleic acid or the recombinant vector of the present invention is used in clinical applications, it is preferable to use those having a purity suitable for the use of a medicine according to any pharmaceutically acceptable method.

The DNA, the antisense nucleic acid or the recombinant vector of the present invention may be used by directly dissolving or suspending in a suitable solvent. Alternatively, it may be used after encapsulating in a liposome or introducing in a suitable vector. If necessary, it may be used in a suitable dosage form such as injections, tablets, capsules, eye drops, creams, suppositories, spray, poultices in which pharmaceutically acceptable adjuvants are added. Examples of the pharmaceutically acceptable adjuvants are a solvent, a base, a stabilizer, a preservative, a solubilizing agent, an excipient, a buffer and the like.

When the DNA, the antisense nucleic acid or the recombinant vector of the present invention is used in the above dosage form, its administration method and its dose can be selected depending on the age and the sex of a patient, the type and the severity of the disease. Thus, it may be administered in an amount suitable to improve pathological conditions by the suitable method selected from oral, inhalation, transdermal, eye dropping, intravaginal, intraarticular, intrarectal, intravenous, local, intramuscular, subcutaneous and intraperitoneal administrations.

<Screening method>

The present invention relates to a method of screening for an agent capable of controlling the function or the expression of the protein of the present invention, which comprises using the protein of the present invention, a transformant expressing said protein, the DNA of the present invention, a recombinant vector including said DNA, a transformant produced by transforming with said vector or a sj2368 gene recombinant non-human mammalian animal.
More specifically, the screening method includes:

(1) a method comprising providing a candidate and contacting the candidate with the protein of the present invention or the transformant expressing said protein so as to judge whether or not the candidate has a function of controlling the activity of the protein of the present invention;
(2) a method comprising providing a candidate and contacting the candidate with a recombinant vector including the DNA of the present invention or the transformant produced by transforming with said recombinant vector so as to judge whether or not the candidate has a function of controlling the expression of the sj2368 gene;
and the like. Example of the method (1) includes a method comprising determining the activity of the protein of the present invention in the presence/absence of a candidate in a system as illustrated in Example 6 and selecting an agent capable of increasing or lowering the activity of the protein of the present invention rather than the absence of the candidate. Example of the method (2) is a method comprising preparing an expression plasmid prepared by linking a reporter gene such as luciferase or the like to the DNA containing an expression control region, a 5'-non-coding region, a region near a translational initiation site or a part of a translation region of the sj2368 gene and determining an expression amount of the reporter gene under the condition where the gene of the present invention is transcripted or translated in the presence/absence of a candidate so as to confirm a transcriptional promotion activity or a transcriptional inhibition activity of the candidate. The screening method of the present invention comprises the steps of contacting a candidate with the protein of the present, a transformant expressing said protein, the DNA of the present invention, a recombinant vector including said DNA, a transformant produced by transforming with said recombinant vector or a sj2368 gene recombinant non-human animal; detecting a difference in the activity of the protein of the present invention or the expression level of the DNA of the present invention between a group with the addition of the candidate and a group without the addition of the candidate; and selecting the candidate showing the difference as an agent capable of controlling an activity of the protein of the present invention or an agent capable of controlling the expression of the DNA of the present invention.

An agent capable of controlling the activity of the protein of the present invention may be an agent capable of enhancing (agonist) or inhibiting (antagonist) the activity of the SJ2368 protein and/or the soluble SJ2368 protein. An antagonist is preferable. An agent capable of controlling the expression of the DNA of the present invention may be an agent capable of either promoting or inhibiting the expression of the gene sj2368. An agent capable of inhibiting the expression is preferable. For confirming whether a candidate controls the activity of the protein of the present invention or controls the expression of the DNA of the present invention, a difference in the activity of the protein or the expression level of the DNA is determined between the addition and no addition of a candidate in a system capable of confirming the activity of the protein or a system capable of confirming the expression of the DNA. The expression level of the DNA may be detected on the basis of an expression intensity of the sj2368 gene into mRNA or the protein. Instead of the expression level of the sj2368 gene or the SJ2368 protein per se, an expression level of a reporter gene may be detected. The reporter-assay system means an assay system in which an expression amount of a reporter gene arranged downstream of a transcriptional control region is determined so as to screen for an agent affecting the transcriptional control region. Examples of the transcriptional control region include a promoter, an enhancer, a CAAT box, a TATA box and the like generally found in a promoter region. As a reporter gene, a CAT (chloramphenicol acetyl transferase) gene, a luciferase gene, a β-galactosidase gene and the like can be used. The expression control region and the 5'-untranslated region of the gene of the present invention can be obtained according to a known method (see "New Experimental Protocol in Cell Engineering", published by Shujunsha Co., Ltd. (1993)). Having function of inhibiting (or suppressing) or enhancing (or promoting) means that a determined value with respect to the activity of the protein or the expression level of the DNA is different between a group with the addition of a candidate and a group without the addition of a candidate. For example, the inhibition (or suppression) or the enhancement (or promotion) ratio calculated by the following equation is 10% or higher, preferably 30% or higher, more preferably 50% or higher, even preferably 70% or higher, especially preferably 90% or higher.

inhibition (or suppression) or enhancement (or promotion) ratio (%) = [an absolute value of (determined value of a group without the addition of a candidate) minus (determined value of a group with the addition of a candidate) ] / (determined value of a group without the addition of a candidate) x 100

Either inhibition or enhancement is suitably determined depending on the kind of a system capable of confirming the activity of the protein or a system capable of confirming the expression of the DNA. The determined value is the same. For example, if a system capable of confirming the activity of the protein is a luciferase system as illustrated in Example 6, luciferase activity can be determined. When the determined value in a group with the addition of a candidate is lower than that in a group without the addition of a candidate, the candidate can be judged to have a function of inhibiting the activity of the SJ2368 protein. If a system capable of confirming the activity of the protein is a system of determining the production of IFN-γ by T cell as illustrated in Example 10, an amount of IFN-γ produced can be determined. When the determined value in a group with the addition of a candidate is lower than that in a group without the addition of a candidate, the candidate can be judged to have a function of inhibiting the activity of the soluble SJ2368 protein. Of course, if values from background and/or noises is contained in a determination system, they should be subtracted.

Compounds obtained through the search using the screening method or the transgenic animal described above are expected to be effective therapeutic or preventive agents for diseases such as septic organ disorders, autoimmune diseases, inflammatory diseases, allergic diseases, tumours and the like. Non-limiting examples of a candidate include proteins, peptides, oligonucleotides, synthetic compounds, naturally occurring compounds, fermented products, cell extracts, plant extracts, animal tissue extracts and the like. The test substance may be either new or known.

<Assay method, reagent and kit using the present antibody>

The present invention provides

(1) an assay method for the soluble SJ2368 protein in a test sample which comprises using the antibody of the present invention;
(2) an assay reagent or kit for the soluble SJ2368 protein in a test sample which comprises containing the antibody of the present invention ;
(3) an assay method for the soluble SJ2368 protein as mentioned in (1) which comprises determining the increase or decrease of an amount of the soluble SJ2368 protein in a human body fluid to predict, detect or diagnose a dysfunction of SJ2368, diseases accompanied with the dysfunction or conditions associated with the diseases; and
(4) an assay method as mentioned in (3) wherein the disease is at least one disease selected from hepatic failure, dementia, osteoporosis, cardiac failure, pulmonary carcinoma and angina pectoris.

The assay method of the present invention comprises a step of using the antibody of the present invention. Preferably this step is a step comprising trapping a test substance, i.e. the soluble SJ2368 protein, in a test sample by an antigen-antibody reaction of said test substance with the antibody of the present invention. Principle of detecting a test substance by the assay method of the present invention are not particularly limited, examples of which include an agglutination method, a sandwich method, a solid phase direct method, a solid phase binding method, a competitive method and the like. Among them, a sandwich method and a competitive method are preferable, a sandwich method being especially preferable.

In an agglutination method, an antibody binds to surfaces of particles such as latex particles and erythrocytes (for example, sheep erythrocytes) such that the particles are agglutinated if the soluble SJ2368 protein is present. In this method, the soluble SJ2368 protein is determined as an index of an agglutination degree of the particles. In this agglutination method, conventionally used particles such as gelatin, microbeads, carbon particles and the like can be used in place of the latex particles and the erythrocytes.

In a sandwich method, a solid phase direct method, a solid phase binding method and a competitive method, a labeled antibody or antigen is used. The determination can be conducted according to the principles of enzyme immunoassay method (EIA), radioimmunoassay method (RIA), chemiluminescence immunoassay method, fluoroimmunoassay method, time-resolved fluoroimmunoassay method (TR-FIA), immunochromatography assay method (ICA) and the like.

A sandwich method, a solid phase direct method and a competitive method based on the principle of EIA which is one of the preferable embodiment of the assay method of the present invention will be described below.

In an EIA by sandwich method, an antibody or a secondary antibody which recognizes the soluble SJ2368 protein and is labeled with an enzyme such as peroxidase, alkali phosphatase, β-galactosidase or the like is provided. A polyperoxidase labeled antibody is especially preferable. And, an antibody recognizing the soluble SJ2368 protein is adsorbed on a solid phase to be used. After a sample or a standard is added to the solid phase, the above enzyme-labeled antibody is added so as to bring about an antigen-antibody reaction. Excess enzyme-labeled antibody is removed by washing. Thereafter a chromophoric substrate selected depending on an enzyme used, for example o-phenylenediamine and $H_2O_2$, p-nitrophenyl phosphoric acid, 2-nitrophenyl-β-galactoside or the like is added to react with the enzyme. Since the substrate is colored depending on an amount of the enzyme and thereby an amount of the soluble SJ2368 protein in a sample, the soluble SJ2368 protein can be quantified by determining an amount of the resultant colored product.

In a solid phase direct method, a sample is directly adsorbed on a solid phase. Surfaces on the solid phase where the soluble SJ2368 protein is not adsorbed are blocked with a protein not affecting its assay system, for example BSA (bovine serum albumin) or the like and then an enzyme-labeled antibody recognizing the soluble SJ2368 protein is added and reacted. The subsequent procedures are similar to those in the above sandwich method to thereby qualitatively or quantitatively determine the soluble SJ2368 protein.

In a competitive method, a predetermined amount of the soluble SJ2368 protein recognized by an antibody used is directly adsorbed on a solid phase. After the solid phase is blocked, an enzyme-labeled antibody recognizing the soluble SJ2368 protein and a sample are added thereto. They are reacted for certain period and the solid phase is washed to remove substances unbound to the solid phase, to which a chromophoric substrate is added to react with the enzyme. After the reaction, the inhibition in binding of the enzyme-labeled antibody to the soluble SJ2368 protein onto the solid phase is determined to thereby quantify the soluble SJ2368 protein in the sample.

The soluble SJ2368 protein in a sample may be quantified by adsorbing an antibody onto a solid phase, adding an enzyme-labeled soluble SJ2368 protein and a sample simultaneously and then determining the inhibition in binding of the enzyme-labeled product to the immobilized antibody due to the addition of the sample.

Any method other than the above-mentioned assay methods includes an assay method comprising conducting an antigen-antibody reaction in a liquid phase, separating the soluble SJ2368 protein bound to a labeled antibody from the soluble SJ2368 protein unbound according to an agglutinating precipitation method using an antibody or a physical-chemical technique and then quantifying. Alternatively, it is possible to determine the soluble SJ2368 protein by preparing a secondary antibody recognizing an antibody which recognizes the soluble SJ2368 protein, labeling the secondary antibody and then effecting an antigen-antibody reaction.

In either a sandwich method, a solid direct method or a competitive method, the combination of a labeled enzyme and a chromophoric substrate may be replaced with the combination of a labeled enzyme and a bioluminescent or chemiluminescent substrate or the combination of a labeled enzyme and a fluorescent substrate or the like. Typical examples of the combination of an enzyme and a luminescent substrate include alkali phosphatase-AMPPD, horse radish peroxidase-luminol, luciferase-luciferin and the like. Examples of the combination of an enzyme and a fluorescent substrate include alkali phosphatase-umbelliferyl phosphate, horse radish peroxidase-p-hydroxyphenyl propionic acid and the like.

Further, in the above three assay methods, it is possible to quantify the soluble SJ2368 protein in a sample by using an antibody or antigen directly or indirectly labeled with a radioactive substance, a chemiluminescent substance or a fluoroluminescent substance in stead of an enzyme and determining an intensity of radioactivity, luminescence or fluorescence.

As a radioactive substance, $^{125}I$, $^{131}I$ and the like are generally used. Typical chemiluminescent substances include acridium ester and the like. For determining a fluorescent intensity, an assay method comprising directly or indirectly binding a chelating agent to an antibody or an antigen, exposing it to an excitation radiation and determining a fluorescent intensity issued from a rare earth metal bound to the chelating agent with time-resolution so as to quantify the soluble SJ2368 protein in a sample is also useful. This method is more sensitive. Typical examples of the rare earth metal include europium.

The object of the assay method of the present invention is to detect or determine the soluble SJ2368 protein in a sample. A sample to be tested includes a body fluid, a tissue or a cell of an animal (especially human), a body of a bacteria, and their extract, culture supernatant, smear and slice. The body fluid is preferable. More preferable sample is selected from blood, plasma, serum, urine, liquor, lymph, saliva, ascites and pleural effusion.

By using the assay method of the present invention, the determination of the soluble SJ2368 protein in body fluids of healthy subjects and patients suffering from different diseases is possible. And, the concentration of the soluble SJ2368 protein in body fluids could first determined by the present invention. In addition, it became clear that the concentration of the soluble SJ2368 protein varies depending on the type of diseases. When the concentration of the soluble SJ2368 protein in body fluids of patients suffering from different diseases is compared with those of healthy subjects, the determined values are statistically analyzed according to a method conventionally used by those skilled in the art so as to judge whether or not a difference therebetween is significant.

The assay reagent and the assay kit of the present invention can be constituted in accordance with the above assay method and the like.

## EXAMPLES

The present invention will be described in more detail by referring to the following examples which are not to be construed as limiting the scope of the invention.

### Example 1 : Cloning of human gene sj2368

(1) Cloning of cDNA from human spleen

A cDNA library from human spleen is constructed according to the method of Ohara et al. (DNA Research, Vol. 4, pp. 53-59 (1997)). That is, a double-stranded cDNA was synthesized by Superscript II reverse transcriptase kit (Gibco BRL) with oligonucleotide (GACTAGTTCTAGATCGCGAGCGGCCGCCC$(T)_{15}$ ; SEQ ID NO: 4) containing NotI site (Gibco BRL) as a primer and a human spleen mRNA (Clontech) as a template. The cDNA and an adaptor having SalI site (Gibco BRL) were ligated, digested with NotI and electrophoresed on 1% agarose having a low melting point to purify a DNA fragment of 3 kb or more.

The purified cDNA fragment was ligated to pBluescript II SK+ plasmid previously digested with SalI-NotI. This recombinant plasmid was introduced in E. coli ElecroMax DH10B cell line (Gibco BRL) by the electroporation method. Next, about 10, 000 recombinants were selected from the thus-constructed cDNA library and DNA sequences at both terminals of these clones were determined. Among them, about 150 cDNA clones containing the novel gene were sequenced.

For the sequencing, the DNA sequencer (RISA) manufactured by Shimadzu Corporation and the reaction kit manufactured by PE Applied Biosystems were used. The shot gun clone was sequenced by the dye terminator method to determine most of the sequences.

(2) Sequencing of cDNA clone and analysis of information about its deduced protein

The plasmid sj02368 obtained by the method described in the above (1) contained a cDNA comprising the nucleotide sequence of full length 4519 base pairs (SEQ ID NO: 3) containing an open reading frame comprising the nucleotide sequence of 1560 bases represented by SEQ ID NO: 1, encoding a novel protein comprising 520 amino acids represented by SEQ ID NO: 2. The plasmid sj02368 containing the open reading frame comprising the nucleotide sequence of 1560 bases represented by SEQ ID NO:1 was deposited on September 18, 2001 in International Patent Organism Depositary (Tsukuba City, Ibaragi Prefecture, Japan) of National Institute of Advanced Industrial Science and Technology and thereafter transferred to the International Deposition under Budapest Treaty on September 17, 2002 (Accession No. FERM BP-8182).

(3) Homology analysis and motif analysis of cDNA clone and its deduced protein

For the homology searching, the agreement in local sequences was searched using BLAST (J. Mol. Evol. , Vol. 36, pp. 290-300 (1993) ; J. Mol. Biol., Vol. 215, pp. 403-410 (1990).

The protein sequence represented by SEQ ID NO: 2 was subjected to BLAST homology searching for Genbank protein data base (http://www.ncbi.nlm.nih.gov/) using the blastp program searching a homology. As the result, the homology and the identity with mouse interferon (alpha and beta) receptor (GenBank Accession: NP_034638) were found over 312 residues at a level of 37% and 23%, respectively. The homology and the identity with human interleukin 20 receptor, alpha (GenBank Accession: NP_055247) were found over 190 residues at a level of 39% and 28%, respectively.

Further, from the prediction of a transmembrane helix using the program tmap predicting a transmembrane (B. Persson., P. Argos., J. Mol. Biol., Vol., 237, pp. 182-192 (1994)) based on weight matrix, a transmembrane region was predicted over 27 amino acid residues ranging from Nos. 226 to 252. And, from the prediction of a signal peptide using the program predicting a signal peptide cleavage site(s) (G.von Heijne, Nucleic Acid Research, Vol. 1, No. 4, pp. 4683-4690 (1986)), a signal peptide was predicted over 13 amino acid residues ranging from Nos. 8 to 20.

By the motif analysis, class II cytokine receptor motifs such as tryptophan (W) at Nos. 43, 68 and 111 and pairs of cysteine residues at Nos. 74 and 82, Nos. 195 and 211 were observed in the extracellular region of SJ2368. These characteristics are also found in a I1-20 receptor, a I1-10 receptor, an interferon alpha/beta receptor. Further, three tyrosine residues expected to be involved in the activation of a JAS-STAT system were observed in the intracellular region. The aforementioned characteristics of SJ2368 are shown in Fig. 1.

Example 2 : Analysis of expression profile using ATAC-PCR

We outsourced the adaptor-tagged competitive PCR (ATAC-PCR) so as to conduct according to the method described in Nucleic Acids Research, Vol. 25, pp. 4694-4696. The following human RNA was used in this analysis. Total RNAs were extracted from human coronary arterial endothelial cells (HCAEC; TAKARA) and human coronary arterial smooth muscle cells (CASMC; TAKARA) according to a routine method to prepare an mRNA. And, leucocytes from human peripheral blood were cultured in the presence or absence of 50 μg/ml of PHA for 5 hours followed by extracting a total RNA according to a routine method to prepare an mRNA. mRNAs of human lung, pancreas, heart, adrenal gland, testis, fetal liver, fetal kidney and brain were purchased from Clontech. And total RNAs of kidney, liver, small intestine, thymus and spleen were also purchased from Clontech and from them, mRNAs were prepared. An mRNA of colon was purchased from Stratagene. Next, a single-stranded cDNA was synthesized from 0.12 μg of each mRNA using an oligodT primer. As a reverse transcriptase, SuperScript II RNase $H^-$ Reverse Transcriptase (Invitrogen) was used. The sequence of the primer specific for sj2368 used in the PCR was 5' -AAG CTA TGT CAG AAA TTA AAC TC-3 ' (SEQ ID NO: 5) . As the analytical result, sj2368 was strongly expressed in peripheral leukocytes, fetal liver and brain and it was weakly expressed in HCAEC, fetal kidney, liver and CASMC.

Example 3 : Expression of human SJ2368

A cDNA sequence containing a coding region is prepared from the plasmid sj 02368 obtained in Example 1 and inserted in a mammalian cell expression plasmid according to a routine method, thereby a human SJ2368 expression plasmid is constructed. Then, 12.5 μg of this plasmid and 50 μl of FuGENE6 (Rosche Diagnostics) were mixed according to the provider's protocol and the mixture was added in COS cells semiconfluently grown in a 150 $cm^2$ flask, thereby the human SJ2368 can be expressed. That is, sj02368 obtained in Example 1 was digested with a restriction enzyme to prepare a cDNA fragment containing a coding region. This DNA fragment was ligated to a mammalian cell expression plasmid separately prepared so that a competent cell JM109 (Takara Bio Inc.) was transformed. After a plasmid was prepared from the resultant colonies, the cDNA sequence was confirmed, thereby the SJ2368 expression plasmid was obtained.

Example 4 : Expression of soluble human SJ2368

In order to use as an administering antigen for the production of an antibody, a chimeric (fusion) protein comprising an extracellular domain of the human SJ2368 and a histidine tag or a human IgG Fc fragment was produced. An expression plasmid for human SJ2368-His which was a chimeric protein comprising the extracellular domain of the human SJ2368 and a histidine tag, was constructed according to the following method. That is, a sense primer 1 (5'-CCG CTC GAG CAG GAA GGC CAT GGC GGG GCC CGA G-3' : SEQ ID NO: 6) and an antisense primer 1 (5'-CGC GGA TCC GGC TTC TGG GAC CTC CAG CAA GAA-3': SEQ ID NO:7) were synthesized. Using them, 30 cycles of the PCR reaction, each cycle comprising heating at 98°C for 10 seconds, at 55°C for 30 seconds and at 72°C for one minute, were performed by Pyrobest DNA Polymerase (TAKARA) with the plasmid sj02368 obtained in Example 1 as a template. The resultant PCR product of about 0.7 kb was digested with XhoI and BamHI and then electrophoresed on agarose gel to collect a DNA fragment. This fragment was ligated with pcDNA 3.1/Myc-His (-) A previously digested with XhoI and BamHI. The human SJ2368-His expression plasmid was obtained by transforming a competent cell JM109 according to a routine method. An expression plasmid of the human SJ2368-Fc which was a chimeric protein comprising the extracellular domain of the human SJ2368 and a human IgG Fc fragment was constructed according to the following method. A sense primer 2 (5'-CCG GAA TTC AGG AAG GCC ATG GCG GGG CCC GAG C- 3 ' : SEQ ID NO: 8) was synthesized. Using it together with the antisense primer 1, 30 cycles of the PCR reaction, each cycle comprising heating at 98°C for 10 seconds, at 55°C for 30 seconds and at 72°C for one minute, were performed by Pyrobest DNA Polymerase (TAKARA) with the plasmid sj02368 as a template. The resultant PCR product of about 0.7 kb was digested with EcoRI and BamHI and then electrophoresed on agarose gel to collect a DNA fragment.

While, pM1304 described in WO 97/42319 was digested with EcoRI and KpnI to collect a DNA fragment of about 7.1 kb. And, pM1304 was double digested with BamHI and KpnI to collect a DNA fragment of about 0.7 kb. These two DNA fragments and the above DNA fragment were ligated. A human SJ2368-Fc expression plasmid was obtained by transforming a competent cell JM109 according to a routine method. The amino acid sequence of the human SJ2368-Fc protein obtained in the above is represented by SEQ ID NO: 19. The resultant expression plasmids were introduced into COS cells according to the following method. That is, 50 μl of FuGENE6 (Rosche Diagnostics) and 12.5 μg of each plasmid were mixed according to the provider's protocol and the mixture was added in COS cells semiconfluently grown in a 150 $cm^2$ flask. The cells were cultured at 37°C in 5% $CO_2$ for three days and then the culture supernatant was collected. After the supernatant was applied to SDS-PAGE, the human SJ2368-His and the human SJ2368-Fc contained in the supernatant were detected by the western blotting with an anti-histidine tag antibody (Penta•His antibody; QIAGEN and peroxidase labeled mouse immunoglobulin antibody; DAKO) or an anti-Fc antibody (peroxidase labeled anti-human IgA, IgG, IgM, Kappa, Lamda antibody; DAKO) (Fig. 3). The expression of the human SJ2368-His and the human SJ2368-Fc in the culture supernatant was demonstrated.

From the supernatant, the human SJ2368-His was purified by passing it through a nickel column and the human SJ2368-Fc was purified by passing it through a Protein A column.

Example 5 : Production of anti-SJ2368 antibody

(1) Preparation of partial peptide of SJ2368

In order to produce an antibody against SJ2368, the sequences at N- and C-terminals of the extracellular domain of SJ2368 is analyzed using the Chou-Fasman and Robson program predicting a secondary structure. A sequence expected to have higher hydrophilicity and be exposed to a surface of the protein such that its structure contains α-helix or the like is selected and synthesized. The synthesis of the peptide is performed in a peptide synthesizer Model 433A (Applied Biosystems Japan) . During the synthesis, cysteine is added to N-terminal so that the peptide can be bound to a carrier protein. The peptide is excised from the resin according to a routine method, deblocked and then purified by a C18 reverse phase HPLC (CAPDELL-PAK; Shiseido Co., Ltd. ).

(2) Preparation of peptide immunogen of SJ2368

The thus-synthesized peptide is dissolved in distilled water at 10 mg/ml and then mixed with 10 mg/ml of maleimidated keyhole limpet hemocyanine (PIERCE) . They are reacted at room temperature for two hours and then the reaction is desalted through a NAP-10 column (Amersham Pharmacia Biotech) . The protein concentration is calculated by dividing an amount of KLH used with a liquid volume.

(3) Production of SJ2368 antibody

(3-1) Production of polyclonal antibody

In order to produce a rabbit polyclonal antibody against SJ2368, the peptide antigens as prepared above are mixed at a ratio of 40 μg/each antigen such that the total volume is 0.5 ml. Then, an equal amount of Freund's complete adjuvant (DIFCO) was mixed and the mixture was subcutaneously injected to a back of a rabbit. After two weeks, the same amount of the antigen is mixed with Freund's incomplete adjuvant and the mixture is injected similarly. After two weeks, a blood is taken from auricular vein to prepare an antiserum. Similarly, 30 μg/body of SJ2368-Fc prepared and purified in Example 4 is injected to prepare an antiserum.

(3-2) Production of peptide antibody

20 μg of each of the peptide carrier antigens prepared above is dissolved in 100 μl of a physiological saline solution and then mixed with an equal amount of Freund's complete adjuvant. The mixture is peritoneally injected to a BALB/c female mouse of 5-week old. After two weeks, 20 μg of the peptide carrier antigen is dissolved in 100 μl of a physiological saline solution and mixed with an equal amount of Freund's incomplete adjuvant. The mixture is peritoneally injected similarly. After one week, a blood is taken from an ocular fondus and the increase in antibody titer is confirmed by the western blotting. That is, the recombinant SJ2368 protein is electrophoresed on 4 to 20% SDS/polyacrylamide (TEFCO) and transferred onto a PVDF membrane according to the method of Millipore. After the transfer, the blocking is conducted using a 0.076M phosphate buffer solution (pH 6.4) containing 5% skimmed milk and 0.05% Tween 20 (hereinafter referred to as "T-PBS"). The antiserum is diluted with T-PBS containing 0.5% BSA 500-fold and reacted with the transferred PVDF membrane at 4°C overnight. The membrane was washed with T-PBS three times and reacted with a peroxidase labeled anti-mouse immunoglobulin antibody (DAKO) previously diluted with T-PBS containing 0.5% BSA 500-fold at room temperature for one hour. Then, the membrane is washed with T-PBS three times and detected by ECL (Amersham Pharmacia Biotech). By the aforementioned procedures, the increase in antibody titer is confirmed. After three days from the final injection of the antigen, lymphocytes are separated from spleen cells, mixed with Sp2-O-Ag14 (ATCC No. CRL1581) and subjected to cell fusion using polyethylene glycol according to "Guide to experimental operations of monoclonal antibodies", written by Tamie ANDOH and Takeshi CHIBA, published by Kodansha Ltd. Hybridomas are selected with a HAT medium and after one week a hybridoma producing the desired antibody is screened.

SJ2368-His prepared and purified in Example 4 is diluted in a 0.01M carbonate buffer (pH 9.5) to 1 μg/ml and added to an immunoplate (Maxisorb; NUNC) at 50 μl/well. After the reaction at 37°C for one hour, the plate is washed with an ion-exchanged water five times and then 100 μl of a 0.076M phosphate buffer solution (pH 6.4) (hereinafter referred to as "PBS") containing 0.5% BSA is added to each well for blocking. Further the culture supernatant is added to each well and reacted at 37°C for one hour and then the plate is washed with a physiological saline solution containing 0.05% Tween 20 three times. 50 μl of a peroxidase labeled anti-mouse immunoglobulin antibody previously diluted in PBS containing 10% rabbit serum 100-fold is added to each well. After the reaction at 37°C for one hour, the plate is washed with a physiological saline solution containing 0.05% Tween 20 five times and then a tetramethylbenzidine solution containing 0.01% hydrogen peroxide is added to each well. After the reaction at room temperature for 10 minutes, the reaction is stopped with a 0.5M sulfuric acid solution. An absorbance at 450 nm is determined using a plate spectrophotometer (NJ-2100; Nippon Intermed). Based on this result, cells reacting with SJ2368 is selected and cloned according to the limiting dilution method. After ten days, the similar screening is conducted so that a clone producing an antibody reacting with SJ2368 can be obtained. The thus-screened hybridoma is cultured in a 10% FCS/ RPMI 1640 medium (Invitrogen) and then a Hybridoma-SFM medium (Invitrogen) so that an antibody is produced. This antibody is purified through a Prosep-A column (Millipore).

(3-3) Production of anti-SJ2368 monoclonal antibody

20 μg of the purified SJ2368-Fc protein mixed with an equal amount of Freund's complete adjuvant is peritoneally injected into a BALB/c female mouse of 6-week old. After two weeks from the initial injection, 20 μg of the antigen dissolved in a physiological saline solution and mixed with an equal amount of Freund's incomplete adjuvant is peritoneally injected. After one week, the increase in antibody titer is confirmed according to the method described in the above (3-2) and then finally injected. 100 μg of the antigen is peritoneally injected to the mouse and after three days, his spleen is excised. Lymphocytes are separated from the spleen, mixed with P3x63-Ag.8.U.1 in the ratio of 10:1 and used for cell fusion with polyethylene glycol. Hybridomas are selected with a HAT medium and after one week, a hybridoma producing the desired antibody is screened. Wells producing the anti-SJ2368 antibody are screened according to the method described in (3-2). A well reacting with SJ2368-His is cloned according to the limiting dilution method and screened again, thereby the anti-SJ2368 monoclonal antibody can be obtained.

The hybridoma is cultured in a 10% FCS/RPMI 1640 medium (Invitrogen) and then a Hybridoma-SFM medium (Invitrogen) to produce an antibody. The antibody is purified through a Prosep-A column (Millipore).

Example 6 : Determination of activities of SJ2368

Biological activities of SJ2368 can be determined by using the following systems for determining the activities. These methods are useful for not only the screening and identification of IL-10 family and the like which causes an intercellular signal transduction via SJ2368 but also the discovery of agents capable of inhibiting the biological activities of SJ2368 such as pharmaceutical candidates including a neutralizing antibody.

(1) Luciferase assay

The determination of an activity based on the binding between SJ2368 and cytokine belonging to IL-10 family can be conducted in a reporter gene assay reacting with activated STATs using suitable cells according to any method, for example, the method of Laure et al. (Proc. Natl. Acad. Sci. USA, Vol. 94, pp. 10144-10149 (2000)). The cells used may be cultured cells, HepG2 and HEK293 expressing SJ2368. Alternatively, cells in which the sj2368 gene is transfected by a suitable method may be used. A gene construct (pGRR5 reporter) containing 5 copies of the GRR sequence upstream of a firefly luciferase gene controlled by a thymidine kinase (TK) promoter is prepared. And, as an internal standard a pRL-TK vector (Promega) containing a Renilla luciferase gene controlled by a TK promoter is used. 15 μg of the above pGRR5 reporter and 1 μg of the above pRL-TK are transferred in $10^6$ HepG2 cells at 74Ω and 1200 μF. 42,000 Cells are inoculated in each well of a 24-well plate and after one hour the objective IL-10 family or a culture supernatant containing IL-10 family is added to each well. After two hours, the increase in luciferase activity is observed using Dual-Luciferase Reporter Assay System kit (Promega). It is desirable that the increase in activity upon the addition of 300 U/ml of human IL-6 and the inhibition of the increase in IL-6 activity with an anti-Il-6 neutralizing antibody or an anti-gp130 neutralizing antibody are simultaneously conducted. When a culture supernatant of cells in which a cytokine gene is transfected, a culture supernatant of tumour cells, a culture supernatant of peripheral leucocytes activated with endotoxin or the like is used in place of a purified cytokine for transducing signals within cells via SJ2368, it is desirable to confirm that a culture supernatant (mock) of cells including only a vector used for transfection of a cytokine gene as a negative control and a culture supernatant to which any stimulation is not applied have no activity. In order to confirm that the action is specific via SJ2368, a neutralization experiment with SJ2368-Fc and a neutralizing experiment introducing into HepG2 cells a dominant negative wherein an intracellular thyrosine residue of SJ2368 is replaced with alanine can be conducted.

(2) Gel shift assay

A gel shift assay can confirm which STAT is activated by SJ2368 (Demoulin et al., Mol. Cell. Biol., Vol., 16, pp. 4710-4716 (1996)). That is, a nuclear extract of cells in which signals are transduced via SJ2368 is prepared. Its binding to $^{32}$P labeled oligoDNA (a double-stranded DNA comprising 5'-ATGTATTTCCCAGAAA-3' (SEQ ID NO:10) and 5'-CCTTTTCTGGGAAATAC-3' (SEQ ID NO:9)) corresponding to a γ-response region (GRP) of the Fc receptor I is observed. It is possible to observe supershifts on an electropherogram by adding 0.75 to 1 μg/lane of an anti-STAT1 antibody, an anti-STAT3 antibody or the like and to identify STAT capable of binding to the oligoDNT.

(3) Method of preparing IL-10 family usable in tests (1) and (2)

The IL-10 family whose gene has been already identified can be expressed by transferring the gene into suitable cells as the above-mentioned cytokine per se or a protein fused with a FLAG tag, a Fc fragment, a hexahistidine tag or the like and then purified. I1-19 is obtained in a culture supernatant of peripheral monocytes stimulated with endotoxin or granulocyte and monocyte colony stimulating factor for 4 to 24 hours (Gallagher et al., Genes Immun., Vol. 7, pp. 442-450 (2000)). And, IL-TIF can be obtained in a culture supernatant of peripheral blood T cells stimulated with an anti-CD3 antibody and concanavalin A for 6 to 24 hours or with IL-9 for 6 to 24 hours (Xie et al., J. Biol. Chem., Vol. 275, No. 40, pp. 31335-31339 (2000)).

<u>Example 7 : Cloning of mouse sj2368 gene</u>

(1) In silico cloning of 5'-side sequence

Using the program sim4 searching a homology, the human genome (as data source, ftp://ftp.ncbi.nih.gov/genbank/genomes/H_sapiens/hs_phase*.fna .gz; *=0, 1, 2 or 3 is available) of the cDNA sequence (SEQ ID NO: 3) of the human sj2368 gene was mapped. As the result, as shown in Fig. 4, the human sj2368 gene consisted of 7 exons. This gene structure was identical with that of the gene of IL10R family. As one example, the gene structure of IL10R α-chain is shown in Fig. 5. Next, in order to obtain a mouse homolog gene, the cDNA sequence (SEQ ID NO:3) of the human sj2368 gene was subjected to the BLAST searching for the mouse genome sequence (as data source, ftp://ftp.ncbi.nih.gov/pub/TraceDB/mus_musculus/ is available). As the result, many sequences having high homology were confirmed somewhere. When the thus-obtained sequences were analyzed in detail, a repeat sequence was confirmed and therefore it is considered that the result may be pseudopositive. Since this repeat sequence is near 3800 to 4100 bp which is the 3'-non-coding region of sj2368, this region was masked and then the mouse genome sequence was searched again. As the result, two whole shot gun sequences (WGS), i.e. G10P642115RE8.T0 and G10P625411RB5.T0, were detected. These sequences corresponded to exon 4 and exon 5 regions, respectively. In order to obtain the sequences of other exon regions, the above mouse exon sequences were searched using the mouse EST data base (as data source, ftp://ftp.ncbi.nih.gov/blast/db/est_mouse.z is available). As the result, BI104593 covering from the first exon to the fifth exon was detected. The positional relationship between the human sj2368 genome sequence and the mouse genome and the EST sequences is summarized in Fig. 6.

(2) RT-PCR cloning of 5'-side sequence

Based on the result of Example 7 (1) , a PCR primer was designed and the 5'-sided part of mouse sj2368 cDNA was subjected to the RT-PCR cloning. That is, a sense primer mS1 (5'-GGC CCC AGG ATC GAA ATG TG-3') and an antisense primer mA1 (5'-CTG GAG CCT CTA GGA AGA TG-3') were designed. With these primers, the RT-PCR was performed using a total RNA from the liver of BALB/c mouse as a template. Further, an antisense primer mA2 (5'-CAG CTG GGC TCA GAG AAC TG-3') was designed. With mS1 and mA2 primers, the PCR was performed again using the above RT-PCR product as a template. The resultant fragment of about 0.7 kb was collected by the

electrophoresis on agarose gel. Then, the 5'-terminal of the DNA fragment was phosphorylated and inserted into EcoRV of pBluescript II SK(+), thereby a plasmid containing the 5'-sided cDNA sequence of the mouse sj2368 (No.3-1/pBluescript II SK+) was obtained. The resultant plasmid contained the mouse cDNA of 661 bp represented by SEQ ID NO:11.

(3) in silico cloning of 3'-side sequence

In order to clone the 3'-side of the mouse sj2368 gene, the mouse genome sequence and the mouse EST sequence were subjected to the BLAST searching using the sequence of about 3.8 kb ranging exon 6 to a translation stop codon of human sj2368. As the result, high homologies with the mouse genome sequence(Mse40095-347f04.qlc, jtx06h08.b1, G10P665193FF5.T0) and the mouse EST sequence (BI6576668 and BB716537) were confirmed. And, 3'-side of the mouse sj2368 gene was cloned (the positional relationship between the human sj2368 genome sequence and the mouse genome and EST sequences detected is summarized in Fig. 7)

(4) RT-PCR cloning of 3'-side sequence

With the PCR primer designed based on the result of Example 7(3), the RT-PCR cloning of the 3'-side cDNA of the mouse sj2368 was performed. That is, an antisense primer mA4 (5'-CCG GCC AGC TCA CCT GAC CA-3') containing a stop codon sequence was designed. With this primer together with the sense primer mS1 described in Example 7(1), the RT-PCR was performed using a total RNA from the liver of BALB/c mouse as a template. Further, a sense primer mS3 (3'-CAG TTC TCT GAG CCC AGC TG-3 ' ) , a sense primer mS5 (5 ' -ATG CCG TCG CTG GAA CTG AA-3 ' ) and an antisense primer mA5 (5' -TCA CCT GAC CAA GTA ATC TC-3') were designed. With the mS3/mA5 primer set or the mS5/mA5 primer set, the PCR was performed using the above RT-PCR product as a template. The resultant fragment of about 1.0 kb or about 1.2 kb was collected by electrophoresing on agarose gel. After the DNA fragment was phosphorylated at 5'-terminal, it was inserted in EcoRV of pBluescript II SK(+) to be cloned (S3#2/pBluescript II SK+ or S5#1/pBluescript II SK+). The resultant clone contained the mouse cDNA of 981 bp represented by SEQ ID NO: 12 or 1161 bp represented by SEQ ID NO: 13. When the nucleotide sequence of S3#2/pBluescript II SK+ was compared with that of S5#1/pBluescript II SK+, the deletion of 51 bases at Nos. 41 to 91 in SEQ ID NO:12 of S3#2/pBluescript II SK+ was found in S5#1/pBluescript II SK+. From these results and the result as described in Example 7 (2), it was demonstrated that the cDNA sequence of the mouse sj2368 comprised the full length 1622 bp represented by SEQ ID NO:16, containing an open reading frame of 1605 bp represented by SEQ ID NO:15 encoding the 535 amino acids represented by SEQ ID NO:14. The homology of the human SJ2368 with the mouse SJ2368 was tested using GENETYX-WIN: Maximum Matching. As the result, the identity therebetween was about 59% (the comparison in sequence is shown in Fig. 8).

(5) Gene structure of mouse sj2368

The gene structure of the mouse sj2368 cloned in Example 7 (4) was determined. That is, using the cDNA sequence (SEQ ID NO:16) of the mouse sj2368, the mouse genome sequence (as data source, ftp://ftp.ensemble.org/pub/assembly/mouse/ is available) was mapped by sim4. As the result, it is demonstrated that the mouse sj2368 gene consisted of 7 exons. When the gemone structure of the mouse sj2368 is compared with that of the human sj2368, both were very similar with respect to the size and the number of exons and its structure (Fig. 9), provided that the length of an introns is somewhat different. Although joints of the exons are shown in Fig. 8, it could be confirmed that the 51 bp deletion variant obtained in Example 7(4) was an alternative splicing variant produced for the reason that the 3'-terminal of the intron 5 is recognized displacing downstreamly by 51 bp. Since this deleted region is a region encoding a transmembrane domain, it was considered that the splicing variant protein may be secreted extracellularly. The amino acid sequence of the splicing variant protein (mouse SJ2368 del) is represented by SEQ ID NO:18.

(6) Construction of mouse SJ2368 expression plasmid

A mouse SJ2368del expression plasmid was constructed according to the following method. That is, No. 3-1/pBluescript II SK+ was digested with EcoRI to collect a DNA fragment of about 0.5 kb. And, S5#1/pBluescript II SK+ was digested with EcoRI to collect a DNA fragment of about 4.1 kb. After its 5'-terminal was dephosphorylated, the above fragment of about 0. 5 kb was inserted thereto. The resultant plasmid was double digested with EcoRI + SphI or SphI + KpnI to collect a DNA fragment of about 0.5 kb or about 1.2 kb. These DNA fragments were inserted into the EcoRI/KpnI site of a mammalian cell expression vector by the 3-way ligation.

The mouse SJ2368 full length expression plasmid was constructed according to the following method. First, a sense primer mS6 (5'-GTT CAA AGG ACG AGT ACA GG-3'), a sense primer mS7 (5'-CTC TGA GCC CAG CTG CAT CTT CCT AGA GGC-3'), an antisense primer mA8 (5'-GCC TCT AGG AAG ATG CAG CTG GGC TCA GAG-3')and an antisense primer mA10 (5'-TGT CAC CGT CGT CAT CGT AG-3') were designed. Next, with the mS1/mA8 primer set, the PCR was performed using No. 3-1/pBluescript II SK+ as a template to collect an amplified fragment of about 0.7 kb. Similarly, the PCR was performed with the mS7/mA5 primer set using S3#2/pBluescript II SK+ as a template to collect an amplified fragment of about 1.0 kb. Further, the PCR was performed with the mS6/mA10 primer set using a mixture of the above two amplified fragments as a template to obtain an amplified fragment of about 0.7 kb. This fragment was double digested with SphI and SeaI to collect a DNA fragment of about 0.5 kb. And, pCGmsj2368 (del) was double digested with SphI + PstI or SeaI + PstI to collect a DNA fragment of about 4.7 kb or about 1.2 kb. These two DNA fragments and the above fragment of about 0.5 kb resulting from digesting the PCR amplified fragment with the enzymes were ligated by the 3-way ligation, thereby a msj2368 full length expression plasmid pCGmsj2368 was obtained. This plasmid was deposited on July 9, 2000 in International Patent Organism Depositary (Tsukuba City, Ibaragi Prefecture, Japan) of National Institute of Advanced Industrial Science and Technology and thereafter transferred to International Deposition under Budapest Treaty on September 25, 2002 (Accession No. FERM BP-8141).

Example 8 : Expression of soluble mouse SJ2368

A chimeric (fusion) protein comprising the extracellular domain of the mouse SJ2368 and a human IgG Fc fragment was produced. An expression plasmid for mouse SJ2368-Fc having the amino acid sequence represented by SEQ ID NO:17 which is a chimeric protein comprising the extracellular domain of the mouse SJ2368 and a human IgG Fc fragment was constructed as follows: 25 Cycles of the PCR reaction, each cycle comprising heating at 98°C for 10 seconds, at 55°C for 30 seconds and at 72°C for one minute, were performed by Pyrobest DNA Polymerase with the synthesized antisense primer mA9B (5'-CGC GGA TCC GTC CCC TGG AGC CTC TAG GAA-3') and the sense primer mS1 using S5#1/pBluescript II SK+ as a template. The resultant PCR product of about 0. 7 kb was digested with BamHI. After its 5'-terminal was phosphorylated, it was electrophoresed to obtain a DNA fragment (DNA fragment A) . While, pM1304 described in WO 97/42319 was digested with EcoRI, blunted using a DNA Blunting kit and further digested with KpnI to obtain a DNA fragment of about 7.0 kb (DNA fragment B) . Next, pM1304 was double digested with BamHI and KpnI to obtain a DNA fragment of about 0.7 kb (DNA fragment C). These three DNA fragments (A, B and C) were ligated by the 3-way ligation and a competent cell JM109 was transformed according to a routine method to obtain a mouse SJ2368-Fc expression plasmid. The resultant expression plasmid was transfected into COS cells according to the following method. That is, 50 μl of FuGENE6 and 12.5 μg of the above mouse SJ2368-Fc expression plasmid were mixed according to the provider's protocol and added to COS cells semiconfluently grown in a 150 $cm^2$ flask. The cells were cultured at 37°C in the presence of 5% $CO_2$ for three days and then its culture supernatant was collected. MSJ2368-Fc contained in the culture supernatant was detected by applying the supernatant to SDS-PAGE and subjecting to the western blotting with an anti-human Fc antibody (peroxidase labeled anti-human IgA, IgG, IgM, Kappa, Lamda antibodies; DAKO) (Fig. 10). The expression of the mouse SJ2368-Fc in the culture supernatant was confirmed.

Next, the mouse SJ2368-Fc was produced in a large amount. COS cells were semiconfluently grown in Cell Factory-10 (Nunc), to which the above mouse SJ2368-Fc expression plasmid was transfected using FuGENE6. The cells were cultured for three days and then its culture supernatant was collected. The mouse SJ2368-Fc expressed in the supernatant was purified through a HiTrap rProtein A FF column (Amersham Bioscience).

Example 9 : Screening of human sj2368 splicing variant

As described in Example 7-(4), the splicing variant wherein the deletion of 51 bp encoding a transmembrane domain had occurred was cloned in the mouse sj2368. It was confirmed whether any splicing variant of the human sj2368 is present or not. That is, the PCR was performed using cDNAs from liver, brain, PBL, fetal liver and fetal kidney (Human MTC Panel I and II, Human Fetal MTC Panel; Clontech Company) as templates. First, an antisense primer 2 (5'-CGG GGT ACC TTG CAA AGG CAG CAG CAG-3') was designed, then the PCR reaction was performed by platinum taq DNA polymerase (Invitrogen) using the sense primer 1 described in Example 4. After the heating at 94°C for 2 minutes to start the PCR reaction, 35 cycles of PCR were performed, each cycle comprising heating at 94°C for 30 seconds, at 55°C for 30 seconds and at 72°C for one minute. In addition, a sense primer 3 (5'-CCT GCT GCA GGC CGC TCC AG-3') and an antisense primer 3 (5'-GCA TCG CTG TCC TCA ATT TC-3') were designed. The PCR was performed using the above PCR products as a template under the same condition. The resultant fragment of about 1.4 kb was integrated in pBluescript II SK(+) in order to confirm its sequence. As the result, three splicing variants, i. e. (i) a clone wherein exon 2 was deleted, (ii) a clone wherein exon 6 was deleted and (iii) a clone wherein exon 2 and exon 6 were deleted, were confirmed in addition to a clone having the sequence identical with that of sj2368. A frame shift occurred during the amino acid translation in all of the splicing variants and the polypeptide represented by SEQ ID NO:21 was encoded in the variants (i) and (iii). However, this polypeptide has only about 20 residues among the sequence at N-terminal of SJ2368 and this region corresponds to a signal sequence. Therefore, it was considered that this polypeptide will be expressed as a functional protein. On the other hand, in the variant (ii), the polypeptide represented by SEQ ID NO:20 was encoded and an extracellular domain of SJ2368 was conserved, but a transmembrane domain is lost. Therefore, it is considered that this polypeptide may be expressed as a soluble protein and compete with SJ2368 for the binding to a ligand molecule. The splicing variant (ii) was double digested with SphI and XhoI so that a DNA fragment of about 340 bp wherein the deletion of exon 6 sequence had occurred was obtained. And, the SJ2368 expression plasmid as constructed in Example 3 was double digested with SphI and XhoI and then ligated to the above DNA fragment. That is, a human SJ2368 variant expression plasmid was constructed by exchanging the fragment of about 470 bp containing the exon 6 sequence with the DNA fragment of about 340 bp from the variant. After the ligation product was transformed into competent cells JM109, a plasmid was prepared according to a routine method.

Example 10 : Physiological activities of soluble SJ2368

(1) Effect on mixed lymphocyte reaction (MLR)

In order to assess physiological activities of the soluble SJ2368, the effect on MLR was determined using the mouse SJ2368-Fc. Spleens were extracted from BAL B/C and C57BL/6 male mice of 7 to 9-week old to prepare splenic cells. Both splenic cells were mixed in equal amounts and the mixture was inoculated in a 96-well plate at 0.6 x $10^6$ cells/well and cultured at 37°C in 5% $CO_2$ for four days in the presence or absence of the mouse SJ2368-Fc. As a control, the cells were cultured similarly in the presence of a chimeric protein (CONT-Fc) comprising an extracellular domain of other type I transmembrane protein molecule and a human IgG1 Fc domain, or a human IgG1 protein. After BrdU (Cell Proliferation ELISA, BrdU (colorimetric) ; Rosche Diagnostics) was added, the cells were cultured for additional four hours and then an amount of BrdU incorporated (i.e., cell proliferation rate) was determined according to the provider's protocol. As the result, the dose-dependent inhibitory effect was confirmed in the presence of SJ2368-Fc as shown in Fig. 11. While, neither CONT-Fc nor human IgF1 showed inhibitory effect. Thus, the mouse SJ2368-Fc specifically inhibited the proliferation of the mouse MLR.

(2) Effect on Th1/Th2 differentiation

The effect of the mouse SJ2368-Fc in an *in vitro* Th1/Th2 differentiation system was assessed.

(2-1) Preparation of antibody plate for stimulating cells

0.2 ml of a solution of an anti-mouse CD3 antibody (145-2C11; PharMingen) diluted in PBS to 5 μg/ml was added to each well of a 48-well plate and incubated at 4°C overnight such that the antibody was immobilized on the plate. Just before the use, the plate was washed with PBS twice and a medium (RPMI 1640 containing 10 mM HEPES, 1 mM sodium pyruvate, 50 μM 2-ME, 100 units/ml penicillin -streptomycin) once and then 0.35 ml/well of the medium was added so that the plate was equilibrated.

(2-2) Preparation and stimulation of naive T cells

Naive T cells were obtained from C57 BL/6 mice according to the magnetic labeled cell separation method. That is, spleens were extracted from 12 C57 BL/6 mice and splenic cells were suspended in 30 ml of PRMI 1640. After the centrifugation, the cells were resuspended in a 0.15M Tris-HCl (pH 7.65) hemolytic buffer containing 0.085% ammonium chloride and treated at 37°C for 5 minutes. After the cells were washed with 20 ml of PRMI 1640 once and 30 ml of MACS buffer (Hanks' balanced salt (GIBCO-BRL) containing 0.5% FBS and 2.5 mM EDTA), they were suspended in MACS buffer containing 5% mouse serum at 1 x $10^8$ cells/ml. Further, 0.5 μg of an anti-mouse CD4-FITC (PharMingen) was added to the cells and incubated at 6°C for 60 minutes. After the cells were washed with 30 ml of MACS buffer twice, they were resuspended in MACS buffer at 1 x $10^8$ cells/ml. 40 μl/1 x $10^8$ cells of anti-FITC microbeads (Daiichi Pure Chemicals Co. , Ltd.) were added to the cells and incubated at 6°C for 60 minutes again. The cells were washed with 30 ml of MACS buffer again and suspended in 1.5 ml of MACS buffer. The thus-prepared cells were applied to autoMACS (Daiichi Pure Chemicals Co., Ltd.) to obtain CD4 positive cells according to the program POSSELS. To the cells, 300 μl/15 ml of a release reagent was added and treated at 6°C for 20 minutes. The cellular suspension was applied to autoMACS so that the anti-FITC microbead and the cells were separated. Further, in order to obtain CD62L positive CD4T cells, the above CD4 positive T cells were suspended in 50 μl/1 x $10^7$ cells of MACS buffer. To the cells, 30 μl/1 x $10^7$ of a stop solution (Daiichi Pure Chemicals Co., Ltd.) and 20 μl/1 x $10^7$ of CD62L microbeads (anti-mouse CD62L microbeads (MEL-14); Daiichi Pure Chemicals Co., Ltd.) were added to the cells and incubated at 6°C for 30 minutes. After the reaction was finished, the cells were washed with 30 ml of MACS buffer twice and 1.5 ml of the cell suspension was applied to autoMACS to obtain CD4CD62L positive T cells according to the program PESSELS. These cells were washed with a T cell culturing solution (RPMI 1640 containing 10 mM HPES, 1 mM sodium pyruvate, 50 μM 2-mercaptoethanol, 100 units/ml penicillin-streptomycin) containing 10% FBS and then suspended in the T cell culturing solution containing 10% FBS at 5 x $10^5$ cells/ml. The resultant naive T cells were inoculated in the antibody plate prepared in the above (2-1) at 0.5 ml/well. Further, 100 μl/well of the SJ2368-Fc solution was added such that the final concentration of 3 μg/ml or 30 μg/ml was attained and then the cells were cultured at 37°C in 5% $CO_2$. As a control, 100 μl of a physical saline solution or 100 μl of a human IgG protein solution such that the final concentration of 30 μg/ml was attained was added in place of the addition of SJ2368-Fc. After 72 hours from the start of the culture, 1 ml of a supernatant was collected. A cytokine concentration in the supernatant was determined by ELISA.

(2-3) Determination of cytokine concentration by ELISA

A cytokine concentration in the culture supernatant collected after three days from the start of the culture was determined. For the determination, OptEIA Mouse IL-4 Set (PharMingen) and Murine IFN-γ Eli-pair (Diaclone) were used.

The activity of the mouse SJ2368-Fc to induce the production of IFN-γ and IL-4 in the Th1/Th2 differentiation system was expressed based on the induction by the addition of a physiological saline solution, the latter being 1 (Table 1).

Table 1

| | IFN-γ | IL-4 |
|---|---|---|
| physiological saline solution | 1.00 ± 0.05 | 1.00 ± 0.24 |
| human IgG (30μg/ml) | 1.14 ± 0.07 | 1.14 ± 0.13 |
| SJ2368-Fc ( 3μg/ml) | 1.21 ± 0.10* | 0.97 ± 0.07 |
| SJ2368-Fc (30μg/ml) | 1.33 ± 0.11** | 0.79 ± 0.12 |

* p<0.05
** p<0.01
test by the Dunnett method

As shown in Table 1, it was recognized that the production of IFN-γ was significantly enhanced by the mouse SJ2368-Fc. And, it was recognized that there was a tendency to inhibit the production of IL-4 by the mouse SJ2368-Fc. This is considered that the mouse SJ2368-Fc acted on the mouse naive T cells so that the Th1 differentiation was induced, suggesting that a receptor of the soluble SJ2368 protein may be present in T cells. As an intercellular signal transduction factor inducing the Th1 differentiation, T-bet and Txk have been known. The soluble SJ2368 protein may bind to a receptor present in T cells and activate the above signal transduction factor. While, when the SJ2368 protein (transmembrane type) is present in the naive T cells, it is also considered that the soluble SJ2368 protein competitively inhibited functions of the SJ2368 protein (transmembrane type) present on the T cells and as the result the Th1 differentiation was induced. In this case, it is assumed that the SJ2368 protein has naturally an activity of inducting the Th2 differentiation.

<u>Example 11 : Production of anti-human and -mouse SJ2368 antibody</u>

(1) Preparation of partial peptides of human and mouse SJ2368

In order to produce an antibody cross-reacting with both mouse and human SJ2368, the amino acid sequences of both SJ2368 were compared. When the amino acid sequence (RPRLAPPRNVTL) at Nos. 21 to 32 at N-terminal of the mouse SJ2368 (SEQ ID NO: 14) was compared with that of the human SJ2368, only one amino acid is different therebetween. And, this is at N-terminal. Therefore, this peptide was selected as a peptide for immunization used in the production of a human/mouse SJ2368 antibody. While, it was observed that two amino acids are different when the sequence (VELAPPTLVLTQMEK) at Nos. 122 to 136 of the mouse SJ2368 was compared with that of the human SJ2368. From the analysis using the Chou-Fasman and Robson program predicting a secondary structure, it is expected that this peptide may take a combination structure partially having a turn structure. Therefore, the peptide is assumed to be exposed on the protein surface and it was selected as a peptide for immunization used in the production of a mouse SJ2368 antibody. Since the peptide has not any reactive group binding to a carrier protein, cysteine residue was added to C-terminal. The peptide was synthesized in an ABI433A peptide synthesizer (Applied) . After the excision from the resin and the deblocking were conducted according to a routine method, the peptide was purified by C18 reverse phase HPLC (CAPCELL-PAK; Shiseido Co., Ltd.).

(2) Preparation of peptide immunogen of SJ2368

The peptide synthesized above was dissolved in distilled water at 10 mg/ml and the solution was mixed with 10 mg/ml of meleimidated keyhole limpet hemocyanin (PIERCE). They were reacted at room temperature for two hours followed by desalting through a NAP-10 column (Pharmacia).

(3) Production of polyclonal antibody

In order to product a polyclonal antibody against the human and mouse SJ2368, 100 μg of the peptide immunogen prepared above was mixed with an equal amount of Freund's complete adjuvant (FCA; DIFCO) and the mixture was subcutaneously injected on each back of NZW female rabbits having 2 to 2.4 kg body weight (Kitayama Labes, Co., Ltd.) or Wister female rats of 8-week old (SLC). After two weeks, the same amount of the peptide immunogen mixed with Freund' s incomplete adjuvant (FIA; DIFCO) was injected similarly. After one week, a blood was taken from auricular vein in case of the rabbit or heart in case of the rat to prepare an antiserum. Then, a saturated ammonium sulfate solution was added to the antiserum such that 50% saturation was attained followed by dialysis. The resultant precipitates were centrifuged, dissolved in PBS and then dialyzed. Then, the rabbit antibody was purified through a protein A column (Prosep-A; Millipore) or the rat antibody was purified through a protein G column (Prosep-G; Millipore), thereby an IgG fraction was obtained. The protein concentration was calculated based on an absorbance at 280 nm.

Example 12 : Production of antibody specific for human soluble SJ2368 protein (splicing variant)

(1) Preparation of peptide of human soluble SJ2368 protein (splicing variant)

The peptide sequence at Nos. 224 to 244 which is not identical with the sequence of full length SJ2368 among the amino acid sequence of the human soluble SJ2368 protein (splicing variant) was analyzed using the Chou-Fasman and Robson program predicting a secondary structure. The sequence (CGNLSAQQTRVRE) at Nos. 232 to 244 is assumed to have a high hydrophilicity and a structure containing a turn structure and be exposed on the protein surface and therefore it was used as a peptide for immunization. The peptide was synthesized in an ABI433A peptide synthesizer (Applied). After the excision from the resin and the deblocking were conducted according to a routine method, the peptide was purified by C18 reverse phase HPLC (CAPCELL-PAK; Shiseido Co., Ltd.).

(2) Preparation of immunogen of soluble SJ2368

The peptide synthesized above was dissolved in distilled water at 10 mg/ml and the solution was mixed with 10 mg/ml of meleimidated keyhole limpet hemocyanin (PIERCE). They were reacted at room temperature for 2 hours followed by desalting through a NAP-10 column (Pharmacia).

(3) Production of polyclonal antibody

In order to produce a polyclonal antibody against the human soluble SJ2368 protein (splicing variant), 100 μg of the peptide antigen prepared above was mixed with an equal amount of FCA (DIFCO) and the mixture was subcutaneously injected on each back of Wister female rats of 8-week old (Kitayama Labes, Co., Ltd.). After two weeks, the same amount of the peptide antigen mixed with an equal amount with FIA was injected. After one week, a blood was taken from heart to prepare an antiserum. Then, a saturated ammonium sulfate solution was added to the antiserum such that 50% saturation was attained followed by dialysis. The resultant precipitates were centrifuged, dissolved in PBS, dialyzed and then purified through a protein G column (Prosep-G; Millipore), thereby an IgG fraction was obtained. The protein concentration was calculated based on an absorbance at 280 nm. It was confirmed by the western blotting that the resultant antibody specifically binds to the human soluble SJ2368 protein (splicing variant). Thus, the human soluble SJ2368 protein (splicing variant) was electrophoresed on 5-20% SDS-polyacrylamide gel (ATTO) and transferred to a PVDF membrane according to the Millipore's method. After transferring, the membrane was blocked with PBS containing 5% skimmed milk and 0.05% Tween 20. The purified antibody was diluted in PBS containing 5% BSA and 0.05% Tween 20 to 5 μg/ml and then reacted with the transferred PVDF membrane at room temperature for one hour. Next, the membrane was washed with PBS containing 0.05% Tween 20 three times and reacted with a peroxidase labeled anti-rat immunoglobulin antibody (DAKO diluted in PBS containing 0.5% BSA and 0.05% Tween 20 1000-fold at room temperature for one hour. The membrane was washed similarly three times and then detected using ECL (Amersham). As the result, a band of the human soluble SJ2368 protein (splicing variant) was detected around about 27kDa. No band of the full length SJ2368 used as a control was detectable.

Example 13 : Production of anti-SJ2368 monoclonal antibody

(1) Production of rat monoclonal antibody

A mixture of 20 μg of the purified human SJ2368-Fc fusion protein prepared in Example 4 and an equal amount of FCA was injected into each footpad of Wistar female rats of 8-week old (Kitayama Labes, Co., Ltd.). After two weeks from the initial injection, 20 μg of the antigen mixed with an equal amount of FIA was similarly injected. After three days from the final injection, iliac lymphonodes were extracted from the rat to be used for cell fusion. That is, lymphocytes were separated from the lymphonodes using a cell strainer (Falcon), mixed with myeloma cells (Sp2/O-Ag14) and then the mixture was subjected to cell fusion using polyethylene glycol according to "Guide to experimental operations of monoclonal antibodies", p. 83, written by Tamie ANDOH and Takeshi CHIBA, published by Kodansha Ltd. (1991) . Hybridomas were selected with a HAT medium and after one week a hybridoma producing the desired antibody was screened. That is, the purified human SJ2368-His protein was diluted in a 0.076M phosphate buffer solution (pH 6.4) (hereinafter referred to as "PBS") to 2.5 μg/ml and applied to an immunoplate (Maxisorb; NUNC) at 50 μl/well. After the reaction at 37°C for one hour, the plate was washed with an ion-exchanged water five times and 100 μl of PBS containing 0.5% BSA was added to each well for blocking. Then, the culture supernatant was added to each well, reacted at 37°C for one hour and then the well was washed with a physiological saline solution containing 0.05% Tween three times. To each well, 50 μl of a peroxidase labeled anti-rat immunoglobulin antibody (DAKO) diluted in PBS containing 10% rabbit antiserum 1000-fold was added. After the reaction at 37°C for one hour, the plate was washed five times similarly and then a TMB solution (BioFix) was added to each well. After the reaction at room temperature for 10 minutes, it was stopped with a 0.5M sulfuric acid solution. Absorbance at 450 nm was determined using a plate spectrophotometer (NJ-2100; Nippon Intermed) . As the result, cells reacting with the purified SJ2368-His protein were selected and cloned according to the limiting dilution method. After 10 days, the similar screening was conducted. As the result, five clones which produced antibodies reacting with the purified SJ2368-His protein were obtained. F1120-21-3 antibody especially having a high reactivity among the resultant antibodies was determined for subtype using a rat typing kit (ZYMED). The subtype was IgG2b/K.

(2) Production of mouse monoclonal antibody

50 μl of CpG adjuvant (ImmunEasy Mouse Adjuvant; QIAGEN) was mixed with 20 μg of the purified human SJ2368-Fc fusion protein to obtain an administering antigen of 100 μl. This antigen was injected into footpads of a ddY female mouse of 5-week old (SLC) at 50 μl/footpad using a microsyringe. After 9 days, 20 μg of the administering antigen diluted in 100 μl of a physiological saline solution was injected to the footpads at 50 μl/each footpad. After three days, lymphocytes were separated from iliac lymphonodes, mixed with myeloma cells (P3U1) and then the mixture was subjected to cell fusion using polyethylene glycol according to "Guide to experimental operations of monoclonal antibodies", written by Tamie ANDOH and Takeshi CHIBA, published by Kodansha Ltd. (1991). Hybridomas were selected with a HAT medium and after one week a hybridoma producing the desired antibody was screened in the same way as that described in (1). The cells reacting with the purified SJ2368-His protein was cloned according to the limiting dilution method. After 10 days, the similar screening was conducted. As the result, ten clones which produced antibodies reacting with the purified SJ2368-His protein were obtained. These hybridomas were cultured in a 10% FCS/RPMI-1640 medium (GIBCO), further cultured in a Hybridoma-SFM medium (GIBCO) to produce antibodies and the resultant antibodies were purified through a Prosep-A column (Millipore). F1157-10-2 antibody having a high reactivity among the resultant antibodies was determined for subtype using IsoStrip Mouse Monoclonal Antibody Isotyping Kit (Roche). The subtype was IgG1/κ and IgG2a/κ.

Example 14 : Specificity of rat and mouse monoclonal antibodies

In the way described in Example 4, a SJ23368 expression plasmid, a human soluble SJ2368 protein (splicing variant) plasmid or a vector plasmid was introduced in COS cells. After the cells were cultured for two days, they were lysed in a sample buffer for SDS-PAGE. The lysate was electrophoresed on SDS-polyacrylamide gel (ATTO) with 5 to 20% gradient and the protein was electrically transferred onto a PVDF membrane (Millipore). This membrane was reacted with the antibody prepared in Example 13 previously diluted in a 0.1% Tween 20 containing PBS (hereinafter referred to as "T-PBS") containing 10% Blockace to 5 μg/ml at room temperature for one hour. After the antibody was washed with T-PBS three times, it was reacted with a HRP labeled anti-mouse immunoglobulin antibody (DAKO) or a HRP labeled anti-rat immunoglobulin antibody (DAKO) at room temperature for one hour. The reaction was washed with T-PBS three times and then detected using an ECL kit (Amersham Bioscience). As the result, specific bands of about 57 kDa and 27 kDa were detected in the lysates of COS cells in which the SJ23368 expression plasmid and the human soluble SJ2368 protein were introduced, respectively.

Example 15 : Preparation of SJ2368 determination system

(1) Production of labeled antibody

In order to prepare a sandwich ELISA system, the F1120-21-3 antibody produced in Example 13 was labeled with peroxidase. That is, according to the method of Nakane et al. (J. Histochem. Cytochem., Vol. 22, p. 1084 (1974)), 1 mg of peroxidase (Toyobo Co., Ltd.) was dissolved in distilled water, to which 100 mM of periodic acid dissolved in distilled water was added and reacted at 25°C for 20 minutes. After the reaction was finished, 1.5% ethylenegycol was added and reacted at 25°C for 10 minutes. The reaction was dialyzed against 1 mM acetate buffer (pH 4.4). Then, the purified F1120-21-3 antibody was dialyzed against 10 mM carbonate buffer (pH 9.5). 1 mg of peroxidase activated by adding 1M carbonate butter (pH 9.5) was added per mg of the antibody and reacted at 25°C for two hours. 4 mg/ml of sodium borohydride was added and reacted at 4°C for additional two hours. The reaction was dialyzed against PBS, thereby a peroxidase labeled antibody was obtained. The antibody concentration was calculated from an amount of the antibody used by determining the volume of the solution.

(2) Construction of sandwich ELISA system

An ELISA system was constructed using F1157-10-2 antibody solid phase / peroxidase labeled F1120-21-3. First, a F1157-10-2 antibody immobilized plate was made. That is, the antibody was diluted in a 50 mM Tris-HCl buffer (pH 8.0) to 10 μg/ml and 50 μl of the dilution was added to each well of an immunoplate (Maxisorp; NUNC) and reacted at 45°C for 30 minutes. The plate was washed with ion-exchanged water five times and 100 μl of PBS (pH 6.4) containing 20% Blockace (Snow Brand Milk Products Co., Ltd.) was added to each well for blocking. A set of standard sample was prepared by diluting the purified human SJ2368--His protein in PBS (pH 6.4) containing 0.1% BSA to 3.1, 6.3, 12.5, 25, 50, 100 and 200 ng/ml. As a blank sample, PBS (pH 6.4) containing 0.1% BSA was used. For the determination, first the blocking agent was discarded, each 25 μl of the above-prepared standard samples and the blank samples were added and then 25 μl of the peroxidase labeled F1120-21-3 antibody diluted in D-PBS (pH 7.4; SIGMA) containing 1% BSA, 1% rat serum and 1% mouse serum to 2 μg/ml was added and reacted at 25°C overnight. The plate was washed with a physiological saline solution containing 0.05% Tween 20 three times and a TMB solution (BioFX) was added to each well. After the reaction was conducted at room temperature for 20 minutes, it was stopped with a 0.5M sulfuric acid solution. Absorbance at 450 nmwas determined using a plate spectrophotometer (NJ-2100; Nippon Intermed), thereby a calibration curve as shown in Fig. 13 was prepared.

Example 16 : Determination of soluble SJ2368 protein in culture supernatants of different cell lines

A concentration of the SJ2368 protein in each of culture supernatants of 173 cell lines was determined using the determination system described in Example 15. As the result, the concentration of the soluble SJ2368 protein in the culture supernatants was around 5 to 15 ng/ml in many cell lines, as shown in Fig. 14, but this protein was hardly produced in megakaryoblasts. The increase in concentration was observed in a part of melanomas, human lymphomas, human bladder carcinomas, sarcomas, B-lymphocytes, The above results suggest that the soluble SJ2368 protein is constantly produced in different cell lines and it has the possibility of participating in certain regulatory mechanism.

Example 17 : Determination of soluble SJ2368 protein in sera of different patients

A concentration of the soluble SJ2368 protein in each of sera of 40 healthy subjects (20 male subjects and 20 female subjects) and 103 patients suffering from different diseases (35 diseases, three patients per disease and one patient suffering from autoimmune hepatitis) was determined using the determination system described in Example 15. As the result, the concentration of the soluble SJ2368 protein in the sera of the healthy subjects ranged from 8 to 18 ng/ml, an average being 11.4 ng/ml. The concentration of the male healthy subjects was somewhat higher. It was recognized that the concentration of the soluble SJ2368 protein in the sera was increased in the patients suffering from pulmonary tumor, angina pectoris, cardiac failure, hepatic failure, osteoporosis and dementia, as shown in Fig. 15. Especially, two patients among three patients suffering from dementia and hepatic failure showed higher value. The above results suggest that the soluble SJ2368 protein is constantly produced in blood and it has the possibility of participating in the maintenance of biological homeostasis and the above diseases by increasing its concentration due to any cause(s).

Since two antibodies used in the ELISA system also bind to the human soluble SJ2368 protein (splicing variant) as shown in Example 14, it is considered that a soluble SJ2368 protein produced by cleaving the extracellular region of the SJ2368 protein and the soluble SJ2368 protein which is a splicing variant were determined in this system.

Example 18 : Expression of SJ2368 in human peripheral blood mononuclear cells

(1) Fluorescent staining of SJ2368 antibody

The F1120-21-3 antibody prepared in Example 13 was stained with a fluorescent dye to be used in the flowcytometry. That is, the antibody was labeled with Oregon Green 488 dye using Oregon Green 488 Protein Labeling Kit (Molecular Probes) according to the provider's protocol (hereinafter F1120-21-3 labeled with Oregon Green 488 is referred to as "OG labeled F1120-21-3"). Further in order to confirm the reactivity of the OG labeled F1120-21-3 antibody, this antibody was analyzed by the flowcytometry according to the following procedures. The SJ2368 expression plasmid or a vector plasmid was transfected into COS cells in the way as described in Example 4 and then the cells were detached in PBS- containing 0.1% EDTA. The thus-detached COS transfectant was incubated with 10 μg/ml of the OG labeled F1120-21-3 on ice for 30 minutes, washed with $PBS^-$ containing 0.25% inactivated FBS and 0.1% EDTA three times and then analyzed using FACSCalibur (Nippon Becton, Dickinson and Company). As the result, a specific staining could be demonstrated in the transfectant in which the SJ2368 expression plasmid was transfected. Thus, the expression of SJ2368 on a cell membrane was confirmed (Fig. 16).

(2) Expression of SJ2368 in human peripheral blood mononuclear cells (PBMC)

A mononuclear cells fraction expressing SJ2368 was determined using human PBMC. Human PBMC purchased from AllCells, LLC (Agent: Veritas Corporation) was suspended in a RPMI 1640 medium (Sigma) containing 5% inactivated FBS and then 0.8 to 1.0 x $10^6$ cells/well were inoculated in a 24-well plate. Optionally, 5 ng/ml of Phorbol 12-myristate 13-acetate (hereinafter abbreviated to "PMA"; Sigma) and 0.5 μg/ml of Ionomycin (Sigma) were added to the medium. PBMC were cultured in 5% $CO_2$ at 37°C for 18 hours and then collected followed by incubating with 10 μg/ml of a rat IgG2a control antibody on ice for 40 minutes. After washing with $PBS^-$ containing 0.25% inactivated FBS and 0.1% EDTA three times, PBMS was incubated with 10 μg/ml of the OG labeled F1120-21-3 antibody prepared in (1) and the commercially available phycoerythrin (PE) labeled cell surface marker (Nippon Becton, Dickinson and Company) on ice for 40 minutes so that PBMC was double-stained. After PBMC was washed with $PBS^-$ containing 0.25% inactivated FBS and 0.1% EDTA three times, an expression status was observed using FACSCalibur. As the result, the expression of SJ2368 was observed in a part of CD3 positive cells. By adding PMA and Ionomycin, the SJ2368 expressing cells were increased. Similar expression was observed in CD4 positive cells and induced by PMA and Ionomycin. The expression was hardly observed in CD8 positive cells under no stimulation, but it was confirmed that the expression was induced by stimulating with PMA and Ionomycin. The SJ2368 expression in each of CD19, CD16 and CD33 positive cells was not observed under no stimulation and under the stimulation with PMA and Ionomycin. Results are summarized in Table 2. Ratio (%) of SJ2368 and each of CD positive cells with respect to total PBMC was expressed.

Table 2

| | CD3/SJ2368 | CD4/SJ2368 | CD8/SJ2368 | CD16/SJ2368 | CD19/SJ2368 | CD33/SJ2368 |
|---|---|---|---|---|---|---|
| no stimulation | 6.7 | 4.2 | 2.6 | 0.2 | 0.3 | 2.6 |
| PMA + Ionomycin | 20.5 | 11.4 | 6.1 | 0.3 | 1.9 | 0.3 |

Example 19 : Gel shift assay

(1) Cloning of human IL10R α-chain and β-chain

In order to elucidate a mechanism how SJ2368 transduces signals within cells, the gel shift assay was conducted by referring to the method of Kotenko (The EMBO Journal, Vol. 16, pp. 5894-5903 ) . First, for cloning human IL10R α-chain and human IL10R β-chain, a sense primer RS1 (5'-CCG GAA TTC AGG CCG GCT CCG CTC CGG-3'), a sense primer RS2 (5'-CCG GAA TCT GCG GCG CGC CCA GGA TGC-3'), a sense primer RS3 (5'-CCG GTC GAG TGC TTG GAG GAA GCC GCG-3'), a sense primer RS4 (5'-CCG GTC GAG CGT CCG TCC ATG GCG TGG-3'), an antisense primer RA1 (5'-CGG GGT ACC TCC TGG TCC AGG CAG AGG-3'), an antisense primer RA2 (5'-CGG GGT ACC TCT CAG CCC GAG TCA CTC-3' ) , an antisense primer RA3 (5'-CCC AAG CTT CTA GAT GTG GGG CTG GCT-3') and an antisense primer RA4 (5'-CCC AAG CTT GCT GCC CTG ATC CCT CAC-3' ) were designed. 30 Cycles of the PCR, each cycle comprising heating at 98°C for 10 seconds, at 55°C for 30 seconds and at 72°C for 90 seconds, were performed by Pyrobest DNA Polymerase (Takara Bioscience) with a cDNA from human PBL (Human MTC Panel II, Clontech) as a template. For IL10R α-chain, a set of the sense primer RS1 and the antisense primer RA1 was used. For IL10R β-chain, a set of the sense primer RS3 and the antisense primer RA3 was used. Next, 30 cycles of the PCR, each cycle comprising heating at 98°C for 10 seconds, at 55°C for 30 seconds and at 72°C for 90 seconds, were again performed by Pyrobest DNA Polymerase with each of the resultant PCR products liquids as a template. For IL10R α-chain, a set of the sense primer RS2 and the antisense primer RA2 was used. For IL10R β-strand, a set of the sense primer RS4 and the antisense primer RA4 was used. The resultant fragments of about 1.8 kb and 1.1 kb were introduced into mammalian cell expression plasmids, thereby expression plasmids for IL10R α-chain and IL10R β-chain were constructed.

(2) Construction of chimeric receptor expression plasmid

Chimeric receptors (hereinafter referred to as "CR10") comprising IL10R α-chain and SJ2368 were constructed. A cDNA fragment comprising an extracellular domain of human IL10R α-chain fused with transmembrane and intracellular domains of the human SJ2368 was prepared according to the recombinant PCR method. That is, two chimeric receptors similar to the chimeric receptor construct of IL10R α-chain and IFN-γR as described in the article of Kotenko et al. were prepared. One construct was CR10A having the amino acid sequence represented by SEQ ID NO: 22 which was a fusion protein of a peptide comprising extracellular domain and several amino acids of transmembrane domain of the human IL10R α-chain and transmembrane and intracellular domains of the human SJ2368. Another construct was CR10B having the amino acid sequence represented by SEQ ID NO:23 which was a fusion protein of a peptide comprising an extracellular domain of the human IL10R α-chain and transmembrane and intracellular domains of the human SJ2368. First, a sense primer CRS1 (5'-AGG CAG TAT TTC ACC GTG ACC AAC TGG GCT TTC CTG GTG CTG CCA-3'), a sense primer CRS2 (5'-GAG TGC ATC TCC CTC ACC AGG AAC TGG GCT TTC CTG GTG CTG-3'), an antisense primer CRA1 (5'-TGG CAG CAC CAG GAA AGC CCA GTT GGT CAC GGT GAA ATA CTG CCT-3') and an antisense primer CRA2 (5'-CAG CAC CAG GAA AGC CCA GTT CCT GGT GAG GGA GAT GCA CTC-3') were designed. The recombinant PCR was performed with a set of the sense primer CRS1 and the antisense primer CRA1 for CR10A and a set of the antisense primer CRS2 and the antisense primer CRA2 for CR10B using the SJ2368 expression plasmid prepared in Example 3 and the IL10R α-chain expression plasmid prepared in the above (1) as templates so that the chimeric receptor cDNA fragments were amplified. Then the thus-amplified DNA fragments and the IL10R α-chain expression plasmid were ligated at the restriction enzyme site respectively, thereby CR10 expression plasmids were prepared. Using the IL10R β-chain expression plasmid, a unit of expression promoter/ IL10R β-chain cDNA/polyA addition signal was excised and this unit was inserted tandemly downstream of the poly A signal for IL10R α-chain in the above CR10 expression plasmid. By the above procedures, a CR10A and IL10R β-chain expression plasmid (pCR10A + Rb) and a CR10B and IL10R (β-chain expression plasmid (pCR10B + Rb) were constructed.

(3) Expression of CR10A and CR10B

In order to confirm the expression of CR10A and CR10B, a SJ2368 expression plasmid, an IL10R α-chain expression plasmid, pCR10A + Rb, pCR10B + Rb or a vector plasmid was transfected into HEK 293 cells or COS cells using FuGENE6. The cells were cultured for two days and then they were detached using $PBS^-$ containing 0.1% EDTA and incubated with a PE labeled anti-CD210 antibody (anti-IL10R α-chain antibody; Fujisawa Pharmaceutical Co., Ltd.) on ice for 30 minutes. The staining status was determined using FACSCalibur. As the result, the cells in which pCR10A + Rb or pCR10B + Rb was transfected demonstrated specific staining similar to the staining of the cells in which the IL10R expression plasmid was transfected. The cells in which the SJ2368 expression plasmid or the vector plasmid was transfected did not stained (the flowcytometric result using HEK293 cells is shown in Fig. 17). Considering the above, it was confirmed that CR10A or CR10B is expressed on a cell membrane by transfecting with pCR10A + Rb or pCR10B + Rb.

(4) Gel shift assay

It becomes possible to prepare cells expressing a chimeric receptor comprising IL10R α-chain and SJ2368, and an IL10R β-chain using the method described in the above (3) . A nuclear extract can be prepared by stimulating the expressed cells with IL10 according to the method described in the article of Kotenko et al. And, GAS element (5'-GAT CGA TTT CCC CGA AAT CAT G-3') of a human IRF-1 gene is prepared as a probe according to the method described in the same article. By conducting the gel shift assay, the activation of STAT by stimulating with IL-10 can be confirmed. That is, although a ligand inherent to SJ2368 has not been clarified, the intercellular signal transduction can be caused by using the chimeric receptor with an IL10R α-chain so that a receptor function of SJ2368 can be elucidated.

Example 20 : Analysis by DNA array

CR10 transformants are provided according to the method described in Example 19. Next, the intercellular signal transduction of SJ238 is switched to on/off by optionally adding IL-10. From these cells, an RNA is prepared. A gene inducing or inhibiting the SJ2368 signal transduction can be confirmed using the commercial DNA array (or DNA chips) so that a molecule group participating in the signal transduction of SJ2368 can be identified.

INDUSTRIALLY APPLICABILITY

SJ2368 of the present invention is a protein which may be associated with the onset or progress of inflammatory diseases and therefore it is very useful in the development of preventive or therapeutic agents for diseases such as septic organ disorders, autoimmune diseases, inflammatory diseases, allergic diseases, tumors and the like.

The gene sj2368 can be used as antisense drugs or in gene therapy. The protein SJ2368 is useful as such or as soluble protein drugs by preparing its soluble fragment (extracellular region or each domain). Further, an antibody having a reactivity with SJ2368 or its partial peptide, a part of the antibody, or a low molecular compound capable of modifying an intracellular signal transduction system of SJ2368 are useful as medicines controlling the functions of SJ2368 *in vivo.*

## Claims

**1.** The following protein (a) or (b):

(a) a protein comprising the amino acid sequence represented by SEQ ID NO:2; or
(b) a class II cytokine receptor comprising an amino acid sequence wherein deletion, substitution or addition of one or more amino acids has occurred in the amino acid sequence of SEQ ID NO:2.

**2.** The following protein (a) or (b):

(a) a protein comprising the amino acid sequence represented by SEQ ID NO:14; or
(b) a class II cytokine receptor comprising an amino acid sequence wherein deletion, substitution or addition of one or more amino acids has occurred in the amino acid sequence of SEQ ID NO:14.

**3.** The following protein (a) or (b):

(a) a protein comprising the amino acid sequence represented by SEQ ID NO:20; or
(b) a soluble SJ2368 protein comprising an amino acid sequence wherein deletion, substitution or addition of one or more amino acids has occurred in the amino acid sequence of SEQ ID NO:20.

**4.** The following protein (a) or (b):

(a) a protein comprising the amino acid sequence represented by SEQ ID NO:18; or
(b) a soluble SJ2368 protein comprising an amino acid sequence wherein deletion, substitution or addition of one or more amino acids has occurred in the amino acid sequence of SEQ ID NO:18.

**5.** A fusion protein comprising the protein as defined in claim 3 or 4.

**6.** An SJ2368 partial peptide as defined in any one of (a) to (h) :

(a) a polypeptide comprising the amino acid sequence at Nos. 21 to 225 in the amino acid sequence represented by SEQ ID NO:2;

(b) a human SJ2368 extracellular domain comprising an amino acid sequence wherein deletion, substitution or addition of one or more amino acids has occurred in the amino acid sequence of the polypeptide as defined in (a);
(c) a polypeptide comprising the amino acid sequence at Nos. 21 to 224 in the amino acid sequence represented by SEQ ID NO:14;
(d) a mouse SJ2368 extracellular domain comprising an amino acid sequence wherein deletion, substitution or addition of one or more amino acids has occurred in the amino acid sequence of the polypeptide as defined in (c);
(e) a polypeptide comprising the amino acid sequence at Nos. 253 to 520 in the amino acid sequence represented by SEQ ID NO:2;
(f) a human SJ2368 intracellular domain comprising an amino acid sequence wherein deletion, substitution or addition of one or more amino acids has occurred in the amino acid sequence of the polypeptide as defined in (e);
(g) a polypeptide comprising the amino acid sequence at Nos. 253 to 535 in the amino acid sequence represented by SEQ ID NO:14; or
(h) a mouse SJ2368 intracellular domain comprising an amino acid sequence wherein deletion, substitution or addition of one or more amino acids has occurred in the amino acid sequence of the polypeptide as defined in (g).

**7.** A fusion protein comprising the partial peptide as defined in claim 6.

**8.** A DNA as defined in any one of (a) to (c):

(a) a DNA comprising the nucleotide sequence represented by SEQ ID NO:1;
(b) a DNA hybridizable with the DNA of SEQ ID NO:1 under stringent conditions and encoding a class II cytokine receptor; or
(c) a DNA encoding the protein as defined in claim 1.

**9.** A DNA as defined in any one of (a) to (c):

(a) a DNA comprising the nucleotide sequence represented by SEQ ID NO:15;
(b) a DNA hybridizable with the DNA of SEQ ID NO:15 under stringent conditions and encoding a class II cytokine receptor; or
(c) a DNA encoding the protein as defined in claim 2.

**10.** A DNA as defined in any one of (a) to (c):

(a) a DNA comprising the nucleotide sequence represented by SEQ ID NO:24;
(b) a DNA hybridizable with the DNA of SEQ ID NO:24 under stringent conditions and encoding a soluble SJ2368 protein; or
(c) a DNA encoding the protein as defined in claim 3.

**11.** A DNA as defined in any one of (a) to (c):

(a) a DNA comprising the nucleotide sequence represented by SEQ ID NO:25;
(b) a DNA hybridizable with the DNA of SEQ ID NO:25 under stringent conditions and encoding the soluble SJ2368 protein; or
(c) a DNA encoding the protein as defined in claim 4.

**12.** A DNA encoding the partial peptide as defined in claim 6.

**13.** A DNA encoding the fusion protein as defined in claim 5 or 7.

**14.** A recombinant vector including the DNA as defined in any one of claims 8 to 13.

**15.** A transformant produced by transforming with the recombinant vector as defined in claim 14.

**16.** An antisense nucleic acid suppressing the expression of the SJ2368 protein.

**17.** An antisense nucleic acid as defined in claim 16 wherein the nucleic acid sequence is a sequence complementary to the entire or a part of the DNA as defined in claim 8 or the DNA comprising the nucleotide sequence of SEQ ID NO:3.

**18.** An antisense nucleic acid as defined in claim 16 wherein the nucleic acid sequence is a sequence complementary to the entire or a part of the DNA as defined in claim 9 or the DNA comprising the nucleotide sequence of SEQ ID NO:16.

**19.** An antibody against the SJ2368 protein.

**20.** An antibody against the soluble SJ2368 protein.

**21.** A method of screening for an agent capable of controlling an activity of the SJ2368 protein comprising contacting said protein or a transformant expressing said protein with a candidate.

**22.** A method of screening for an agent capable of controlling an activity of the soluble SJ2368 protein comprising contacting said protein with a candidate.

**23.** A method of screening for an agent capable of controlling an expression of the gene sj2368 comprising contacting the recombinant vector as defined in claim 14 or the transformant as defined in claim 15 with a candidate.

**24.** A SJ2368 recombinant non-human animal.

**25.** An assay method for the soluble SJ2368 protein in a test sample comprising using the antibody as defined in claim 19 or 20.

**26.** An assay reagent or kit for the soluble SJ2368 protein in a test sample comprising containing the antibody as defined in claim 19 or 20.

Fig. 1

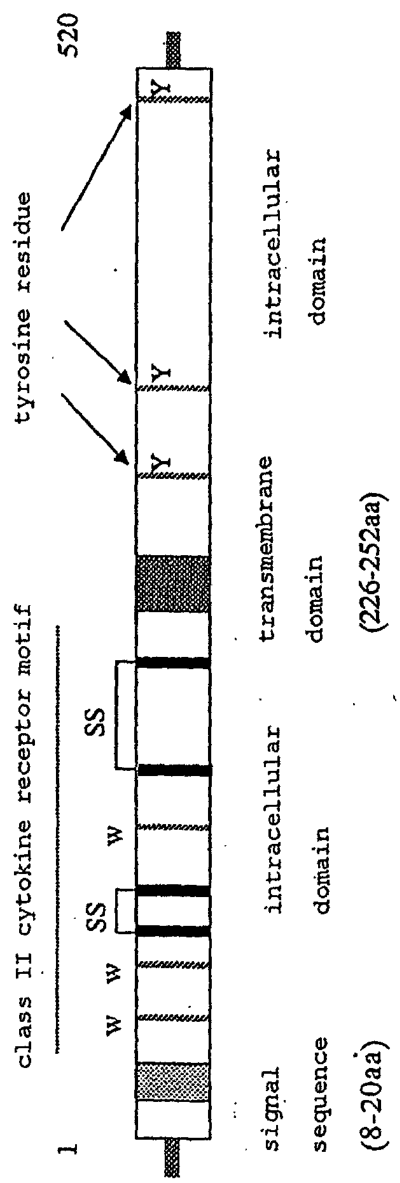
1
520
class II cytokine receptor motif
tyrosine residue
W
W
SS
W
SS
Y
Y
Y
signal
sequence
(8-20aa)
intracellular
domain
transmembrane
domain
(226-252aa)
intracellular
domain

Fig. 2

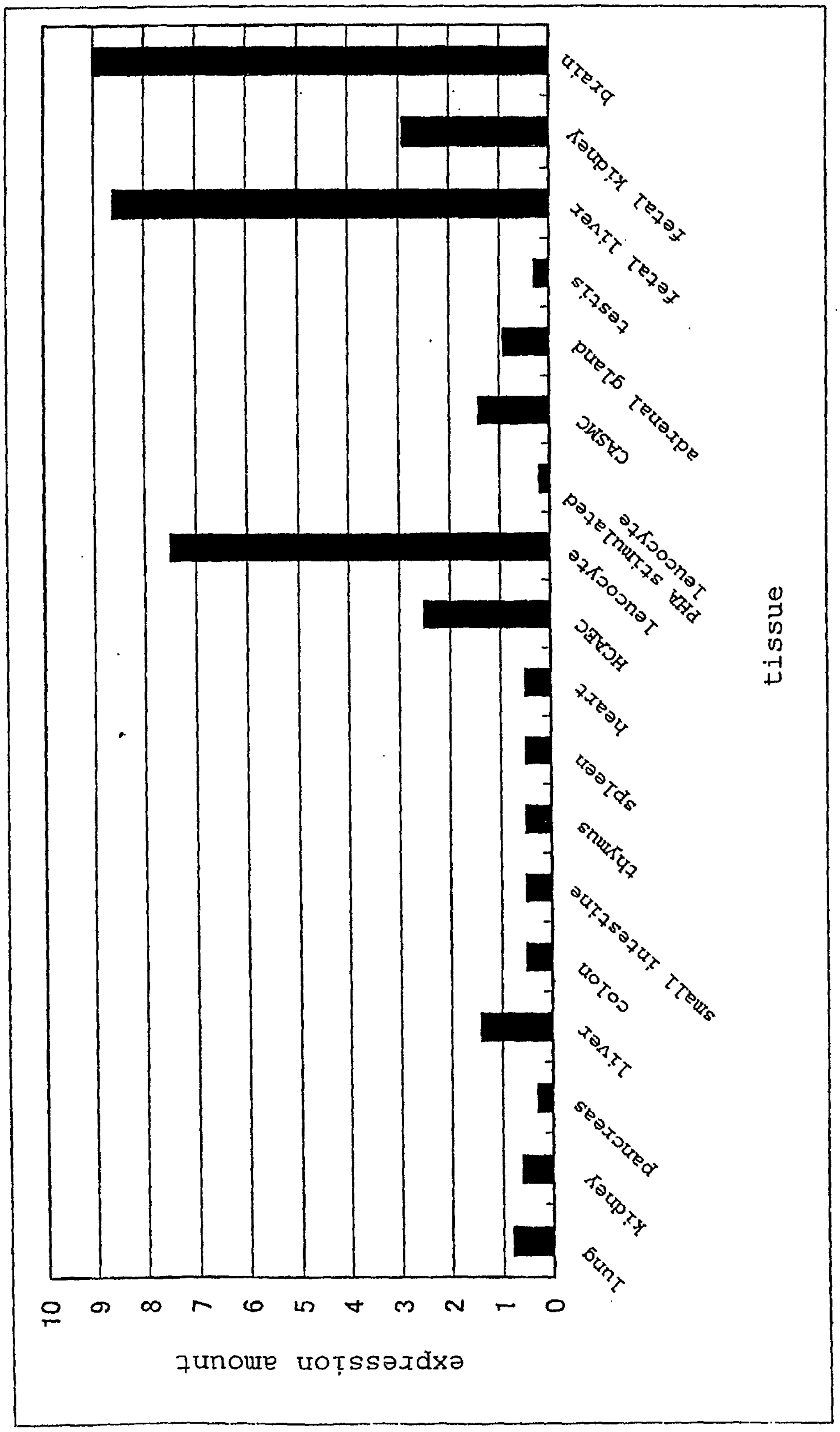
expression amount
0
1
2
3
4
5
6
7
8
9
10
tissue
brain
fetal kidney
fetal liver
testis
adrenal gland
CASMC
PHA stimulated leucocyte
leucocyte
HCAEC
heart
spleen
thymus
small intestine
colon
liver
pancreas
kidney
lung

Fig. 3

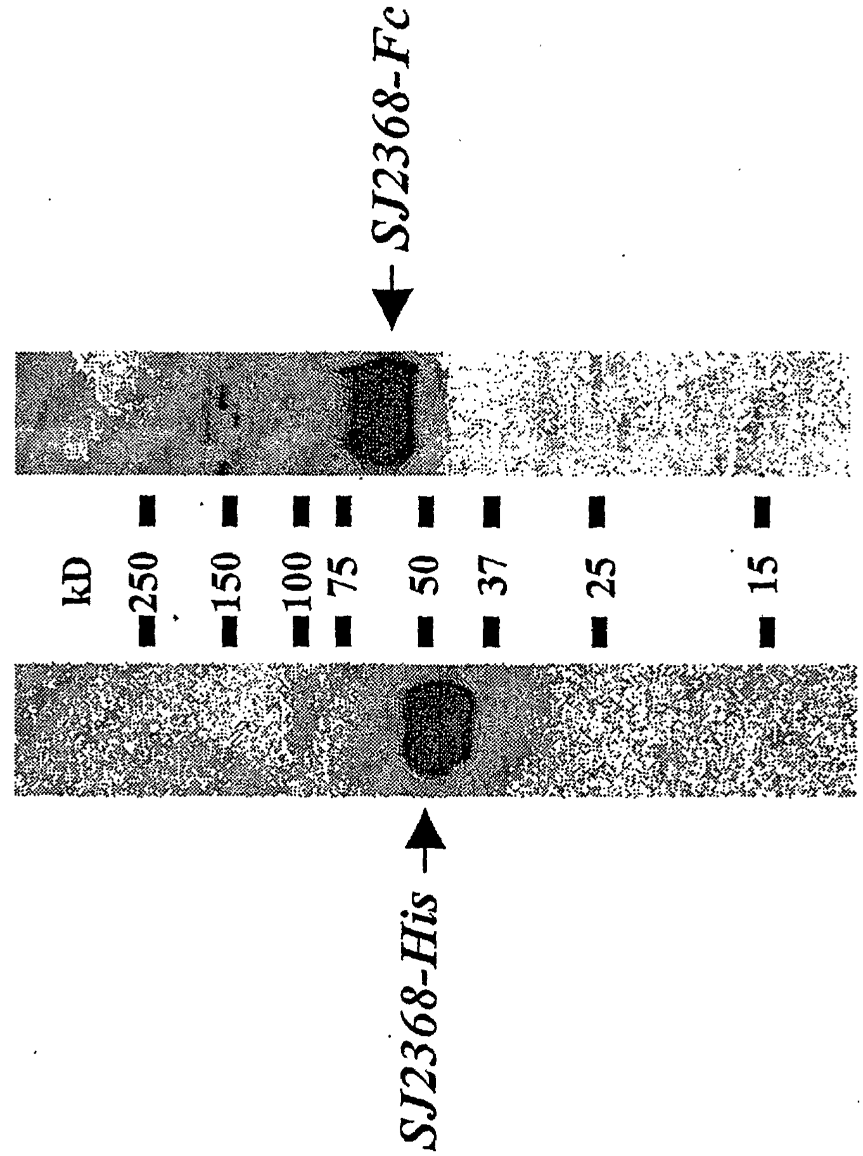

Fig. 4

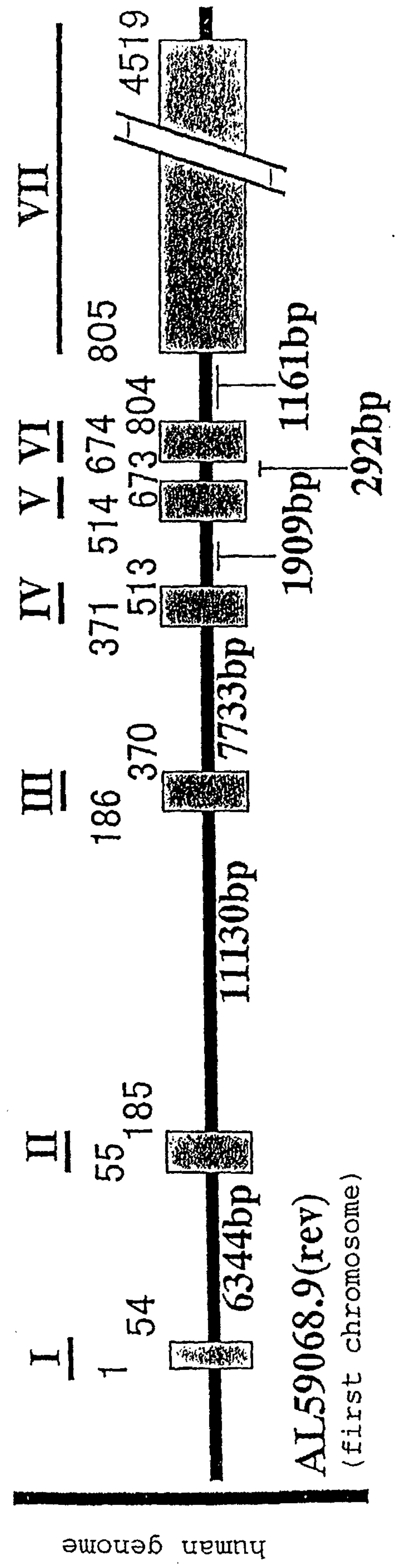
I
II
III
IV
V
VI
VII
1
54
55
185
186
370
371
513
514
673
674
804
805
4519
6344bp
11130bp
7733bp
1909bp
292bp
1161bp
AL59068.9(rev)
(first chromosome)
human genome

Fig. 5

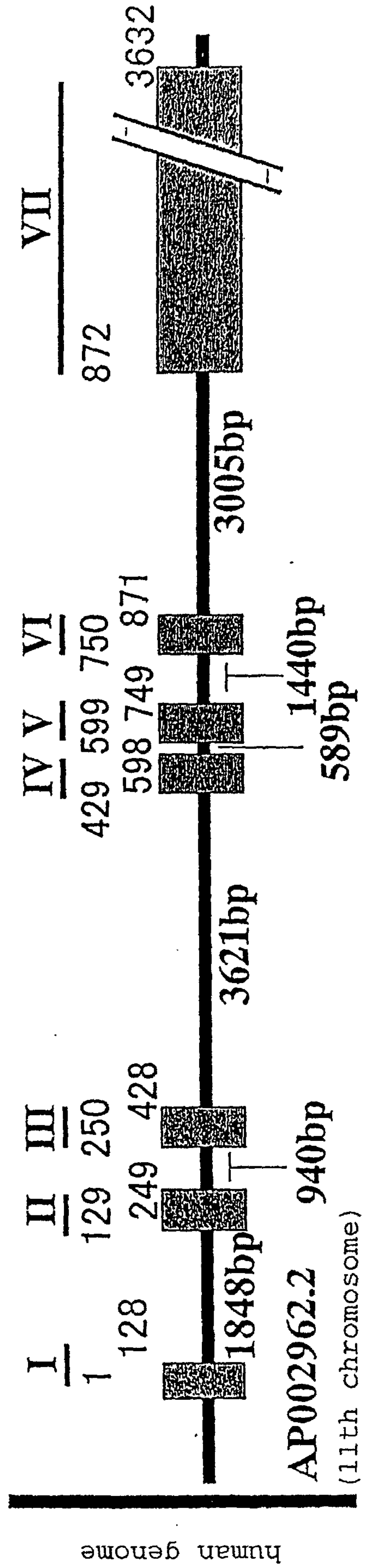
I
II
III
IV
V
VI
VII
1
128
129
249
250
428
429
598
599
749
750
871
872
3632
1848bp
940bp
3621bp
589bp
1440bp
3005bp
AP002962.2
(11th chromosome)
human genome

Fig. 6

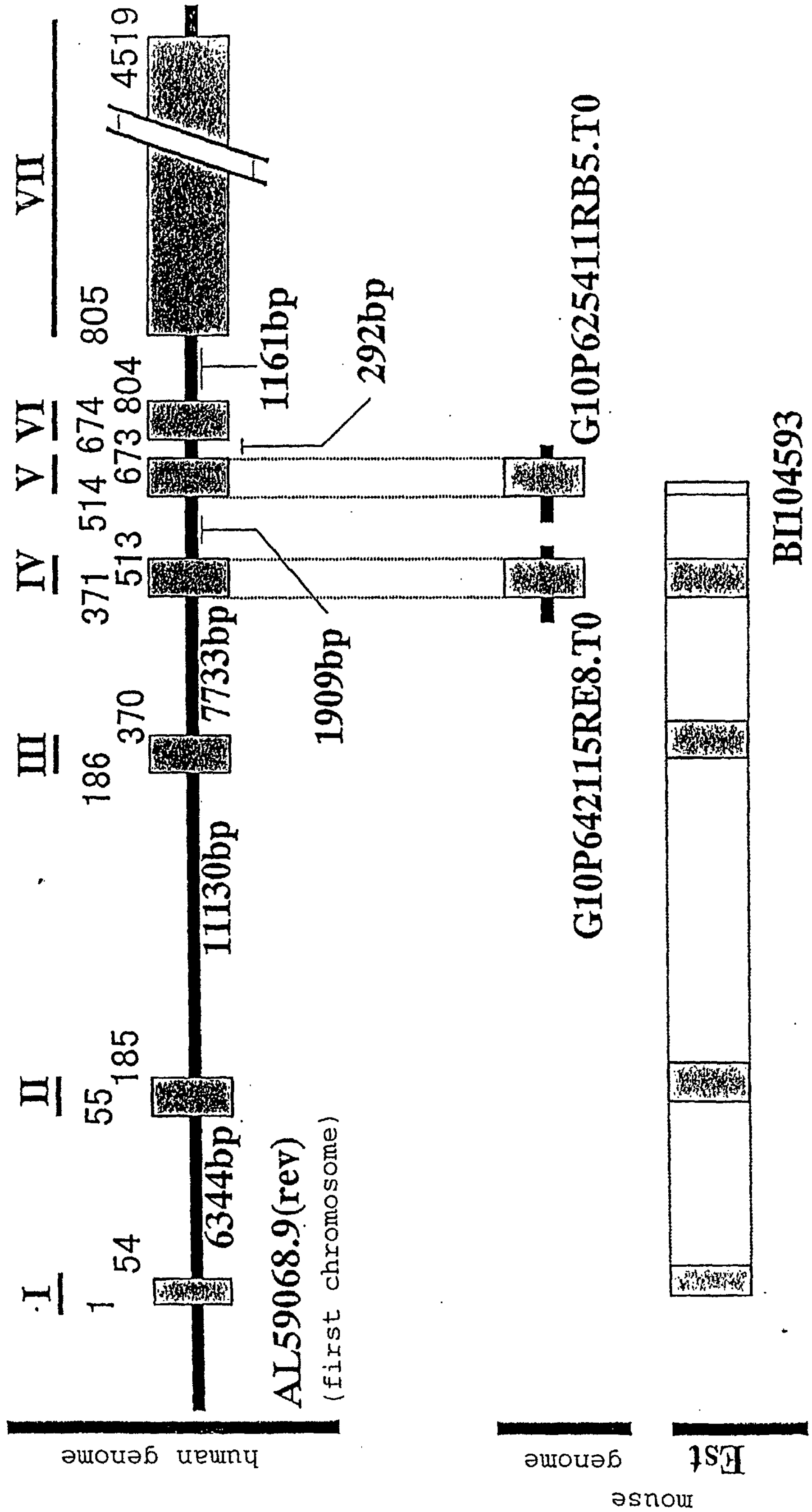

I
II
III
IV
V
VI
VII
1
54
55
185
186
370
371
513
514
673
674
804
805
4519
6344bp
11130bp
7733bp
1909bp
292bp
1161bp
AL59068.9(rev)
(first chromosome)
G10P642115RE8.T0
G10P625411RB5.T0
BI104593
human genome
genome
Est
mouse

Fig. 7

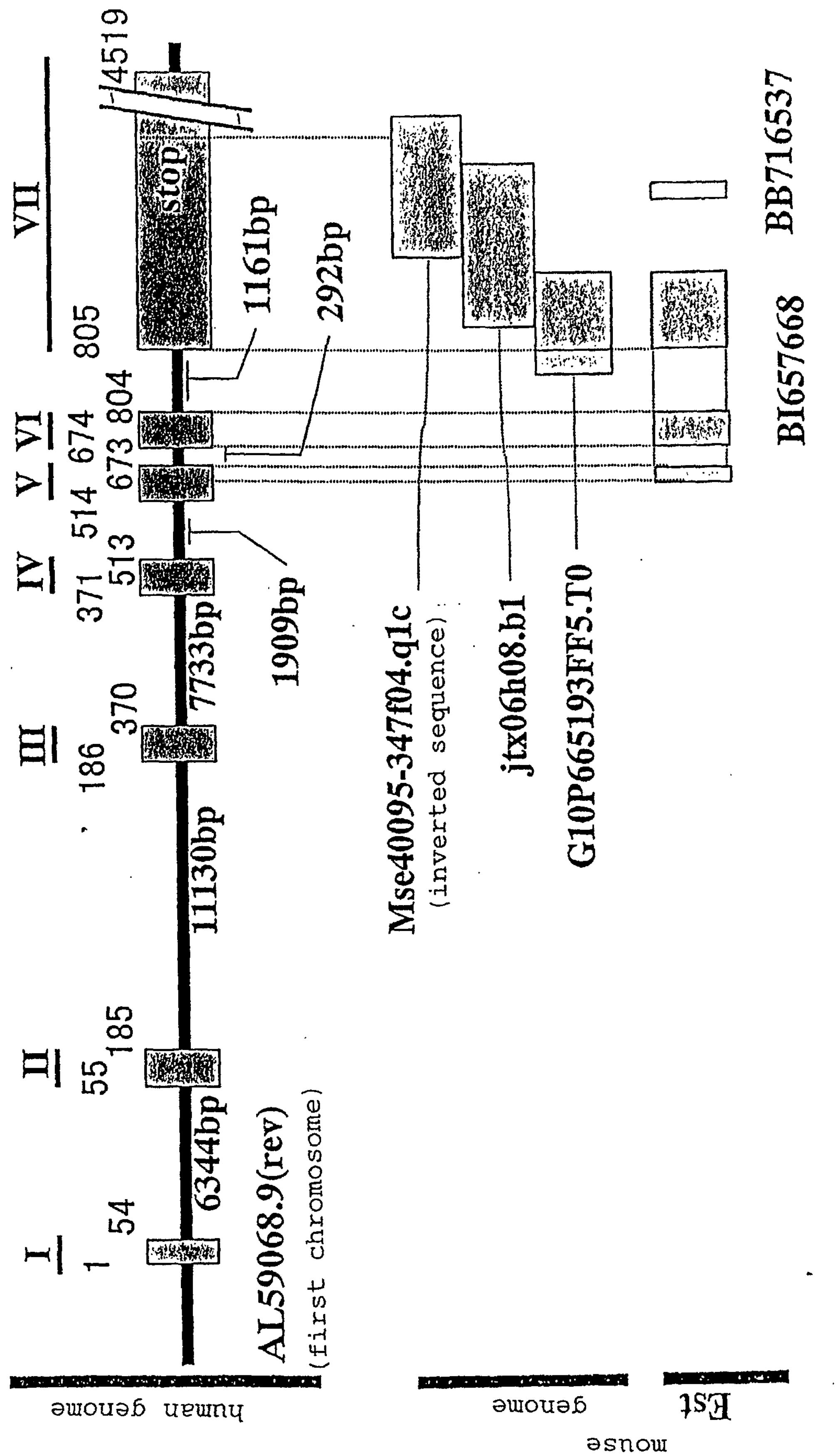
I
II
III
IV
V
VI
VII
1
54
55
185
186
370
371
513
514
673
674
804
805
4519
stop
6344bp
11130bp
7733bp
1909bp
292bp
1161bp
AL59068.9(rev)
(first chromosome)
Mse40095-347f04.q1c
(inverted sequence)
jtx06h08.b1
G10P665193FF5.T0
BI657668
BB716537
human genome
genome
Est
mouse

Fig. 8

```
  1 : MAGPERWGPL LLCLLQAAPG RPRLAPPQNV TLLSQNFSVY LTWLPGLGNP QDVTYFVAYQ SSPTRRRWRE VEECAGTKEL
      *     ** ** ** *** * * ******* ** ** **** ** ******** *    ***** **  *     **  ** *** * *
  1 : MWRADRWAPL LLFLLQSALG RPRLAPPRNV TLFSQNFTVY LTWLPGLGSP PNVTYFVTYQ -SYIKTGWRP VEHCAGIKAL

 81 : LCSMMCLKKQ DLYNKFKGRV RTVSPSSKSP WVESEYLDYL FEVEPAPPVL VLTQTEEILS ANATYQLPPC MPPLDLKYEV
       *  *****    ** ******     *    **  *** ** ** * ** *** * **** * **   ********* ** * *** *
 80 : VCPLMCLKKL NLYSKFKGRV QAASAHGRSP RVESRYLEYL FDVELAPPTL VLTQMEKILR VNATYQLPPC MPSLELKYQV

161 : AFWKEGAGNK TLFPVTPHGQ PVQITLQPAA SEHHCLSART IYTFSVPKYS KFSKPTCFLL EVPEANWAFL VLPSLLILLL
       ***** * * **** ** ** **** **  * * *******  **    ***  ** * *  * * *  * *   **** **
160 : EFWKEGLGSK TLFPDTPYGQ PVQIPLQQGA SRRHCLSART VYTLIDIKYS QFSEPSCIFL EAPGDKRAVL AMPSLLLLLI

241 : VIAAGGVIWK TLMGNPWFQR AKMPRAIDFS GHTHPVATFQ PSRPESVNDL FLCPQKELTR GVRPTPRVRA PATQQTRWKK
        * ** **   ******  * *******    ****** ** **   **  ********   ** * **  *** *
240 : AAVAAGVAWK IMKGNPWFQG VKTPRAIDFS EYRYPVATFQ PSGPEFSDDL ILCPQKELTI RNRPAPQVRN PATLQAGPER

321 : DLAEDEEEED EEDTEDGVSF QPYIEPPSFL GQEHQAPGHS E--AGGVDSG RPRAPLVPSE GSSAWDSSDR SWASTVDSS-
      *  ** * **     ** *  *** * * *          ** *  *****  *   * * ********** ** ** ***
320 : DSTED-EDED TDYDDGDSV  QPYLERPLFI SEKPRVMEHS ETDESGVDSG GPWTSPVGSD GSSAWDSSDR SWSSTGDSSY

398 : WDRAGSSGYL AEKGPGQGPG GDGHQESLPP PEFSKDSGFL EELPEDNLSS WATWGTLPPE PNLVPGGPPV SLQTLTFCW-
       *  ***  *   * * * *  **  ** **  *** * *  **   * **  *   * *    * *  *** *********
399 : KDEVGSSSCL DRKEPDQAPC GDWLQEALPC LEFSEDLGTV EEPLKDGLSG WRISGSLSSK RDLAPVEPPV SLQTLTFCWV

477 : ---------- ---ESSPEEE EEARESEIED SDAGSWGAES TQRTEDRGRT LGHYMAR
                    *   *** ** ***   * ** ** *  **** ***  ** *  *
479 : NNPEGEEEQE DEEEEEEEEE EEDWESEPKG SNAGCWGTSS VQRTEVRGRM LGDYLVR
```

Fig. 9

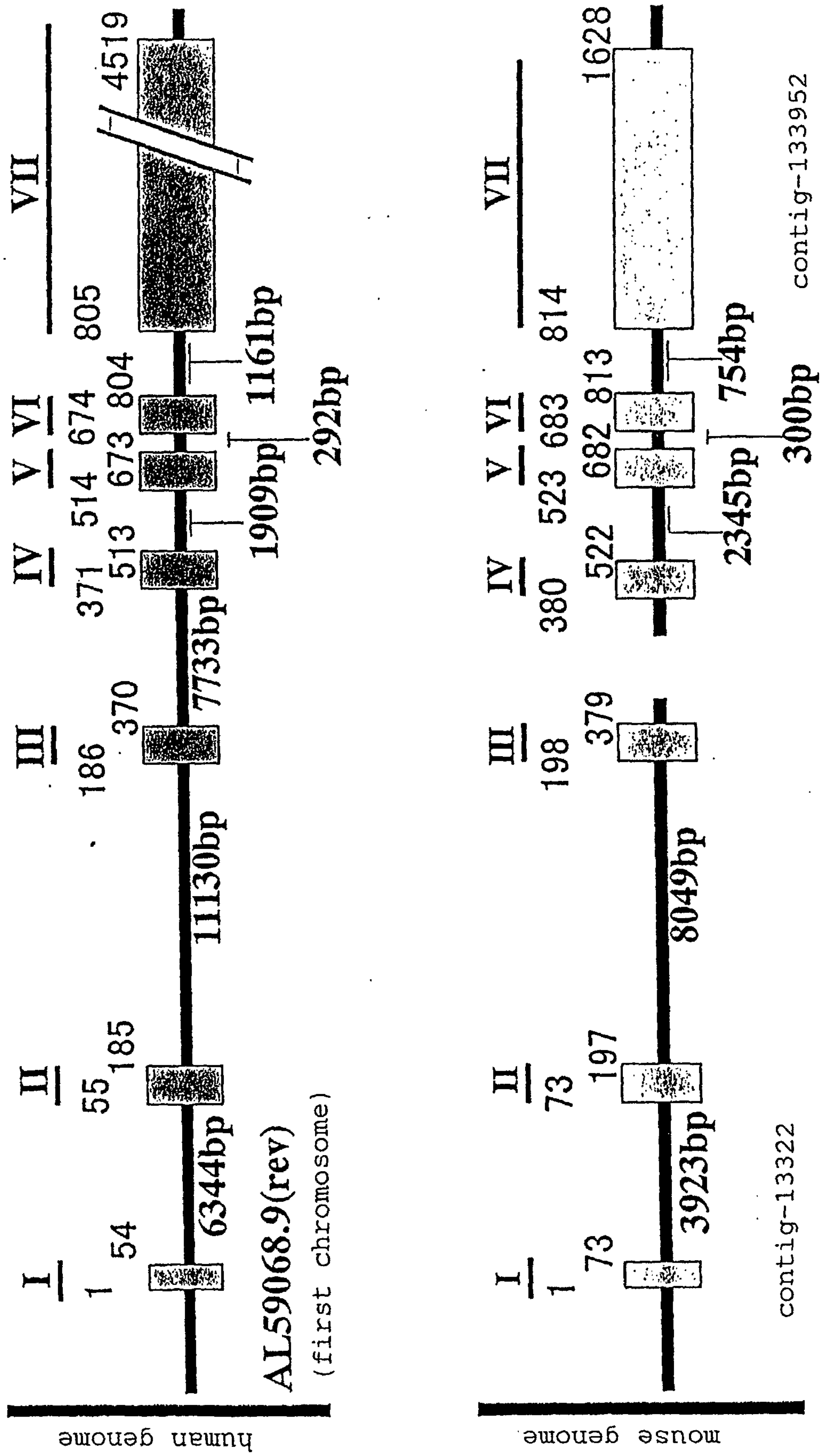
human genome
AL59068.9(rev)
(first chromosome)
I
II
III
IV
V
VI
VII
1
54
55
185
186
370
371
513
514
673
674
804
805
4519
6344bp
11130bp
7733bp
1909bp
292bp
1161bp
mouse genome
contig-13322
contig-133952
1
73
73
197
198
379
380
522
523
682
683
813
814
1628
3923bp
8049bp
2345bp
300bp
754bp

Fig. 10

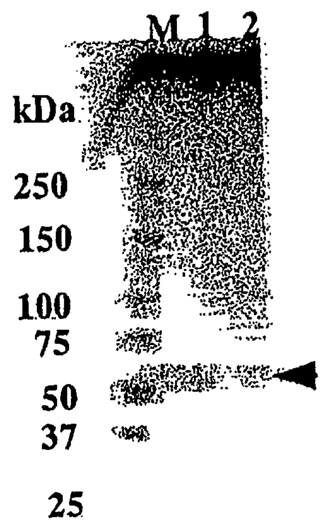

Fig. 11

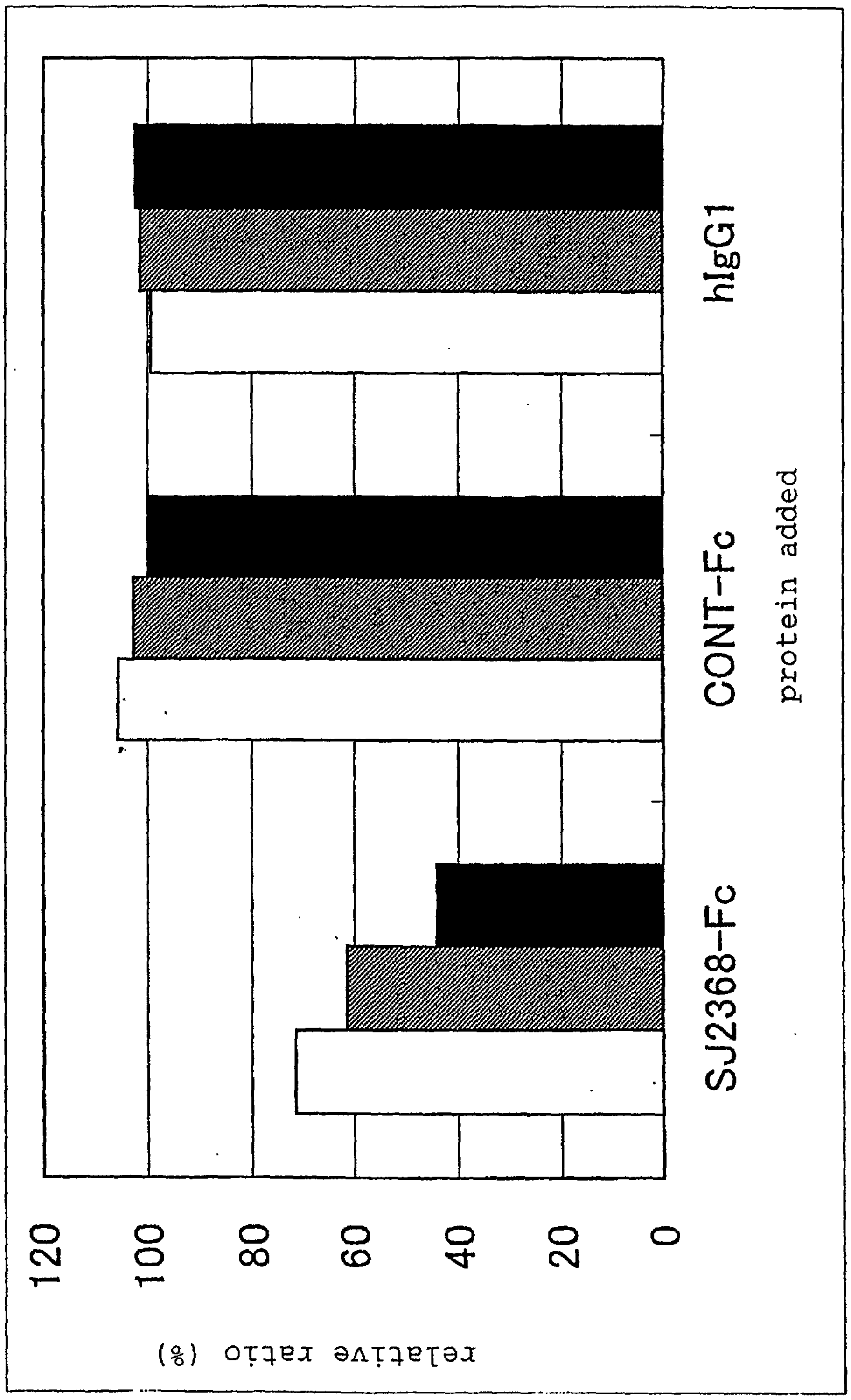
relative ratio (%)
120
100
80
60
40
20
0
SJ2368-Fc
CONT-Fc
hIgG1
protein added

Fig. 12

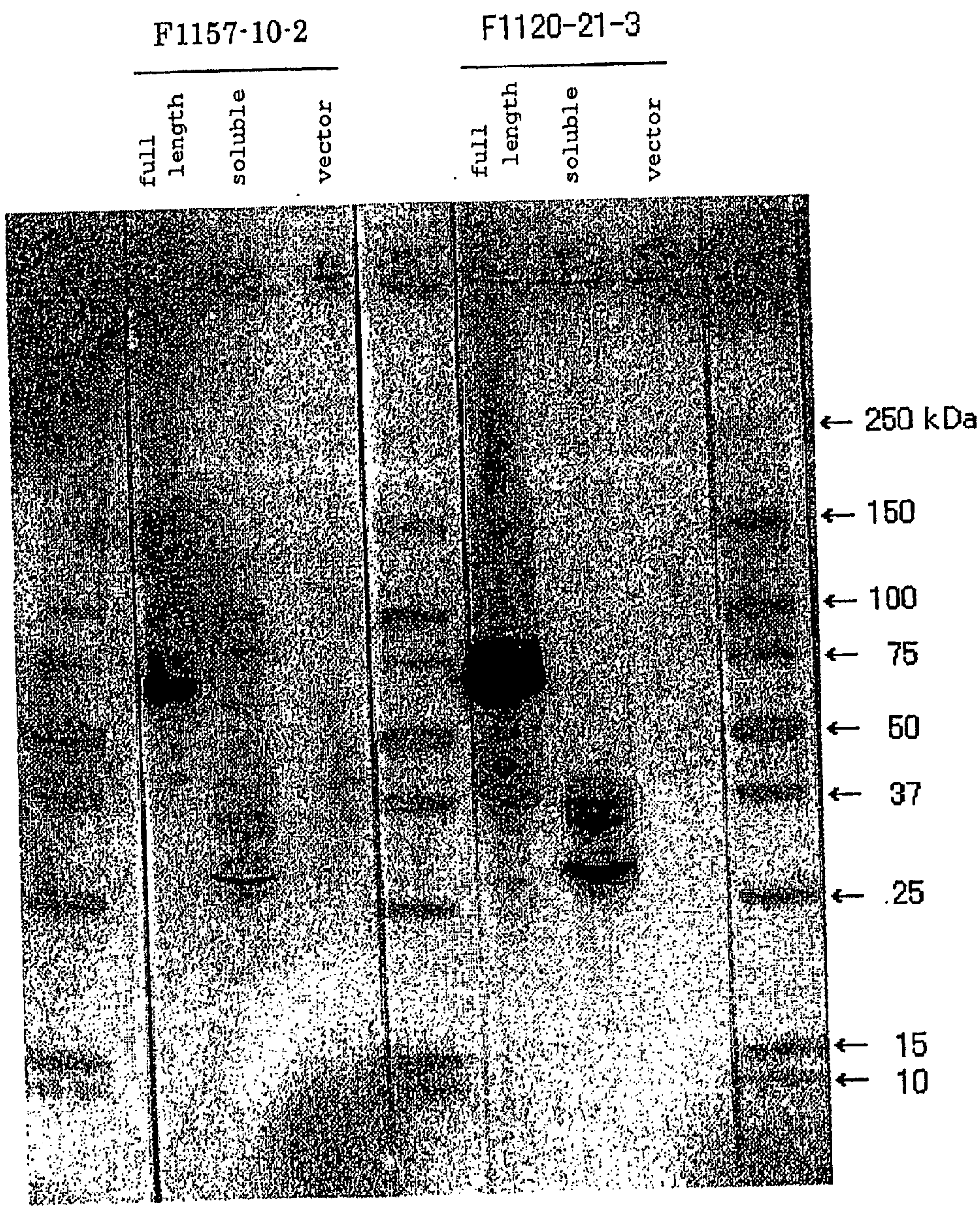
F1157-10-2
full length
soluble
vector
F1120-21-3
full length
soluble
vector
← 250 kDa
← 150
← 100
← 75
← 50
← 37
← 25
← 15
← 10

Fig. 13

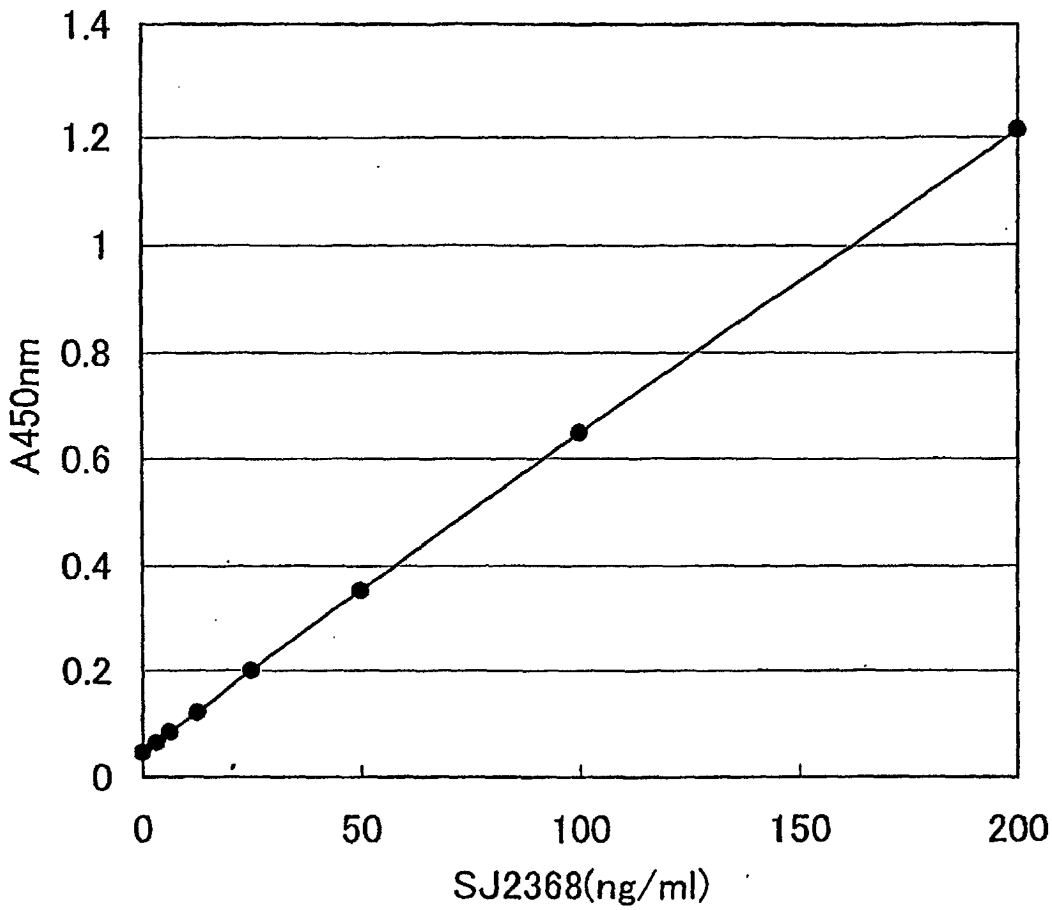

Fig. 14

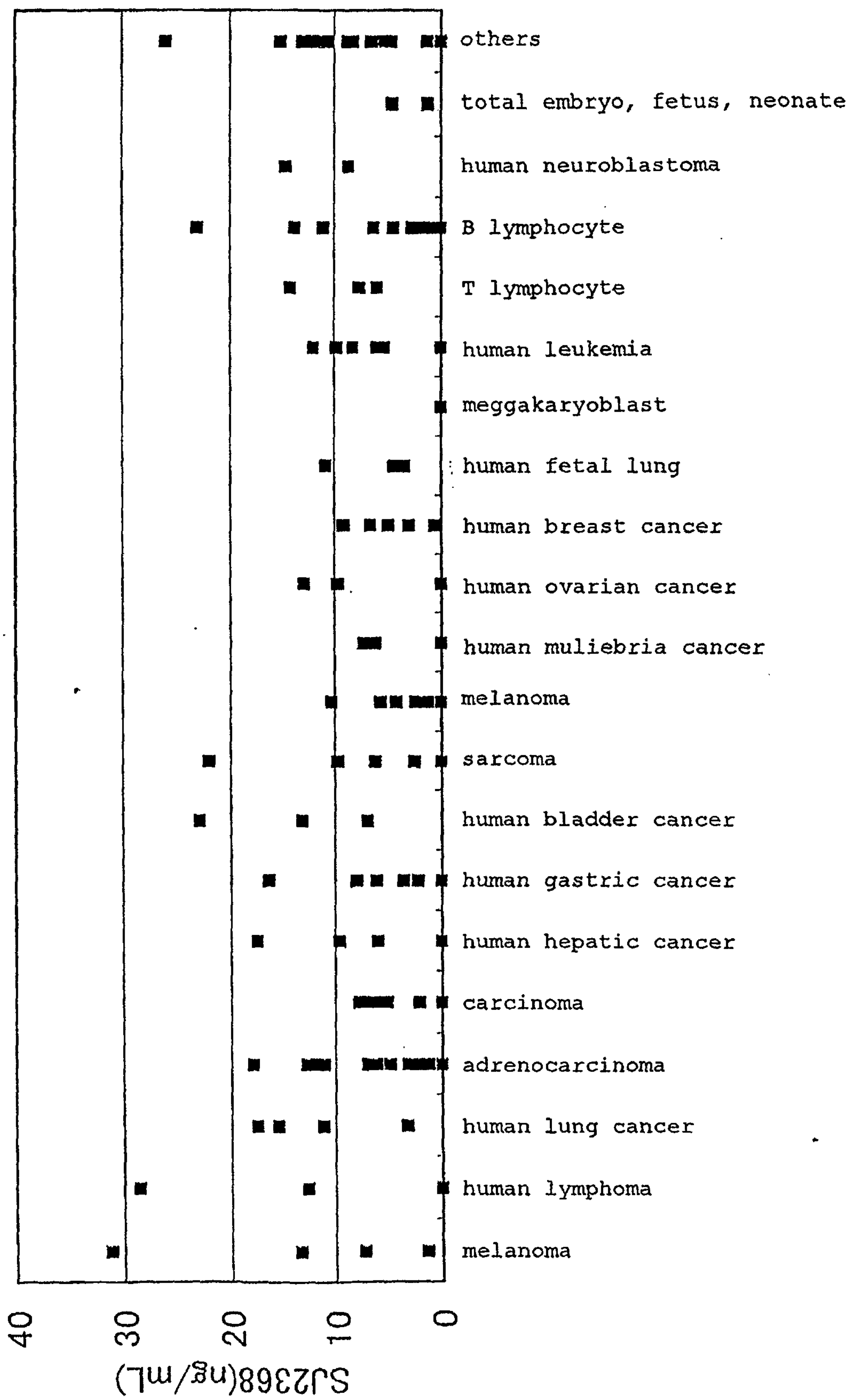

others
total embryo, fetus, neonate
human neuroblastoma
B lymphocyte
T lymphocyte
human leukemia
meggakaryoblast
human fetal lung
human breast cancer
human ovarian cancer
human muliebria cancer
melanoma
sarcoma
human bladder cancer
human gastric cancer
human hepatic cancer
carcinoma
adrenocarcinoma
human lung cancer
human lymphoma
melanoma
40
30
20
10
0
SJ2368(ng/mL)

Fig. 15

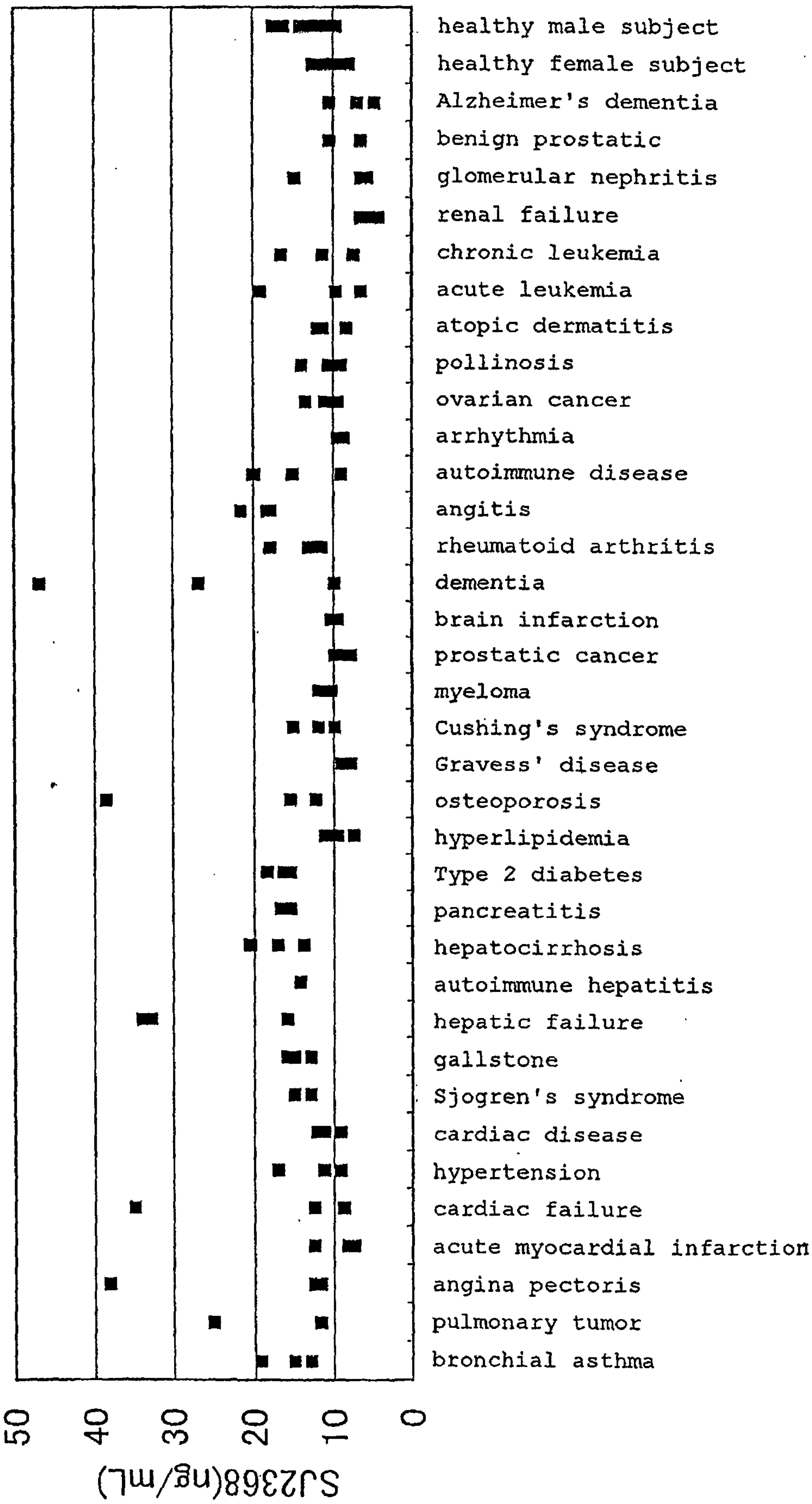
healthy male subject
healthy female subject
Alzheimer's dementia
benign prostatic
glomerular nephritis
renal failure
chronic leukemia
acute leukemia
atopic dermatitis
pollinosis
ovarian cancer
arrhythmia
autoimmune disease
angitis
rheumatoid arthritis
dementia
brain infarction
prostatic cancer
myeloma
Cushing's syndrome
Gravess' disease
osteoporosis
hyperlipidemia
Type 2 diabetes
pancreatitis
hepatocirrhosis
autoimmune hepatitis
hepatic failure
gallstone
Sjogren's syndrome
cardiac disease
hypertension
cardiac failure
acute myocardial infarction
angina pectoris
pulmonary tumor
bronchial asthma
50
40
30
20
10
0
SJ2368(ng/mL)

Fig. 16

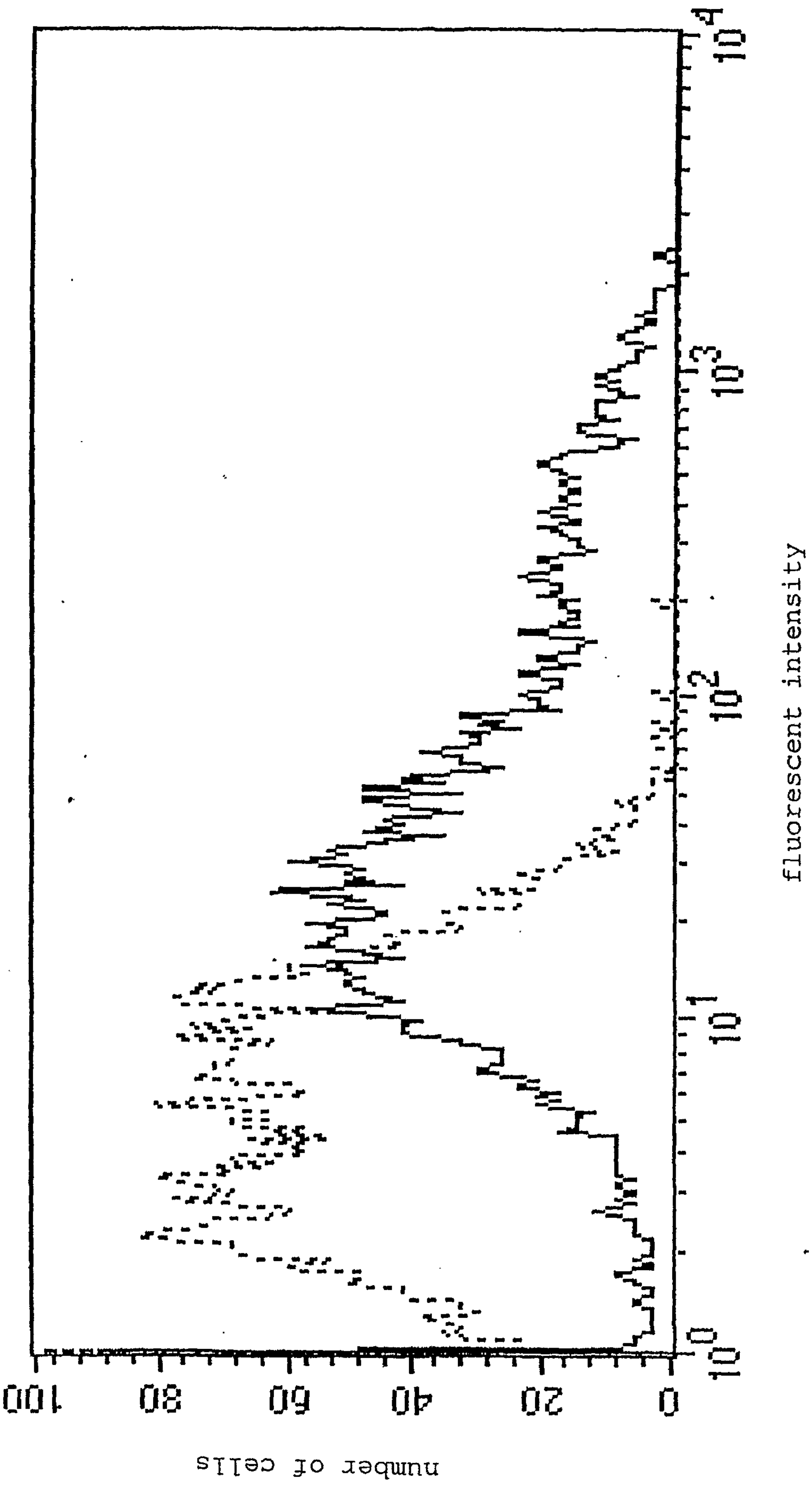
fluorescent intensity
number of cells
0
20
40
60
80
100
10^0
10^1
10^2
10^3
10^4

Fig. 17

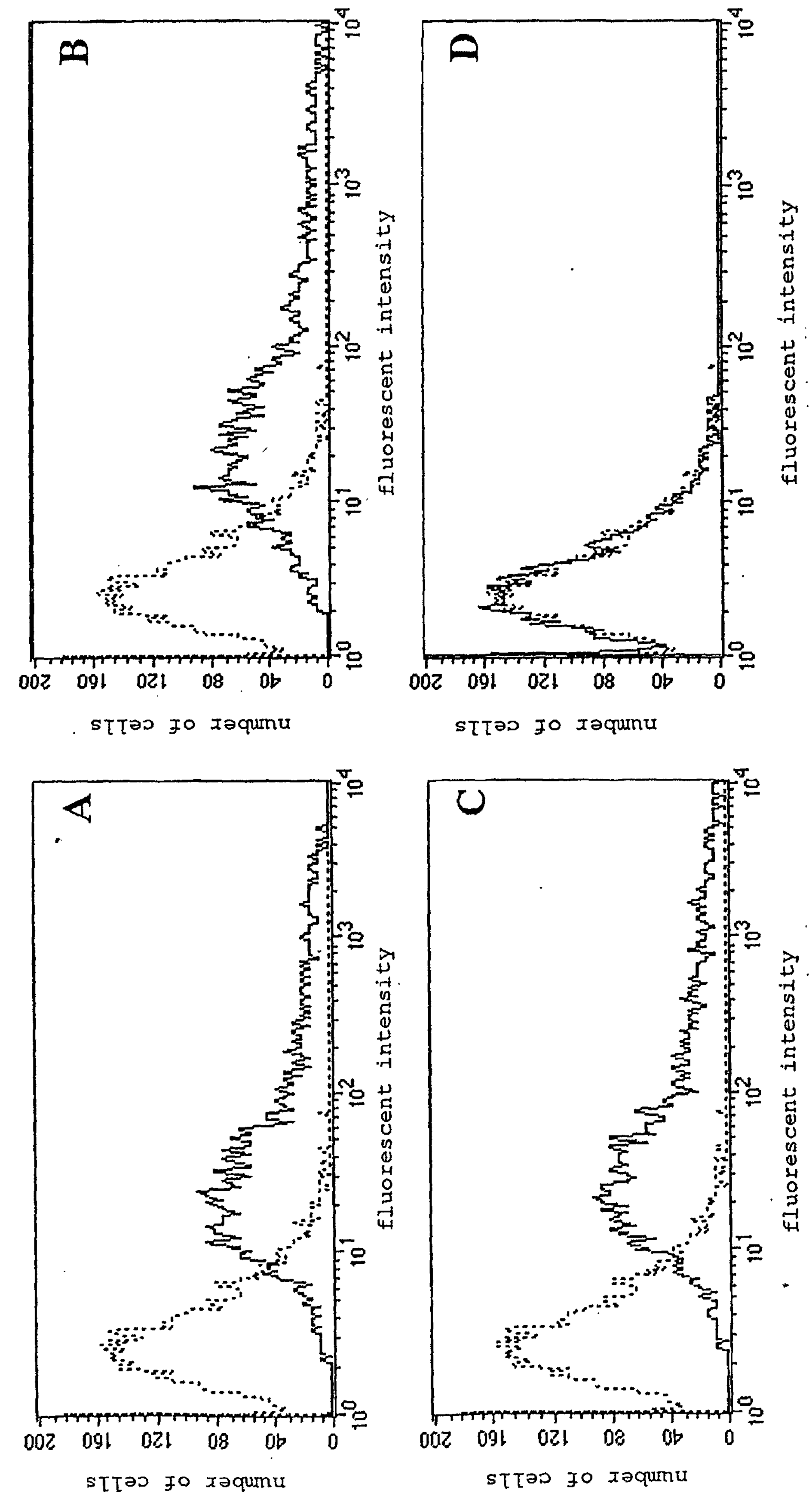
A
B
C
D
number of cells
fluorescent intensity
0
40
80
120
160
200
$10^0$
$10^1$
$10^2$
$10^3$
$10^4$

**INTERNATIONAL SEARCH REPORT**

International application No.
PCT/JP02/10280

A. CLASSIFICATION OF SUBJECT MATTER
Int.Cl7 C12N15/12, C07K14/715, C12N5/10, C12P21/02, C12P21/08, C07K16/28, G01N33/50, G01N33/15, A01K67/027//(C12N15/12, C12R1:91), (C12N5/10, C12R1:91), (C12P21/02, C12R1:91)
According to International Patent Classification (IPC) or to both national classification and IPC

B. FIELDS SEARCHED
Minimum documentation searched (classification system followed by classification symbols)
Int.Cl7 C12N1/00-15/90, C07K14/00-16/46, C12P21/00-08, G01N33/00-98, A01K67/027

Documentation searched other than minimum documentation to the extent that such documents are included in the fields searched

Electronic data base consulted during the international search (name of data base and, where practicable, search terms used)
SwissProt/PIR/GeneSeq, MEDLINE(STN), REGISTRY(STN), Genbank/EMBL/DDBJ/GeneSeq, WPI(DIALOG), BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| P,X<br>P,Y | WO 02/44209 A2 (ZYMOGENETICS, INC.), 06 June, 2002 (06.06.02), & AU 200241533 A | 1,3,5,8,10, 13-15,19, 20,21,22<br>2,4-26 |
| P,X<br>P,Y | WO 02/20569 A2 (SCHERING CORP.), 14 March, 2002 (14.03.02), & AU 200194541 A & US 2002/0142292 A1 | 6,12,14,15<br>1-26 |
| A | Kotenko SERGEI et al., "Identification of the functional interleukin-22(IL-22)receptor complex. The IL-10R2 chain of both the IL-10 and IL-22(IL-10-related T cell-derived inducible factor, IL-TIF)receptor compexes.," Journal of Biological Chemistry, Vol.276, No.4, pages 2725 to 2732, 26 January, 2001 (26.01.01), | 1-26 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>22 November, 2002 (22.11.02) | Date of mailing of the international search report<br>10 December, 2002 (10.12.02) |
| Name and mailing address of the ISA/<br>Japanese Patent Office<br>Facsimile No. | Authorized officer<br><br>Telephone No. |

Form PCT/ISA/210 (second sheet) (July 1998)

**INTERNATIONAL SEARCH REPORT**

International application No.
PCT/JP02/10280

C (Continuation). DOCUMENTS CONSIDERED TO BE RELEVANT

| Category* | Citation of document, with indication, where appropriate, of the relevant passages | Relevant to claim No. |
|---|---|---|
| A | Blumberg H., et al., Interleukin 20: discovery, receptor identification, and role in epidermal function, Cell(2001), Vol.12, No.104, pages 9 to 19 | 1-26 |

Form PCT/ISA/210 (continuation of second sheet) (July 1998)

SEQUENCE LISTING

<110> Mochida Pharmaceutical Co., Ltd.
<110> Kazusa DNA Research Institute
<120> A novel class II cytokine receptor
<130> SAP-689-PCT
<140>
<141> 2002-10-02
<150> JP 2001306851
<151> 2001-10-02
<150> JP 2002204385
<151> 2002-07-12
<160> 19
<170> PatentIn Ver. 2.1

<210> 1
<211> 1560
<212> DNA
<213> homo sapiense
<400> 1

```
atggcggggc ccgagcgctg gggcccccctg ctcctgtgcc tgctgcaggc cgctccaggg   60
aggccccgtc tggcccctcc ccagaatgtg acgctgctct cccagaactt cagcgtgtac  120
ctgacatggc tcccagggct tggcaacccc caggatgtga cctattttgt ggcctatcag  180
agctctccca cccgtagacg gtggcgcgaa gtggaagagt gtgcgggaac caaggagctg  240
ctatgttcta tgatgtgcct gaagaaacag gacctgtaca acaagttcaa gggacgcgtg  300
cggacggttt ctcccagctc caagtccccc tgggtggagt ccgaatacct ggattacctt  360
tttgaagtgg agccggcccc acctgtcctg gtgctcaccc agacggagga gatcctgagt  420
gccaatgcca cgtaccagct gccccccctgc atgcccccac tggatctgaa gtatgaggtg  480
gcattctgga aggaggggc cggaaacaag accctatttc cagtcactcc ccatggccag  540
ccagtccaga tcactctcca gccagctgcc agcgaacacc actgcctcag tgccagaacc  600
atctacacgt tcagtgtccc gaaatacagc aagttctcta agcccacctg cttcttgctg  660
gaggtcccag aagccaactg ggctttcctg gtgctgccat cgcttctgat actgctgtta  720
gtaattgccg caggggtgt gatctggaag accctcatgg ggaacccctg gtttcagcgg  780
gcaaagatgc cacgggccct ggacttttct ggacacacac accctgtggc aacctttcag  840
cccagcagac cagagtccgt gaatgacttg ttcctctgtc cccaaaagga actgaccaga  900
ggggtcaggc cgacgcctcg agtcagggcc ccagccaccc aacagacaag atggaagaag  960
```

gaccttgcag aggacgaaga ggaggaggat gaggaggaca cagaagatgg cgtcagcttc 1020
cagccctaca ttgaaccacc ttctttcctg gggcaagagc accaggctcc agggcactcg 1080
gaggctggtg gggtggactc agggaggccc agggctcctc tggtcccaag cgaaggctcc 1140
tctgcttggg attcttcaga cagaagctgg gccagcactg tggactcctc ctgggacagg 1200
gctgggtcct ctggctattt ggctgagaag gggccaggcc aagggccggg tggggatggg 1260
caccaagaat ctctcccacc acctgaattc tccaaggact cgggtttcct ggaagagctc 1320
ccagaagata acctctcctc ctgggccacc tggggcacct taccaccgga gccgaatctg 1380
gtccctgggg gacccccagt ttctcttcag acactgacct tctgctggga aagcagccct 1440
gaggaggaag aggaggcgag ggaatcagaa attgaggaca gcgatgcggg cagctggggg 1500
gctgagagca cccagaggac cgaggacagg ggccggacat tggggcatta catggccagg 1560

<210> 2
<211> 520
<212> PRT
<213> homo sapiense
<400> 2

Met Ala Gly Pro Glu Arg Trp Gly Pro Leu Leu Leu Cys Leu Leu Gln
1 5 10 15
Ala Ala Pro Gly Arg Pro Arg Leu Ala Pro Pro Gln Asn Val Thr Leu
20 25 30
Leu Ser Gln Asn Phe Ser Val Tyr Leu Thr Trp Leu Pro Gly Leu Gly
35 40 45
Asn Pro Gln Asp Val Thr Tyr Phe Val Ala Tyr Gln Ser Ser Pro Thr
50 55 60
Arg Arg Arg Trp Arg Glu Val Glu Glu Cys Ala Gly Thr Lys Glu Leu
65 70 75 80
Leu Cys Ser Met Met Cys Leu Lys Lys Gln Asp Leu Tyr Asn Lys Phe
85 90 95
Lys Gly Arg Val Arg Thr Val Ser Pro Ser Ser Lys Ser Pro Trp Val
100 105 110
Glu Ser Glu Tyr Leu Asp Tyr Leu Phe Glu Val Glu Pro Ala Pro Pro
115 120 125
Val Leu Val Leu Thr Gln Thr Glu Glu Ile Leu Ser Ala Asn Ala Thr
130 135 140
Tyr Gln Leu Pro Pro Cys Met Pro Pro Leu Asp Leu Lys Tyr Glu Val
145 150 155 160

Ala Phe Trp Lys Glu Gly Ala Gly Asn Lys Thr Leu Phe Pro Val Thr
165 170 175
Pro His Gly Gln Pro Val Gln Ile Thr Leu Gln Pro Ala Ala Ser Glu
180 185 190
His His Cys Leu Ser Ala Arg Thr Ile Tyr Thr Phe Ser Val Pro Lys
195 200 205
Tyr Ser Lys Phe Ser Lys Pro Thr Cys Phe Leu Leu Glu Val Pro Glu
210 215 220
Ala Asn Trp Ala Phe Leu Val Leu Pro Ser Leu Leu Ile Leu Leu Leu
225 230 235 240
Val Ile Ala Ala Gly Gly Val Ile Trp Lys Thr Leu Met Gly Asn Pro
245 250 255
Trp Phe Gln Arg Ala Lys Met Pro Arg Ala Leu Asp Phe Ser Gly His
260 265 270
Thr His Pro Val Ala Thr Phe Gln Pro Ser Arg Pro Glu Ser Val Asn
275 280 285
Asp Leu Phe Leu Cys Pro Gln Lys Glu Leu Thr Arg Gly Val Arg Pro
290 295 300
Thr Pro Arg Val Arg Ala Pro Ala Thr Gln Gln Thr Arg Trp Lys Lys
305 310 315 320
Asp Leu Ala Glu Asp Glu Glu Glu Glu Asp Glu Glu Asp Thr Glu Asp
325 330 335
Gly Val Ser Phe Gln Pro Tyr Ile Glu Pro Pro Ser Phe Leu Gly Gln
340 345 350
Glu His Gln Ala Pro Gly His Ser Glu Ala Gly Gly Val Asp Ser Gly
355 360 365
Arg Pro Arg Ala Pro Leu Val Pro Ser Glu Gly Ser Ser Ala Trp Asp
370 375 380
Ser Ser Asp Arg Ser Trp Ala Ser Thr Val Asp Ser Ser Trp Asp Arg
385 390 395 400
Ala Gly Ser Ser Gly Tyr Leu Ala Glu Lys Gly Pro Gly Gln Gly Pro
405 410 415
Gly Gly Asp Gly His Gln Glu Ser Leu Pro Pro Pro Glu Phe Ser Lys
420 425 430
Asp Ser Gly Phe Leu Glu Glu Leu Pro Glu Asp Asn Leu Ser Ser Trp
435 440 445

Ala Thr Trp Gly Thr Leu Pro Pro Glu Pro Asn Leu Val Pro Gly Gly
450 455 460
Pro Pro Val Ser Leu Gln Thr Leu Thr Phe Cys Trp Glu Ser Ser Pro
465 470 475 480
Glu Glu Glu Glu Glu Ala Arg Glu Ser Glu Ile Glu Asp Ser Asp Ala
485 490 495
Gly Ser Trp Gly Ala Glu Ser Thr Gln Arg Thr Glu Asp Arg Gly Arg
500 505 510
Thr Leu Gly His Tyr Met Ala Arg
515 520

<210> 3
<211> 4519
<212> DNA
<213> homo sapiense
<400> 3
gccatggcgg ggcccgagcg ctggggcccc ctgctcctgt gcctgctgca ggccgctcca 60
gggaggcccc gtctggcccc tccccagaat gtgacgctgc tctcccagaa cttcagcgtg 120
tacctgacat ggctcccagg gcttggcaac ccccaggatg tgacctattt tgtggcctat 180
cagagctctc ccacccgtag acggtggcgc gaagtggaag agtgtgcggg aaccaaggag 240
ctgctatgtt ctatgatgtg cctgaagaaa caggacctgt acaacaagtt caagggacgc 300
gtgcggacgg tttctcccag ctccaagtcc ccctgggtgg agtccgaata cctggattac 360
ctttttgaag tggagccggc cccacctgtc ctggtgctca cccagacgga ggagatcctg 420
agtgccaatg ccacgtacca gctgcccccc tgcatgcccc cactggatct gaagtatgag 480
gtggcattct ggaaggaggg ggccggaaac aagaccctat ttccagtcac tccccatggc 540
cagccagtcc agatcactct ccagccagct gccagcgaac accactgcct cagtgccaga 600
accatctaca cgttcagtgt cccgaaatac agcaagttct ctaagcccac ctgcttcttg 660
ctggaggtcc cagaagccaa ctgggctttc ctggtgctgc catcgcttct gatactgctg 720
ttagtaattg ccgcaggggg tgtgatctgg aagaccctca tggggaaccc ctggtttcag 780
cgggcaaaga tgccacgggc cctggacttt tctggacaca cacaccctgt ggcaaccttt 840
cagcccagca gaccagagtc cgtgaatgac ttgttcctct gtccccaaaa ggaactgacc 900
agaggggtca ggccgacgcc tcgagtcagg gccccagcca cccaacagac aagatggaag 960
aaggaccttg cagaggacga agaggaggag gatgaggagg acacagaaga tggcgtcagc 1020
ttccagccct acattgaacc accttctttc ctggggcaag agcaccaggc tccagggcac 1080
tcggaggctg gtggggtgga ctcagggagg cccagggctc ctctggtccc aagcgaaggc 1140
tcctctgctt gggattcttc agacagaagc tgggccagca ctgtggactc ctcctgggac 1200

agggctgggt cctctggcta tttggctgag aaggggccag gccaagggcc gggtggggat 1260
gggcaccaag aatctctccc accacctgaa ttctccaagg actcgggttt cctggaagag 1320
ctcccagaag ataacctctc ctcctgggcc acctggggca ccttaccacc ggagccgaat 1380
ctggtccctg ggggaccccc agtttctctt cagacactga ccttctgctg ggaaagcagc 1440
cctgaggagg aagaggaggc gagggaatca gaaattgagg acagcgatgc gggcagctgg 1500
ggggctgaga gcacccagag gaccgaggac aggggccgga cattggggca ttacatggcc 1560
aggtgagctg tccccgaca tcccaccgaa tctgatgctg ctgctgcctt tgcaaggact 1620
actgggcttc ccaagaaact caagagcctc cgtacctccc ctgggcggcg gaggggcatt 1680
gcacttccgg gaagcccacc tagcggctgt ttgcctgtcg ggctgagcaa taagatgccc 1740
ctccctcctg tgacccgccc tctttaggct gagctataag aggggtggac acagggtggg 1800
ctgaggtcag aggttggtgg ggtgtcatca cccccattgt ccctagggtg acaggccagg 1860
gggaaaaatt atccccggac aacatgaaac aggtgaggtc aggtcactgc ggacatcaag 1920
ggcggacacc accaaggggc cctctggaac ttgagaccac tggaggcaca cctgctatac 1980
ctcatgcctt tcccagcagc cactgaactc cccatccca gggctcagcc tcctgattca 2040
tgggtcccct agttaggccc agataaaaat ccagttggct gagggttttg gatgggaagg 2100
gaagggtggc tgtcctcaaa tcctggtctt tggagtcatg gcactgtacg gttttagtgt 2160
cagacagacc ggggttcaaa tcccagctct gctgttcact ggttgtatga tcttggggaa 2220
gacatcttcc ttctctgcct cggcttcctc atctgcagct acgcctgggt gtggtgaggg 2280
ttctagggga tctcagatgt gtgtagcacg gagcctgctg tgtcctgggt gctctctacg 2340
tggtggccgg tagaattctc catctatcca ggctccagga gacccctggg catctcccac 2400
ctgtggcccc taaacccaga gtgactgaga gcacttacca ttcagcttgt ctcatcccca 2460
gtctacctcc ttccttctac cctcactgcc tcccagtcag gagagtgagc tctcagaagc 2520
cagagcccca cccaagggga ccctggtctc tccgccttca cctagcaatg ggaaccctgc 2580
ttcccagggg aggaaccaac tgctccacct tctagggacc cagtttgttg gagtaggaca 2640
gtaacatggc aggaatcgga cttctgggcc tgtaatccca gtttggatgg cacgttagac 2700
tcttggttga ctgttgtggt ccttagaagt cccattctcc cttccagtta tgagaaacca 2760
atgccttcta gattcaggtg actatcctta cctgggggtg ctgatgcatc ctcagttaac 2820
ctacacccac ctgaatatag atgagcgtag ctgagttttc acccgtagga ccgaagtgtt 2880
ttgtggtgga gtatctgaac aaccttggct ctgtggccat tcaacctgcc aggactaaca 2940
tttctggatt tgtgaagaag ggatcttcaa agccattgaa cccacagagc tgtgttgctt 3000
taaagccacc acaagggtac agcattaaat ggcagaactg gaaaagcttc ttagggcatc 3060
tcatccaggg attctcaaac catgtccccc agaggccttg ggctgcagtt gcagggggcg 3120
ccatggggct ataggagcct cccactttca ccagagcagc ctcactgtgc cctgattcac 3180
acactgtggc tttccacgtg aggttttgtt tagagggatc cactactcaa gaaaaagtta 3240
gcaaaccact ccttttgttg caaaggagct gaggtcaagg gtggcaaagg cacttgtcca 3300
aggtcgccca gcagtgctgc tctgatgact tgtgcacatc cccaaggta agagcttcga 3360

tctctgcaca gccgggccaa cctctgaccc cttgtccatg tcagtaaaat atgaaggtca 3420
cagccaggat ttctaagggt caggaggcct tcaccgctgc tggggcacac acacacatgc 3480
atacacacat acgacacaca cctgtgtctc cccaggggtt ttccctgcag tgaggcttgt 3540
ccagatgatt gagcccagga gaggaagaac aaacaaacta cggagctggg gagggctgtg 3600
gcttggggcc agctcccagg gaaattccca gacctgtacc gatgttctct ctggcaccag 3660
ccgagctgct tcgtggaggt aacttcaaaa aagtaaaagc tatcatcagc atcatcttag 3720
acttgtatga aataaccact ccgtttctat tcttaaacct taccattttt gttttgtttt 3780
gttttttga gtcggagttt tgttcttttt gcctaggctg gagtgcagtg gtacaatctc 3840
ggctcactgc aacctccacc tcccgggttc aagtgattct cctgcctcag cctcccaagt 3900
agctgggatt acaggcaccc gccaccacac ctggctaatt tttttgtatt tttagtagag 3960
acggggtttc accatgttgg ccaggctggt ctcgaactcc tgacctcagg tgatccgccc 4020
gcctcggcct cccaaagtgc tgggattaca ggcgtgagcc accgcgccca gccaaacctt 4080
actatttttt taaagaattt tttccagagt ttaatttctg acatagctta agttttccag 4140
taactctaaa ctccatctcc tttatcgtca ttaagtcatt cacaaaaagc caggagaagc 4200
atttggaaag ggcatgataa tcagtataat aatttgcctt gtgtggtcag cacttaactg 4260
ttacaaagcc ctttcacgtg cacagcaggt gggaactgcg cggtgtgggc tgggcctgcg 4320
ctggaagcat atcccgtgaa aagtgttagt gccttaggtg aaagcaacat gtatcccttt 4380
agactactaa cggtatatgt tgttcttatg tatttgtatt tatttctatt ttttctatgt 4440
ttatgtcata tttaaacgat atcctactgc ttgttggtat taccctaaac tgtttaaata 4500
aagagctcta tttttaaag 4519

<210> 4
<211> 44
<212> DNA
<400> 4
gactagttct agatcgcgag cggccgccct tttttttttt tttt 44

<210> 5
<211> 23
<212> DNA
<400> 5
aagctatgtc agaaattaaa ctc 23

<210> 6
<211> 34
<212> DNA

<400> 6
ccgctcgagc aggaaggcca tggcggggcc cgag 34

<210> 7
<211> 33
<212> DNA
<400> 7
cgcggatccg gcttctggga cctccagcaa gaa 33

<210> 8
<211> 34
<212> DNA
<400> 8
ccggaattca ggaaggccat ggcggggccc gagc 34

<210> 9
<211> 17
<212> DNA
<400> 9
cctttctgg gaaatac 17

<210> 10
<211> 16
<212> DNA
<400> 10
atgtatttcc cagaaa 16

<210> 11
<211> 661
<212> DNA
<213> mouse

<400> 11
gccccaggat cgaaatgtgg cgggccgacc ggtgggcgcc cctactcctg ttcctgttgc 60
agagcgccct aggaaggccc cgtctagccc cacccagaaa cgtgacactc ttctcccaga 120
acttcactgt ttacctgaca tggcttccgg ggcttgggag ccccccaaat gtgacctatt 180

tcgtgaccta ccaaagctat atcaaaaccg gttggcgacc agtggagcat tgtgcaggta 240
tcaaggctct ggtgtgtccc ctgatgtgcc tgaagaaact gaacctgtac tccaagttca 300
aaggacgagt acaggcagct tccgcacacg gcaggtcccc acgggtggag tcccggtacc 360
tggaatacct ttttgacgtg gagctagccc cgcccacccct ggtgctcacc cagatggaga 420
agatcctaag ggtcaacgct acctaccagc tgccaccttg catgccgtcg ctggaactga 480
agtaccaggt ggaattctgg aaggagggtc tgggaagcaa gaccctgttt cctgacactc 540
cctatggcca gccagtgcag attcctctcc agcaaggtgc tagccgacgc cactgcctca 600
gcgccagaac cgtctatacc ttaattgaca ttaagtacag ccagttctct gagcccagct 660
g 661

<210> 12
<211> 981
<212> DNA
<213> mouse

<400> 12
cagttctctg agcccagctg catcttccta gaggctccag ggacaaaag agctgtcctg 60
gcaatgccct cactcttgct tctactgata gcagccgttg cagcaggtgt ggcatggaag 120
ataatgaaag gaaacccctg gtttcagggg gtgaagacgc cccgggcact ggacttttct 180
gaatacagat acccagtggc aacctttcag cccagtggac ctgagttctc tgatgacttg 240
atcctttgtc cccagaagga actgaccata aggaacaggc cagcccctca ggtcagaaac 300
ccagccacgc tacaggcagg accagaaaga gacagtactg aggatgaaga cgaggacaca 360
gactacgatg acgacggtga cagcgtccag ccctacctgg aacggcccct cttcatcagc 420
gagaagcccc gggttatgga acactcggag acagacgagt ctggggtgga ttcagggggg 480
ccttggacat ccccagttgg gagtgacggc tcctctgcat gggactcttc agacaggagc 540
tggtccagca caggggactc ctcatataag gatgaagttg ggtcttccag ctgtttggac 600
cgaaaggagc cggaccaggc gccctgtggg gattggctcc aggaggctct cccatgcctg 660
gaattttctg aggacttggg caccgtggaa gagcctctga aggatggcct ctccgggtgg 720
aggatttctg gttccttatc ctcaaagaga gatctggctc ctgtggagcc cccagtttct 780
cttcagacac tgactttctg ctgggtcaac aatcctgagg gggaggagga acaggaggac 840
gaggaggaag aggaggagga ggaggaggag gaagactggg aatcagaacc taagggcagc 900
aatgccggct gttggggcac ttcaagcgtg cagaggacgg aggtcagggg ccggatgctg 960
ggagattact tggtcaggtg a 981

<210> 13
<211> 1161

<212> DNA
<213> mouse

<400> 13
atgccgtcgc tggaactgaa gtaccaggtg gaattctgga aggagggtct gggaagcaag 60
accctgtttc ctgacactcc ctatggccag ccagtgcaga ttcctctcca gcaaggtgct 120
agccgacgcc actgcctcag cgccagaacc gtctatacct taattgacat taagtacagc 180
cagttctctg agcccagctg catcttccta gaggctccag gggacaaaag agctgtcctg 240
gcaatgccct cactcttgct tctactgata gcagccgttg cagcaggtgt ggcatggaag 300
ataatgaaag gaaacccctg gtttcagggg gtgaagacgc cccgggcact ggacttttct 360
gaatacagat acccagtggc aacctttcag cccagtggac ctgagttctc tgatgacttg 420
atcctttgtc cccagaagga actgaccata aggaacaggc cagcccctca ggtcagaaac 480
ccagccacgc tacaggcagg accagaaaga gacagtactg aggatgaaga cgaggacaca 540
gactacgatg acgacggtga cagcgtccag ccctacctgg aacggcccct cttcatcagc 600
gagaagcccc gggttatgga acactcggag acagacgagt ctggggtgga ttcagggggg 660
ccttggacat ccccagttgg gagtgacggc tcctctgcat gggactcttc agacaggagc 720
tggtccagca caggggactc ctcatataag gatgaagttg ggtcttccag ctgtttggac 780
cgaaaggagc cggaccaggc gccctgtggg gattggctcc aggaggctct cccatgcctg 840
gaattttctg aggacttggg caccgtggaa gagcctctga aggatggcct ctccgggtgg 900
aggatttctg gttccttatc ctcaaagaga gatctggctc ctgtggagcc cccagtttct 960
cttcagacac tgactttctg ctgggtcaac aatcctgagg gggaggagga acaggaggac 1020
gaggaggaag aggaggagga ggaggaggag gaagactggg aatcagaacc taagggcagc 1080
aatgccggct gttggggcac ttcaagcgtg cagaggacgg aggtcagggg ccggatgctg 1140
ggagattact tggtcaggtg a 1161

<210> 14
<211> 535
<212> PRT
<213> mouse

<400> 14
Met Trp Arg Ala Asp Arg Trp Ala Pro Leu Leu Leu Phe Leu Leu Gln
1 5 10 15

Ser Ala Leu Gly Arg Pro Arg Leu Ala Pro Pro Arg Asn Val Thr Leu
20 25 30

Phe Ser Gln Asn Phe Thr Val Tyr Leu Thr Trp Leu Pro Gly Leu Gly
35 40 45

Ser Pro Pro Asn Val Thr Tyr Phe Val Thr Tyr Gln Ser Tyr Ile Lys
50 55 60

Thr Gly Trp Arg Pro Val Glu His Cys Ala Gly Ile Lys Ala Leu Val
65 70 75 80

Cys Pro Leu Met Cys Leu Lys Lys Leu Asn Leu Tyr Ser Lys Phe Lys
85 90 95

Gly Arg Val Gln Ala Ala Ser Ala His Gly Arg Ser Pro Arg Val Glu
100 105 110

Ser Arg Tyr Leu Glu Tyr Leu Phe Asp Val Glu Leu Ala Pro Pro Thr
115 120 125

Leu Val Leu Thr Gln Met Glu Lys Ile Leu Arg Val Asn Ala Thr Tyr
130 135 140

Gln Leu Pro Pro Cys Met Pro Ser Leu Glu Leu Lys Tyr Gln Val Glu
145 150 155 160

Phe Trp Lys Glu Gly Leu Gly Ser Lys Thr Leu Phe Pro Asp Thr Pro
165 170 175

Tyr Gly Gln Pro Val Gln Ile Pro Leu Gln Gln Gly Ala Ser Arg Arg
180 185 190

His Cys Leu Ser Ala Arg Thr Val Tyr Thr Leu Ile Asp Ile Lys Tyr
195 200 205

Ser Gln Phe Ser Glu Pro Ser Cys Ile Phe Leu Glu Ala Pro Gly Asp
210 215 220

Lys Arg Ala Val Leu Ala Met Pro Ser Leu Leu Leu Leu Leu Ile Ala
225 230 235 240

Ala Val Ala Ala Gly Val Ala Trp Lys Ile Met Lys Gly Asn Pro Trp
245 250 255

Phe Gln Gly Val Lys Thr Pro Arg Ala Leu Asp Phe Ser Glu Tyr Arg
260 265 270

Tyr Pro Val Ala Thr Phe Gln Pro Ser Gly Pro Glu Phe Ser Asp Asp
275 280 285

Leu Ile Leu Cys Pro Gln Lys Glu Leu Thr Ile Arg Asn Arg Pro Ala
290 295 300

Pro Gln Val Arg Asn Pro Ala Thr Leu Gln Ala Gly Pro Glu Arg Asp
305 310 315 320

Ser Thr Glu Asp Glu Asp Glu Asp Thr Asp Tyr Asp Asp Asp Gly Asp
325 330 335

Ser Val Gln Pro Tyr Leu Glu Arg Pro Leu Phe Ile Ser Glu Lys Pro
340 345 350

Arg Val Met Glu His Ser Glu Thr Asp Glu Ser Gly Val Asp Ser Gly
355 360 365

Gly Pro Trp Thr Ser Pro Val Gly Ser Asp Gly Ser Ser Ala Trp Asp
370 375 380

Ser Ser Asp Arg Ser Trp Ser Ser Thr Gly Asp Ser Ser Tyr Lys Asp
385 390 395 400

Glu Val Gly Ser Ser Ser Cys Leu Asp Arg Lys Glu Pro Asp Gln Ala
405 410 415

Pro Cys Gly Asp Trp Leu Gln Glu Ala Leu Pro Cys Leu Glu Phe Ser
420 425 430

Glu Asp Leu Gly Thr Val Glu Glu Pro Leu Lys Asp Gly Leu Ser Gly
435 440 445

Trp Arg Ile Ser Gly Ser Leu Ser Ser Lys Arg Asp Leu Ala Pro Val
450 455 460

Glu Pro Pro Val Ser Leu Gln Thr Leu Thr Phe Cys Trp Val Asn Asn
465 470 475 480

Pro Glu Gly Glu Glu Glu Gln Glu Asp Glu Glu Glu Glu Glu Glu Glu
485 490 495

Glu Glu Glu Glu Asp Trp Glu Ser Glu Pro Lys Gly Ser Asn Ala Gly
500 505 510

Cys Trp Gly Thr Ser Ser Val Gln Arg Thr Glu Val Arg Gly Arg Met
515 520 525

Leu Gly Asp Tyr Leu Val Arg
530 535

<210> 15
<211> 1605
<212> DNA
<213> mouse

<400> 15
atgtggcggg ccgaccggtg ggcgccccta ctcctgttcc tgttgcagag cgccctagga 60
aggccccgtc tagccccacc cagaaacgtg acactcttct cccagaactt cactgtttac 120
ctgacatggc ttccggggct tgggagcccc ccaaatgtga cctatttcgt gacctaccaa 180
agctatatca aaaccggttg gcgaccagtg gagcattgtg caggtatcaa ggctctggtg 240
tgtcccctga tgtgcctgaa gaaactgaac ctgtactcca agttcaaagg acgagtacag 300

gcagcttccg cacacggcag gtccccacgg gtggagtccc ggtacctgga ataccttttt 360
gacgtggagc tagccccgcc caccctggtg ctcacccaga tggagaagat cctaagggtc 420
aacgctacct accagctgcc accttgcatg ccgtcgctgg aactgaagta ccaggtggaa 480
ttctggaagg agggtctggg aagcaagacc ctgtttcctg acactcccta tggccagcca 540
gtgcagattc ctctccagca aggtgctagc cgacgccact gcctcagcgc cagaaccgtc 600
tataccttaa ttgacattaa gtacagccag ttctctgagc ccagctgcat cttcctagag 660
gctccagggg acaaaagagc tgtcctggca atgccctcac tcttgcttct actgatagca 720
gccgttgcag caggtgtggc atggaagata atgaaaggaa acccctggtt tcaggggggtg 780
aagacgcccc gggcactgga cttttctgaa tacagatacc cagtggcaac ctttcagccc 840
agtggacctg agttctctga tgacttgatc ctttgtcccc agaaggaact gaccataagg 900
aacaggccag cccctcaggt cagaaaccca gccacgctac aggcaggacc agaaagagac 960
agtactgagg atgaagacga ggacacagac tacgatgacg acggtgacag cgtccagccc 1020
tacctggaac ggcccctctt catcagcgag aagccccggg ttatggaaca ctcggagaca 1080
gacgagtctg ggtggattc aggggggcct tggacatccc cagttgggag tgacggctcc 1140
tctgcatggg actcttcaga caggagctgg tccagcacag ggactcctc atataaggat 1200
gaagttgggt cttccagctg tttggaccga aaggagccgg accaggcgcc ctgtggggat 1260
tggctccagg aggctctccc atgcctggaa ttttctgagg acttgggcac cgtggaagag 1320
cctctgaagg atggcctctc cgggtggagg atttctggtt ccttatcctc aaagagagat 1380
ctggctcctg tggagccccc agtttctctt cagacactga ctttctgctg ggtcaacaat 1440
cctgaggggg aggaggaaca ggaggacgag gaggaagagg aggaggagga ggaggaggaa 1500
gactgggaat cagaacctaa gggcagcaat gccggctgtt ggggcacttc aagcgtgcag 1560
aggacggagg tcagggccg gatgctggga gattacttgg tcagg 1605

<210> 16
<211> 1622
<212> DNA
<213> mouse

<400> 16
gccccaggat cgaaatgtgg cgggccgacc ggtgggcgcc cctactcctg ttcctgttgc 60
agagcgccct aggaaggccc cgtctagccc cacccagaaa cgtgacactc ttctcccaga 120
acttcactgt ttacctgaca tggcttccgg ggcttgggag ccccccaaat gtgacctatt 180
tcgtgaccta ccaaagctat atcaaaaccg gttggcgacc agtggagcat tgtgcaggta 240
tcaaggctct ggtgtgtccc ctgatgtgcc tgaagaaact gaacctgtac tccaagttca 300
aaggacgagt acaggcagct tccgcacacg gcaggtcccc acgggtggag tcccggtacc 360
tggaatacct ttttgacgtg gagctagccc cgcccaccct ggtgctcacc cagatggaga 420

agatcctaag ggtcaacgct acctaccagc tgccaccttg catgccgtcg ctggaactga 480
agtaccaggt ggaattctgg aaggagggtc tgggaagcaa gaccctgttt cctgacactc 540
cctatggcca gccagtgcag attcctctcc agcaaggtgc tagccgacgc cactgcctca 600
gcgccagaac cgtctatacc ttaattgaca ttaagtacag ccagttctct gagcccagct 660
gcatcttcct agaggctcca ggggacaaaa gagctgtcct ggcaatgccc tcactcttgc 720
ttctactgat agcagccgtt gcagcaggtg tggcatggaa gataatgaaa ggaaacccct 780
ggtttcaggg ggtgaagacg ccccgggcac tggacttttc tgaatacaga tacccagtgg 840
caacctttca gcccagtgga cctgagttct ctgatgactt gatcctttgt ccccagaagg 900
aactgaccat aaggaacagg ccagcccctc aggtcagaaa cccagccacg ctacaggcag 960
gaccagaaag agacagtact gaggatgaag acgaggacac agactacgat gacgacggtg 1020
acagcgtcca gccctacctg gaacggcccc tcttcatcag cgagaagccc cgggttatgg 1080
aacactcgga gacagacgag tctggggtgg attcaggggg gccttggaca tccccagttg 1140
ggagtgacgg ctcctctgca tgggactctt cagacaggag ctggtccagc acaggggact 1200
cctcatataa ggatgaagtt gggtcttcca gctgtttgga ccgaaaggag ccggaccagg 1260
cgccctgtgg ggattggctc caggaggctc tcccatgcct ggaattttct gaggacttgg 1320
gcaccgtgga agagcctctg aaggatggcc tctccgggtg gaggatttct ggttccttat 1380
cctcaaagag agatctggct cctgtggagc ccccagtttc tcttcagaca ctgactttct 1440
gctgggtcaa caatcctgag gggga ggagg aacaggagga cgaggaggaa gaggaggagg 1500
aggaggagga ggaagactgg gaatcagaac ctaagggcag caatgccggc tgttggggca 1560
cttcaagcgt gcagaggacg gaggtcaggg gccggatgct gggagattac ttggtcaggt 1620
ga 1622

<210> 17
<211> 461
<212> PRT
<213> Artificial Sequence

<223> Fusion protein between extracellular domain of mouse SJ2386 and Fc fragment.

<400> 17
Met Trp Arg Ala Asp Arg Trp Ala Pro Leu Leu Leu Phe Leu Leu Gln
1 5 10 15

Ser Ala Leu Gly Arg Pro Arg Leu Ala Pro Pro Arg Asn Val Thr Leu
20 25 30

Phe Ser Gln Asn Phe Thr Val Tyr Leu Thr Trp Leu Pro Gly Leu Gly
35 40 45

Ser Pro Pro Asn Val Thr Tyr Phe Val Thr Tyr Gln Ser Tyr Ile Lys
50 55 60

Thr Gly Trp Arg Pro Val Glu His Cys Ala Gly Ile Lys Ala Leu Val
65 70 75 80

Cys Pro Leu Met Cys Leu Lys Lys Leu Asn Leu Tyr Ser Lys Phe Lys
85 90 95

Gly Arg Val Gln Ala Ala Ser Ala His Gly Arg Ser Pro Arg Val Glu
100 105 110

Ser Arg Tyr Leu Glu Tyr Leu Phe Asp Val Glu Leu Ala Pro Pro Thr
115 120 125

Leu Val Leu Thr Gln Met Glu Lys Ile Leu Arg Val Asn Ala Thr Tyr
130 135 140

Gln Leu Pro Pro Cys Met Pro Ser Leu Glu Leu Lys Tyr Gln Val Glu
145 150 155 160

Phe Trp Lys Glu Gly Leu Gly Ser Lys Thr Leu Phe Pro Asp Thr Pro
165 170 175

Tyr Gly Gln Pro Val Gln Ile Pro Leu Gln Gln Gly Ala Ser Arg Arg
180 185 190

His Cys Leu Ser Ala Arg Thr Val Tyr Thr Leu Ile Asp Ile Lys Tyr
195 200 205

Ser Gln Phe Ser Glu Pro Ser Cys Ile Phe Leu Glu Ala Pro Gly Asp
210 215 220

Gly Ser Arg Ser Asn Glu Pro Lys Ser Cys Asp Lys Thr His Thr Cys
225 230 235 240

Pro Pro Cys Pro Ala Pro Glu Leu Leu Gly Gly Pro Ser Val Phe Leu
245 250 255

Phe Pro Pro Lys Pro Lys Asp Thr Leu Met Ile Ser Arg Thr Pro Glu
260 265 270

Val Thr Cys Val Val Val Asp Val Ser His Glu Asp Pro Glu Val Lys
275 280 285

Phe Asn Trp Tyr Val Asp Gly Val Glu Val His Asn Ala Lys Thr Lys
290 295 300

Pro Arg Glu Glu Gln Tyr Asn Ser Thr Tyr Arg Val Val Ser Val Leu
305 310 315 320

Thr Val Leu His Gln Asp Trp Leu Asn Gly Lys Glu Tyr Lys Cys Lys
325 330 335

Val Ser Asn Lys Ala Leu Pro Ala Pro Ile Glu Lys Thr Ile Ser Lys
340 345 350

Ala Lys Gly Gln Pro Arg Glu Pro Gln Val Tyr Thr Leu Pro Pro Ser
355 360 365

Arg Asp Glu Leu Thr Lys Asn Gln Val Ser Leu Thr Cys Leu Val Lys
370 375 380

Gly Phe Tyr Pro Ser Asp Ile Ala Val Glu Trp Glu Ser Asn Gly Gln
385 390 395 400

Pro Glu Asn Asn Tyr Lys Thr Thr Pro Pro Val Leu Asp Ser Asp Gly
405 410 415

Ser Phe Phe Leu Tyr Ser Lys Leu Thr Val Asp Lys Ser Arg Trp Gln
420 425 430

Gln Gly Asn Val Phe Ser Cys Ser Val Met His Glu Ala Leu His Asn
435 440 445

His Tyr Thr Gln Lys Ser Leu Ser Leu Ser Pro Gly Lys
450 455 460

<210> 18
<211> 518
<212> PRT
<213> mouse

<400> 18
Met Trp Arg Ala Asp Arg Trp Ala Pro Leu Leu Leu Phe Leu Leu Gln
1 5 10 15

Ser Ala Leu Gly Arg Pro Arg Leu Ala Pro Pro Arg Asn Val Thr Leu
20 25 30

Phe Ser Gln Asn Phe Thr Val Tyr Leu Thr Trp Leu Pro Gly Leu Gly
35 40 45

Ser Pro Pro Asn Val Thr Tyr Phe Val Thr Tyr Gln Ser Tyr Ile Lys
50 55 60

Thr Gly Trp Arg Pro Val Glu His Cys Ala Gly Ile Lys Ala Leu Val
65 70 75 80

Cys Pro Leu Met Cys Leu Lys Lys Leu Asn Leu Tyr Ser Lys Phe Lys
85 90 95

Gly Arg Val Gln Ala Ala Ser Ala His Gly Arg Ser Pro Arg Val Glu
100 105 110

Ser Arg Tyr Leu Glu Tyr Leu Phe Asp Val Glu Leu Ala Pro Pro Thr
115 120 125

Leu Val Leu Thr Gln Met Glu Lys Ile Leu Arg Val Asn Ala Thr Tyr
130 135 140

Gln Leu Pro Pro Cys Met Pro Ser Leu Glu Leu Lys Tyr Gln Val Glu
145 150 155 160

Phe Trp Lys Glu Gly Leu Gly Ser Lys Thr Leu Phe Pro Asp Thr Pro
165 170 175

Tyr Gly Gln Pro Val Gln Ile Pro Leu Gln Gln Gly Ala Ser Arg Arg
180 185 190

His Cys Leu Ser Ala Arg Thr Val Tyr Thr Leu Ile Asp Ile Lys Tyr
195 200 205

Ser Gln Phe Ser Glu Pro Ser Cys Ile Phe Leu Glu Ala Pro Ala Ala
210 215 220

Val Ala Ala Gly Val Ala Trp Lys Ile Met Lys Gly Asn Pro Trp Phe
225 230 235 240

Gln Gly Val Lys Thr Pro Arg Ala Leu Asp Phe Ser Glu Tyr Arg Tyr
245 250 255

Pro Val Ala Thr Phe Gln Pro Ser Gly Pro Glu Phe Ser Asp Asp Leu
260 265 270

Ile Leu Cys Pro Gln Lys Glu Leu Thr Ile Arg Asn Arg Pro Ala Pro
275 280 285

Gln Val Arg Asn Pro Ala Thr Leu Gln Ala Gly Pro Glu Arg Asp Ser
290 295 300

Thr Glu Asp Glu Asp Glu Asp Thr Asp Tyr Asp Asp Asp Gly Asp Ser
305 310 315 320

Val Gln Pro Tyr Leu Glu Arg Pro Leu Phe Ile Ser Glu Lys Pro Arg
325 330 335

Val Met Glu His Ser Glu Thr Asp Glu Ser Gly Val Asp Ser Gly Gly
340 345 350

Pro Trp Thr Ser Pro Val Gly Ser Asp Gly Ser Ser Ala Trp Asp Ser
355 360 365

Ser Asp Arg Ser Trp Ser Ser Thr Gly Asp Ser Ser Tyr Lys Asp Glu
370 375 380

Val Gly Ser Ser Ser Cys Leu Asp Arg Lys Glu Pro Asp Gln Ala Pro
385 390 395 400

Cys Gly Asp Trp Leu Gln Glu Ala Leu Pro Cys Leu Glu Phe Ser Glu
405 410 415

Asp Leu Gly Thr Val Glu Glu Pro Leu Lys Asp Gly Leu Ser Gly Trp
420 425 430

Arg Ile Ser Gly Ser Leu Ser Ser Lys Arg Asp Leu Ala Pro Val Glu
435 440 445

Pro Pro Val Ser Leu Gln Thr Leu Thr Phe Cys Trp Val Asn Asn Pro
450 455 460

Glu Gly Glu Glu Glu Gln Glu Asp Glu Glu Glu Glu Glu Glu Glu Glu
465 470 475 480

Glu Glu Glu Asp Trp Glu Ser Glu Pro Lys Gly Ser Asn Ala Gly Cys
485 490 495

Trp Gly Thr Ser Ser Val Gln Arg Thr Glu Val Arg Gly Arg Met Leu
500 505 510

Gly Asp Tyr Leu Val Arg
515

<210> 19
<211> 462
<212> PRT
<213> Artificial Sequence

<223> Fusion protein between extracellular domain of human SJ2386 and Fc fragment

<400> 19
Met Ala Gly Pro Glu Arg Trp Gly Pro Leu Leu Leu Cys Leu Leu Gln
1 5 10 15

Ala Ala Pro Gly Arg Pro Arg Leu Ala Pro Pro Gln Asn Val Thr Leu
20 25 30

Leu Ser Gln Asn Phe Ser Val Tyr Leu Thr Trp Leu Pro Gly Leu Gly
35 40 45

Asn Pro Gln Asp Val Thr Tyr Phe Val Ala Tyr Gln Ser Ser Pro Thr
50 55 60

Arg Arg Arg Trp Arg Glu Val Glu Glu Cys Ala Gly Thr Lys Glu Leu
65 70 75 80

Leu Cys Ser Met Met Cys Leu Lys Lys Gln Asp Leu Tyr Asn Lys Phe
85 90 95

Lys Gly Arg Val Arg Thr Val Ser Pro Ser Ser Lys Ser Pro Trp Val
100 105 110

Glu Ser Glu Tyr Leu Asp Tyr Leu Phe Glu Val Glu Pro Ala Pro Pro

115 120 125

Val Leu Val Leu Thr Gln Thr Glu Glu Ile Leu Ser Ala Asn Ala Thr
130 135 140

Tyr Gln Leu Pro Pro Cys Met Pro Pro Leu Asp Leu Lys Tyr Glu Val
145 150 155 160

Ala Phe Trp Lys Glu Gly Ala Gly Asn Lys Thr Leu Phe Pro Val Thr
165 170 175

Pro His Gly Gln Pro Val Gln Ile Thr Leu Gln Pro Ala Ala Ser Glu
180 185 190

His His Cys Leu Ser Ala Arg Thr Ile Tyr Thr Phe Ser Val Pro Lys
195 200 205

Tyr Ser Lys Phe Ser Lys Pro Thr Cys Phe Leu Leu Glu Val Pro Glu
210 215 220

Ala Gly Ser Arg Ser Asn Glu Pro Lys Ser Cys Asp Lys Thr His Thr
225 230 235 240

Cys Pro Pro Cys Pro Ala Pro Glu Leu Leu Gly Gly Pro Ser Val Phe
245 250 255

Leu Phe Pro Pro Lys Pro Lys Asp Thr Leu Met Ile Ser Arg Thr Pro
260 265 270

Glu Val Thr Cys Val Val Val Asp Val Ser His Glu Asp Pro Glu Val
275 280 285

Lys Phe Asn Trp Tyr Val Asp Gly Val Glu Val His Asn Ala Lys Thr
290 295 300

Lys Pro Arg Glu Glu Gln Tyr Asn Ser Thr Tyr Arg Val Val Ser Val

305 310 315 320

Leu Thr Val Leu His Gln Asp Trp Leu Asn Gly Lys Glu Tyr Lys Cys
325 330 335

Lys Val Ser Asn Lys Ala Leu Pro Ala Pro Ile Glu Lys Thr Ile Ser
340 345 350

Lys Ala Lys Gly Gln Pro Arg Glu Pro Gln Val Tyr Thr Leu Pro Pro
355 360 365

Ser Arg Asp Glu Leu Thr Lys Asn Gln Val Ser Leu Thr Cys Leu Val
370 375 380

Lys Gly Phe Tyr Pro Ser Asp Ile Ala Val Glu Trp Glu Ser Asn Gly
385 390 395 400

Gln Pro Glu Asn Asn Tyr Lys Thr Thr Pro Pro Val Leu Asp Ser Asp
405 410 415

Gly Ser Phe Phe Leu Tyr Ser Lys Leu Thr Val Asp Lys Ser Arg Trp
420 425 430

Gln Gln Gly Asn Val Phe Ser Cys Ser Val Met His Glu Ala Leu His
435 440 445

Asn His Tyr Thr Gln Lys Ser Leu Ser Leu Ser Pro Gly Lys
450 455 460

<210> 20
<211> 244
<212> PRT
<213> homo sapiense

<400> 20
Met Ala Gly Pro Glu Arg Trp Gly Pro Leu Leu Leu Cys Leu Leu Gln

1 5 10 15

Ala Ala Pro Gly Arg Pro Arg Leu Ala Pro Pro Gln Asn Val Thr Leu
20 25 30

Leu Ser Gln Asn Phe Ser Val Tyr Leu Thr Trp Leu Pro Gly Leu Gly
35 40 45

Asn Pro Gln Asp Val Thr Tyr Phe Val Ala Tyr Gln Ser Ser Pro Thr
50 55 60

Arg Arg Arg Trp Arg Glu Val Glu Glu Cys Ala Gly Thr Lys Glu Leu
65 70 75 80

Leu Cys Ser Met Met Cys Leu Lys Lys Gln Asp Leu Tyr Asn Lys Phe
85 90 95

Lys Gly Arg Val Arg Thr Val Ser Pro Ser Ser Lys Ser Pro Trp Val
100 105 110

Glu Ser Glu Tyr Leu Asp Tyr Leu Phe Glu Val Glu Pro Ala Pro Pro
115 120 125

Val Leu Val Leu Thr Gln Thr Glu Glu Ile Leu Ser Ala Asn Ala Thr
130 135 140

Tyr Gln Leu Pro Pro Cys Met Pro Pro Leu Asp Leu Lys Tyr Glu Val
145 150 155 160

Ala Phe Trp Lys Glu Gly Ala Gly Asn Lys Thr Leu Phe Pro Val Thr
165 170 175

Pro His Gly Gln Pro Val Gln Ile Thr Leu Gln Pro Ala Ala Ser Glu
180 185 190

His His Cys Leu Ser Ala Arg Thr Ile Tyr Thr Phe Ser Val Pro Lys

195 200 205

Tyr Ser Lys Phe Ser Lys Pro Thr Cys Phe Leu Leu Glu Val Pro Gly
210 215 220

Leu Phe Trp Thr His Thr Pro Cys Gly Asn Leu Ser Ala Gln Gln Thr
225 230 235 240

Arg Val Arg Glu

<210> 21
<211> 42
<212> PRT
<213> mouse

<400> 21
Met Ala Gly Pro Glu Arg Trp Gly Pro Leu Leu Leu Cys Leu Leu Gln
1 5 10 15

Ala Ala Pro Ala Leu Pro Pro Val Asp Gly Gly Ala Lys Trp Lys Ser
20 25 30

Val Arg Glu Pro Arg Ser Cys Tyr Val Leu
35 40

<210> 22
<211> 529
<212> PRT
<213> Artificial Sequence

<223> Fusion protein between extracellular domain of alpha chain of IL10 receptor and cytosol domain of human SJ2368

<400> 22

Met Leu Pro Cys Leu Val Val Leu Leu Ala Ala Leu Leu Ser Leu Arg
1 5 10 15

Leu Gly Ser Asp Ala His Gly Thr Glu Leu Pro Ser Pro Pro Ser Val
20 25 30

Trp Phe Glu Ala Glu Phe Phe His His Ile Leu His Trp Thr Pro Ile
35 40 45

Pro Asn Gln Ser Glu Ser Thr Cys Tyr Glu Val Ala Leu Leu Arg Tyr
50 55 60

Gly Ile Glu Ser Trp Asn Ser Ile Ser Asn Cys Ser Gln Thr Leu Ser
65 70 75 80

Tyr Asp Leu Thr Ala Val Thr Leu Asp Leu Tyr His Ser Asn Gly Tyr
85 90 95

Arg Ala Arg Val Arg Ala Val Asp Gly Ser Arg His Ser Asn Trp Thr
100 105 110

Val Thr Asn Thr Arg Phe Ser Val Asp Glu Val Thr Leu Thr Val Gly
115 120 125

Ser Val Asn Leu Glu Ile His Asn Gly Phe Ile Leu Gly Lys Ile Gln
130 135 140

Leu Pro Arg Pro Lys Met Ala Pro Ala Asn Asp Thr Tyr Glu Ser Ile
145 150 155 160

Phe Ser His Phe Arg Glu Tyr Glu Ile Ala Ile Arg Lys Val Pro Gly
165 170 175

Asn Phe Thr Phe Thr His Lys Lys Val Lys His Glu Asn Phe Ser Leu
180 185 190

Leu Thr Ser Gly Glu Val Gly Glu Phe Cys Val Gln Val Lys Pro Ser
195 200 205

Val Ala Ser Arg Ser Asn Lys Gly Met Trp Ser Lys Glu Glu Cys Ile
210 215 220

Ser Leu Thr Arg Gln Tyr Phe Thr Val Thr Asn Trp Ala Phe Leu Val
225 230 235 240

Leu Pro Ser Leu Leu Ile Leu Leu Leu Val Ile Ala Ala Gly Gly Val
245 250 255

Ile Trp Lys Thr Leu Met Gly Asn Pro Trp Phe Gln Arg Ala Lys Met
260 265 270

Pro Arg Ala Leu Asp Phe Ser Gly His Thr His Pro Val Ala Thr Phe
275 280 285

Gln Pro Ser Arg Pro Glu Ser Val Asn Asp Leu Phe Leu Cys Pro Gln
290 295 300

Lys Glu Leu Thr Arg Gly Val Arg Pro Thr Pro Arg Val Arg Ala Pro
305 310 315 320

Ala Thr Gln Gln Thr Arg Trp Lys Lys Asp Leu Ala Glu Asp Glu Glu
325 330 335

Glu Glu Asp Glu Glu Asp Thr Glu Asp Gly Val Ser Phe Gln Pro Tyr
340 345 350

Ile Glu Pro Pro Ser Phe Leu Gly Gln Glu His Gln Ala Pro Gly His
355 360 365

Ser Glu Ala Gly Gly Val Asp Ser Gly Arg Pro Arg Ala Pro Leu Val
370 375 380

Pro Ser Glu Gly Ser Ser Ala Trp Asp Ser Ser Asp Arg Ser Trp Ala
385 390 395 400

Ser Thr Val Asp Ser Ser Trp Asp Arg Ala Gly Ser Ser Gly Tyr Leu
405 410 415

Ala Glu Lys Gly Pro Gly Gln Gly Pro Gly Gly Asp Gly His Gln Glu
420 425 430

Ser Leu Pro Pro Pro Glu Phe Ser Lys Asp Ser Gly Phe Leu Glu Glu
435 440 445

Leu Pro Glu Asp Asn Leu Ser Ser Trp Ala Thr Trp Gly Thr Leu Pro
450 455 460

Pro Glu Pro Asn Leu Val Pro Gly Gly Pro Pro Val Ser Leu Gln Thr
465 470 475 480

Leu Thr Phe Cys Trp Glu Ser Ser Pro Glu Glu Glu Glu Glu Ala Arg
485 490 495

Glu Ser Glu Ile Glu Asp Ser Asp Ala Gly Ser Trp Gly Ala Glu Ser
500 505 510

Thr Gln Arg Thr Glu Asp Arg Gly Arg Thr Leu Gly His Tyr Met Ala
515 520 525

Arg

<210> 23
<211> 523
<212> PRT
<213> Artificial Sequence

<223> Fusion protein between extracellular domain of alpha chain of IL10 receptor and cytosol domain of human SJ2368

<400> 23

Met Leu Pro Cys Leu Val Val Leu Leu Ala Ala Leu Leu Ser Leu Arg
1 5 10 15

Leu Gly Ser Asp Ala His Gly Thr Glu Leu Pro Ser Pro Pro Ser Val
20 25 30

Trp Phe Glu Ala Glu Phe Phe His His Ile Leu His Trp Thr Pro Ile
35 40 45

Pro Asn Gln Ser Glu Ser Thr Cys Tyr Glu Val Ala Leu Leu Arg Tyr
50 55 60

Gly Ile Glu Ser Trp Asn Ser Ile Ser Asn Cys Ser Gln Thr Leu Ser
65 70 75 80

Tyr Asp Leu Thr Ala Val Thr Leu Asp Leu Tyr His Ser Asn Gly Tyr
85 90 95

Arg Ala Arg Val Arg Ala Val Asp Gly Ser Arg His Ser Asn Trp Thr
100 105 110

Val Thr Asn Thr Arg Phe Ser Val Asp Glu Val Thr Leu Thr Val Gly
115 120 125

Ser Val Asn Leu Glu Ile His Asn Gly Phe Ile Leu Gly Lys Ile Gln
130 135 140

Leu Pro Arg Pro Lys Met Ala Pro Ala Asn Asp Thr Tyr Glu Ser Ile
145 150 155 160

Phe Ser His Phe Arg Glu Tyr Glu Ile Ala Ile Arg Lys Val Pro Gly

165 170 175

Asn Phe Thr Phe Thr His Lys Lys Val Lys His Glu Asn Phe Ser Leu
180 185 190

Leu Thr Ser Gly Glu Val Gly Glu Phe Cys Val Gln Val Lys Pro Ser
195 200 205

Val Ala Ser Arg Ser Asn Lys Gly Met Trp Ser Lys Glu Glu Cys Ile
210 215 220

Ser Leu Thr Arg Asn Trp Ala Phe Leu Val Leu Pro Ser Leu Leu Ile
225 230 235 240

Leu Leu Leu Val Ile Ala Ala Gly Gly Val Ile Trp Lys Thr Leu Met
245 250 255

Gly Asn Pro Trp Phe Gln Arg Ala Lys Met Pro Arg Ala Leu Asp Phe
260 265 270

Ser Gly His Thr His Pro Val Ala Thr Phe Gln Pro Ser Arg Pro Glu
275 280 285

Ser Val Asn Asp Leu Phe Leu Cys Pro Gln Lys Glu Leu Thr Arg Gly
290 295 300

Val Arg Pro Thr Pro Arg Val Arg Ala Pro Ala Thr Gln Gln Thr Arg
305 310 315 320

Trp Lys Lys Asp Leu Ala Glu Asp Glu Glu Glu Glu Asp Glu Glu Asp
325 330 335

Thr Glu Asp Gly Val Ser Phe Gln Pro Tyr Ile Glu Pro Pro Ser Phe
340 345 350

Leu Gly Gln Glu His Gln Ala Pro Gly His Ser Glu Ala Gly Gly Val

355 360 365

Asp Ser Gly Arg Pro Arg Ala Pro Leu Val Pro Ser Glu Gly Ser Ser
370 375 380

Ala Trp Asp Ser Ser Asp Arg Ser Trp Ala Ser Thr Val Asp Ser Ser
385 390 395 400

Trp Asp Arg Ala Gly Ser Ser Gly Tyr Leu Ala Glu Lys Gly Pro Gly
405 410 415

Gln Gly Pro Gly Gly Asp Gly His Gln Glu Ser Leu Pro Pro Pro Glu
420 425 430

Phe Ser Lys Asp Ser Gly Phe Leu Glu Glu Leu Pro Glu Asp Asn Leu
435 440 445

Ser Ser Trp Ala Thr Trp Gly Thr Leu Pro Pro Glu Pro Asn Leu Val
450 455 460

Pro Gly Gly Pro Pro Val Ser Leu Gln Thr Leu Thr Phe Cys Trp Glu
465 470 475 480

Ser Ser Pro Glu Glu Glu Glu Glu Ala Arg Glu Ser Glu Ile Glu Asp
485 490 495

Ser Asp Ala Gly Ser Trp Gly Ala Glu Ser Thr Gln Arg Thr Glu Asp
500 505 510

Arg Gly Arg Thr Leu Gly His Tyr Met Ala Arg
515 520

<210> 24
<211> 732
<212> DNA

<213> homo sapience

<400> 24
atggcggggc ccgagcgctg gggccccctg ctcctgtgcc tgctgcaggc cgctccaggg 60
aggccccgtc tggcccctcc ccagaatgtg acgctgctct cccagaactt cagcgtgtac 120
ctgacatggc tcccagggct tggcaacccc caggatgtga cctattttgt ggcctatcag 180
agctctccca cccgtagacg gtggcgcgaa gtggaagagt gtgcgggaac caaggagctg 240
ctatgttcta tgatgtgcct gaagaaacag gacctgtaca acaagttcaa gggacgcgtg 300
cggacggttt ctcccagctc caagtccccc tgggtggagt ccgaatacct ggattacctt 360
tttgaagtgg agccggcccc acctgtcctg gtgctcaccc agacggagga gatcctgagt 420
gccaatgcca cgtaccagct gccccccctgc atgcccccac tggatctgaa gtatgaggtg 480
gcattctgga aggaggggc cggaaacaag accctatttc cagtcactcc ccatggccag 540
ccagtccaga tcactctcca gccagctgcc agcgaacacc actgcctcag tgccagaacc 600
atctacacgt tcagtgtccc gaaatacagc aagttctcta agcccacctg cttcttgctg 660
gaggtcccag gacttttctg gacacacaca ccctgtggca acctttcagc ccagcagacc 720
agagtccgtg aa 732

<210> 25
<211> 1554
<212> DNA
<213> mouse

<400> 25
atgtggcggg ccgaccggtg ggcgccccta ctcctgttcc tgttgcagag cgccctagga 60
aggccccgtc tagccccacc cagaaacgtg acactcttct cccagaactt cactgtttac 120
ctgacatggc ttccggggct tgggagcccc ccaaatgtga cctatttcgt gacctaccaa 180
agctatatca aaaccggttg gcgaccagtg gagcattgtg caggtatcaa ggctctggtg 240
tgtcccctga tgtgcctgaa gaaactgaac ctgtactcca agttcaaagg acgagtacag 300
gcagcttccg cacacggcag gtccccacgg gtggagtccc ggtacctgga atacctttt 360
gacgtggagc tagccccgcc caccctggtg ctcacccaga tggagaagat cctaagggtc 420
aacgctacct accagctgcc accttgcatg ccgtcgctgg aactgaagta ccaggtggaa 480
ttctggaagg agggtctggg aagcaagacc ctgtttcctg acactcccta tggccagcca 540
gtgcagattc ctctccagca aggtgctagc cgacgccact gcctcagcgc cagaaccgtc 600
tataccttaa ttgacattaa gtacagccag ttctctgagc ccagctgcat cttcctagag 660
gctccagcag ccgttgcagc aggtgtggca tggaagataa tgaaaggaaa cccctggttt 720

```
caggggtga agacgccccg ggcactggac ttttctgaat acagataccc agtggcaacc 780
tttcagccca gtggacctga gttctctgat gacttgatcc tttgtcccca gaaggaactg 840
accataagga acaggccagc ccctcaggtc agaaacccag ccacgctaca ggcaggacca 900
gaaagagaca gtactgagga tgaagacgag gacacagact acgatgacga cggtgacagc 960
gtccagccct acctggaacg gcccctcttc atcagcgaga agccccgggt tatggaacac 1020
tcggagacag acgagtctgg ggtggattca ggggggcctt ggacatcccc agttgggagt 1080
gacggctcct ctgcatggga ctcttcagac aggagctggt ccagcacagg ggactcctca 1140
tataaggatg aagttgggtc ttccagctgt ttggaccgaa aggagccgga ccaggcgccc 1200
tgtggggatt ggctccagga ggctctccca tgcctggaat tttctgagga cttgggcacc 1260
gtggaagagc ctctgaagga tggcctctcc gggtggagga tttctggttc cttatcctca 1320
aagagagatc tggctcctgt ggagcccccca gtttctcttc agacactgac tttctgctgg 1380
gtcaacaatc ctgaggggga ggaggaacag gaggacgagg aggaagagga ggaggaggag 1440
gaggaggaag actgggaatc agaacctaag ggcagcaatg ccggctgttg gggcacttca 1500
agcgtgcaga ggacggaggt caggggccgg atgctgggag attacttggt cagg       1554
```